# EUROPÄISCHE PATENTANMELDUNG

(11) **EP 0 705 835 A1**
(43) Veröffentlichungstag der Anmeldung: **10.04.1996**
(21) Anmeldenummer: 95810529.8
(22) Anmeldetag: 24.08.1995
(51) Int. Cl.: C07D 498/06, C07D 513/06, A61K 31/495

(54) **Oxa- oder thiaaliphatisch überbrückte Chinoxalin-2,3-dione**

(30) Priorität: 01.09.1994 CH 2680/94; 09.02.1995 CH 402/95
(71) Anmelder: CIBA-GEIGY AG, CH-4002 Basel (CH)
(72) Erfinder: Moretti, Robert, Dr., CH-1627 Vaulruz (CH); Zimmermann, Kaspar, Dr., CH-4125 Riehen (CH)

(57) **Zusammenfassung**

Oxa- und thiaaliphatisch überbrückte Chinoxalin-2,3-dione der allgemeinen Formel I 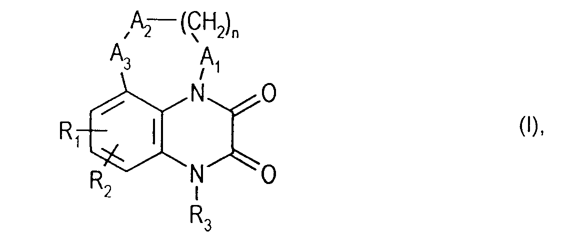
worin
A₁ Niederalkyliden oder eine Gruppe der Formel >CH-A₄-R₄ (Ia) darstellt,
A₂ Niederalkyliden oder eine Gruppe der Formeln >CH-A₄-R₄ (Ia), >C=O (Ib) oder >CH(OH)-A₅-R₄ (Ic) ist,
A₃ Oxy, gegebenenfalls oxidiertes Thio oder eine Gruppe >C(=O) (Ib bedeutet,
A₄ für Niederalkylen steht,
A₅ Niederalkylen oder eine direkte Bindung darstellt,
n für 0 oder 1 steht,
R₁ und R₂ unabhängig voneinander Wasserstoff, gegebenenfalls durch Niederalkyl und/oder Niederalkanoyl substituiertes Amino, Nitro, Niederalkanoyl, gegebenenfalls verethertes oder verestertes Hydroxy, gegebenenfalls verestertes oder amidiertes Carboxy, Cyano, gegebenenfalls halogeniertes Niederalkyl oder Halogen bedeuten,
R₃ Wasserstoff oder Hydroxy bedeutet und
R₄ Wasserstoff, Cyano, gegebenenfalls verestertes oder amidiertes Carboxy, gegebenenfalls verestertes Phosphono oder 5-Tetrazolyl darstellt,
und ihre pharmazeutisch verwendbaren Salze weisen antagonistische Eigenschaften gegenüber excitatorischen Aminosäuren auf und können als antikonvulsive und anti-neurodegenerative Arzneimittelwirkstoffe verwendet werden.

## Beschreibung

Die Erfindung betrifft neue oxa- und thiaaliphatisch überbrückte Chinoxalin-2,3-dione der allgemeinen Formel I 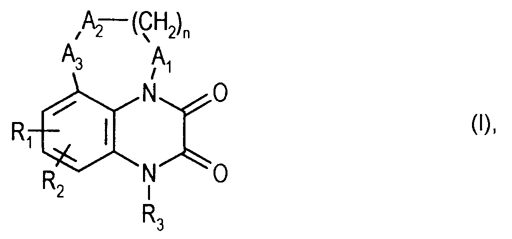
worin
A₁ Niederalkyliden oder eine Gruppe der Formel >CH-A₄-R₄ (la) darstellt,
A₂ Niederalkyliden oder eine Gruppe der Formeln >CH-A₄-R₄ (la), >C=O (Ib) oder >CH(OH)-A₅-R₄ (Ic) ist,
A₃ Oxy, gegebenenfalls oxidiertes Thio oder eine Gruppe >C(=O) (Ib) bedeutet,
A₄ für Niederalkylen steht,
A₅ Niederalkylen oder eine direkte Bindung darstellt,
n für 0 oder 1 steht,
R₁ und R₂ unabhängig voneinander Wasserstoff, gegebenenfalls durch Niederalkyl und/oder Niederalkanoyl substituiertes Amino, Nitro, Niederalkanoyl, gegebenenfalls verethertes oder verestertes Hydroxy, gegebenenfalls verestertes oder amidiertes Carboxy, Cyano, gegebenenfalls halogeniertes Niederalkyl oder Halogen bedeuten,
R₃ Wasserstoff oder Hydroxy bedeutet und
R₄ Wasserstoff, Cyano, gegebenenfalls verestertes oder amidiertes Carboxy, gegebenenfalls verestertes Phosphono oder 5-Tetrazolyl darstellt,
und ihre Salze, die neuen Verbindungen enthaltende pharmazeutische Präparate und ihre Verwendung als Arzneimittelwirkstoffe.

Gegebenenfalls oxidiertes Thio ist Thio, Sulfinyl oder Sulfonyl.

Gegebenenfalls durch Niederalkyl und/oder Niederalkanoyl substituiertes Amino ist beispielsweise Amino, Niederalkylamino, Niederalkanoylamino, Diniederalkylamino oder N-Niederalkanoyl-N-niederalkyl-amino.

Gegebenenfalls verethertes oder verestertes Hydroxy ist beispielsweise gegebenenfalls mit einem Niederalkanol verethertes oder mit einer Niederalkansäure verestertes Hydroxy, insbesondere Hydroxy, Niederalkanoyloxy oder Niederalkoxy, kann aber auch Niederalkenyloxy oder Niederalkinyloxy sein.

Gegebenenfalls halogeniertes Niederalkyl ist beispielsweise Niederalkyl oder Polyhalogenniederalkyl, insbesondere Trifluormethyl.

Gegebenenfalls durch Niederalkanoyl substituiertes Amino ist beispielsweise Amino oder Niederalkanoylamino.

Gegebenenfalls verestertes oder amidiertes Carboxy ist beispielsweise Carboxy, Niederalkoxycarbonyl, Carboxyniederalkoxycarbonyl, Niederalkoxycarbonylniederalkoxycarbonyl, Niederalkanoyloxyniederalkoxycarbonyl, jeweils unsubstituiertes oder durch Niederalkyl, Niederalkoxy, Hydroxy, Halogen und/oder Trifluormethyl substituiertes Phenyloxycarbonyl, Benzoyloxyniederalkoxycarbonyl oder Phenylniederalkoxycarbonyl, Carbamoyl, Niederalkylcarbamoyl, Niederalkenylcarbamoyl, Diniederalkylcarbamoyl, Diniederalkylaminoniederalkylcarbamoyl, Aminoniederalkylaminoniederalkylcarbamoyl, 2-Oxoimidazolidin-1-ylniederalkylcarbamoyl, gegebenenfalls durch Aminoniederalkylamino bzw. 2-Oxoimidazolidin-1-yl substituiertes Niederalkylenaminoniederalkylcarbamoyl, wie Aminoniederalkylaminoniederalkylencarbamoyl oder 2-Oxoimidazolidin-1-ylniederalkylencarbamoyl, Oxaniederalkylenaminoniederalkylcarbamoyl, gegebenenfalls durch Carboxy oder Niederalkoxycarbonyl substituiertes Cycloalkylcarbamoyl, Cycloalkylniederalkylcarbamoyl, gegebenenfalls durch Oxo substituiertes Azacycloalkylcarbamoyl, gegebenenfalls benzokondensiertes Niederalkylencarbamoyl, unsubstituiertes oder durch Niederalkyl, Niederalkoxy, Hydroxy, Halogen und/oder Trifluormethyl substituiertes Phenylniederalkylcarbamyol, unsubstituiertes oder durch Niederalkyl, Niederalkoxy, Hydroxy, Halogen, Amino, Niederalkoxycarbonylamino, Nitro, Carboxy, Niederalkoxycarbonyl, Phenyl, Phenyloxy und/oder Trifluormethyl substituiertes N-Phenyl- oder N-Niederalkyl-N-phenylcarbamoyl, gegebenenfalls partiell hydriertes N-Naphthylcarbamoyl, N-lndanylcarbamoyl, N-Heteroaryl- oder N-Heteroarylniederalkylcarbamoyl, Mono- oder Dihydroxyniederalkylcarbamoyl, Mono- oder Diniederalkoxyniederalkylcarbamoyl, Polyhalogenniederalkylcarbamoyl, gegebenenfalls verestertes Mono- oder Dicarboxyniederalkylcarbamoyl, Cyanoniederalkylcarbamoyl, gegebenenfalls verestertes Carboxyniederalkenylcarbamoyl oder gegebenenfalls verethertes N-Hydroxycarbamoyl, wie N-Hydroxycarbamoyl, N-Niederalkoxycarbamoyl, N-Niederalkoxy-N-niederalkyl-carbamoyl, N-Niederalkenyloxycarbamoyl oder unsubstituiertes oder durch Niederalkyl, Niederalkoxy, Hydroxy, Halogen und/oder Trifluormethyl substituiertes N-Phenyloxy-, N-Phenylniederalkoxy- oder N-Phenylniederalkenyloxycarbamoyl.

Gegebenenfalls durch Carboxy oder Niederalkoxy substituiertes Cycloalkylcarbamoyl ist beispielsweise C₃-C₇-Cycloalkylcarbamoyl, Carboxy-C₃-C₇-cycloalkylcarbamoyl oder C₁-C₄-Alkoxycarbonyl-C₃-C₇-cycloalkylcarbamoyl, wie 1-Carboxy-, 1-Methoxycarbonyl- oder 1-Ethoxycarbonylcyclopropylcarbamoyl.

Gegebenenfalls verestertes Phosphono kann vollständig oder partiell verestert sein und bedeutet beispielsweise Phosphono, Niederalkylphosphono, Diniederalkylphosphono oder Triniederalkylphosphono.

Gegebenenfalls benzokondensiertes Niederalkylencarbamoyl ist beispielsweise Azaridino- oder 2-Methylazaridinocarbonyl, 2,3-Dihydoindolin-1-ylcarbamoyl, Pyrrolidinocarbonyl, Piperidinocarbonyl, Morpholinocarbonyl, Thiomorpholinocarbonyl, Piperazinocarbonyl oder N'-Niederalkyl-, wie N'-Methylpiperazinocarbonyl.

Gegebenenfalls durch Oxo substituiertes Azacycloalkylcarbamoyl ist beispielsweise 3-Aza-2-oxo-cycloheptylcarbamoyl.

Gegebenenfalls partiell hydriertes Naphthylcarbamoyl ist beispielsweise Naphthylcarbamoyl oder 5,6-7,8-Tetrahydronaphthylcarbamoyl.

Gegebenenfalls verestertes Carboxyniederalkenylcarbamoyl ist beispielsweise Carboxyniederalkenylcarbamoyl oder Niederalkoxycarbonylniederalkenylcarbamoyl.

Gegebenenfalls verestertes Mono- oder Dicarboxyniederalkylcarbamoyl ist beispielsweise Carboxyniederalkylcarbamoyl, Niederalkoxycarbonylniederalkylcarbamoyl, Dicarboxyniederalkylcarbamoyl oder Diniederalkoxycarbonylniederalkylcarbamoyl.

Vor- und nachstehend sind unter niederen Resten und Verbindungen beispielsweise solche zu verstehen, die bis und mit 7, insbesondere bis und mit 4, C-Atome enthalten.

Diniederalkylamino ist beispielsweise N,N-Di-C₁-C₇-Alkylamino, vorzugsweise N,N-Di-C₁-C₄-Alkylamino, wie insbesondere Dimethylamino, oder in zweiter Linie Diethylamino, Dipropylamino, Diisopropylamino oder Dibutylamino.

Aminoniederalkylaminoniederalkylcarbamoyl ist beispielsweise N-(Amino-C₂-C₄-alkylamino-C₁-C₄-alkyl)carbamoyl, wie N-[2-(2-Aminoethylamino)ethyl]carbamoyl.

Aminoniederalkylaminoniederalkylencarbamoyl ist beispielsweise Amino-C₂-C₄-alkylaminopiperidinocarbonyl, wie 4-(2-Aminoethylamino)piperidinocarbonyl.

Carboxyniederalkoxycarbonyl ist beispielsweise Carboxy-C₁-C₄-alkoxycarbonyl, wie Carboxymethoxycarbonyl, 2-Carboxyethoxycarbonyl, 3-Carboxypropyloxycarbonyl oder 4-Carboxybutyloxycarbonyl.

Carboxyniederalkenylcarbamoyl ist beispielsweise Carboxy-C₂-C₄-alkenylcarbamoyl, wie Carboxyvinylcarbamoyl, 3-Carboxyprop-2-en-2-ylcarbamoyl oder 3-Carboxyprop-2-en-1-ylcarbamoyl.

Carboxyniederalkylcarbamoyl ist beispielsweise Carboxy-C₁-C₄-alkylcarbamoyl, wie Carboxymethylcarbamoyl, 2-Carboxyethylcarbamoyl oder 1-Carboxy-2,2-dimethyl-propylcarbamoyl.

Cyanoniederalkylcarbamoyl ist beispielsweise Cyano-C₁-C₄-alkylcarbamoyl, insbesondere Cyanomethylcarbamoyl.

Cycloalkylcarbamoyl ist beispielsweise N-C₃-C₆-Cycloalkylcarbamoyl, wie Cyclopropylcarbamoyl, Cyclobutylcarbamoyl, Cyclopentylcarbamoyl oder Cyclohexylcarbamoyl, kann aber auch polycyclisches Cycloalkylcarbamoyl, wie Bicyclo[2,2,1]heptyl-, Bicyclo[2,2,2]octyl- oder Adamantylcarbamoyl, sein.

Cycloalkylniederalkylcarbamoyl ist beispielsweise N-(C₃-C₆-Cycloalkyl)-C₁-C₄-alkylcarbamoyl, wie N-(Cyclopropylmethyl)carbamoyl, N-(Cyclobutylmethyl)carbamoyl, N-(Cyclopentylmethyl)carbamoyl oder N-(Cyclohexylmethyl)carbamoyl.

Dihydroxyniederalkyl ist beispielsweise β,γ-Dihydroxy-C₃-C₄-alkyl, wie 2,3-Dihydroxypropyl.

Dihydroxyniederalkylcarbamoyl ist beispielsweise N,N-Di(hydroxy-C₂-C₄-alkyl)carbamoyl, wie N,N-Di(2-hydroxymethyl)carbamoyl, N-(2-Hydroxyethyl)-N-hydroxymethyl-carbamoyl oder N,N-Di(2-hydroxyethyl)carbamoyl.

Dicarboxyniederalkylcarbamoyl ist beispielsweise Dicarboxy-C₁-C₄-alkylcarbamoyl, wie Di-carboxymethylcarbamoyl.

Diniederalkoxyniederalkylcarbamoyl ist beispielsweise Di-C₁-C₄-alkoxy-C₁-C₄-alkylcarbamoyl, wie Dimethoxymethoxycarbonyl oder Diethoxymethoxycarbonyl.

Diniederalkylaminoniederalkylcarbamoyl ist beispielsweise N,N-Di-C₁-C₄-Alkylamino-C₂-C₄-alkylcarbamoyl, wie N-(2-Dimethylaminoethyl)carbamoyl.

Diniederalkylcarbamoyl ist beispielsweise N,N-Di-C₁-C₇-Alkylcarbamoyl, vorzugsweise N,N-Di-C₁-C₄-Alkylcarbamoyl, wie insbesondere Dimethylcarbamoyl, oder in zweiter Linie Diethylcarbamoyl, Dipropylcarbamoyl, Diisopropylcarbamoyl oder Dibutylcarbamoyl.

Diniederalkoxycarbonylniederalkylcarbamoyl ist beispielsweise Di-C₁-C₄-alkoxycarbonyl-C₁-C₄-alkylcarbamoyl, wie Dimethoxycarbonylmethylcarbamoyl oder Diethoxycarbonylmethylcarbamoyl.

Diniederalkylphosphono ist beispielsweise Di-C₁-C₇-Alkylphosphono, vorzugsweise Di-C₁-C₄-Alkylphosphono, wie insbesondere Dimethylphosphono, oder in zweiter Linie Diethylphosphono, Dipropylphosphono, Diisopropylphosphono oder Dibutylphosphono.

Halogen ist beispielsweise Halogen der Atomnummer bis und mit 35, wie Chlor oder Fluor, ferner Brom.

Hydroxyniederalkylcarbamoyl ist beispielsweise N-(Hydroxy-C₂-C₄-alkyl)carbamoyl, wie Hydroxymethylcarbamoyl oder 2-Hydroxyethylcarbamoyl.

Heteroarylcarbamoyl ist beispielsweise Fur-2-ylcarbamoyl, Thien-2-ylcarbamoyl, Thiazol-2-ylcarbamoyl oder N-Benzthiazol-2-ylcarbamoyl.

Heteroarylniederalkylcarbamoyl ist beispielsweise Fur-2-yl-C₁-C₄-alkylcarbamoyl, Thien-2-yl-C₁-C₄-alkylcarbamoyl oder Thiazol-2-yl-C₁-C₄-alkylcarbamoyl, wie Fur-2-ylmethylcarbamoyl, Thien-2-ylmethylcarbamoyl, Thiazol-2-ylmethylcarbamoyl oder N-(3-Oxo-2,3-dihydro-4H-1,4-benzoxazin-7-yl)methylcarbamoyl.

N-Niederalkanoyl-N-niederalkyl-amino ist beispielsweise N-C₁-C₇-Alkanoyl-N-C₁-C₄-alkylamino, insbesondere N-C₁-C₄-Alkanoyl-N-C₁-C₄-alkylamino, wie N-Formyl-N-methylamino, N-Acetyl-N-methyl-amino, N-Acetyl-N-ethyl-amino, N-Ethyl-N-propionyl-amino, N-MethylN-propionyl-amino, N-Butyryl-N-methyl-amino oder N-lsobutyryl-N-methyl-amino.

Niederalkanoyl ist beispielsweise N-C₁-C₇-Alkanoyl, insbesondere N-C₁-C₄-Alkanoyl, wie Formyl, Acetyl, Propionyl, Butyryl oder Isobutyryl, kann aber auch C₅-C₇Alkanoyl, wie Pivaloyl, sein.

Niederalkanoylamino ist beispielsweise N-C₁-C₇-Alkanoylamino, insbesondere N-C₁-C₄-Alkanoylamino, wie Formylamino, Acetylamino, Propionylamino, Butyrylamino oder Isobutyrylamino, kann aber auch C₅-C₇-Alkanoylamino, wie Pivaloylamino, sein.

Niederalkanoyloxy ist beispielsweise N-C₁-C₇-Alkanoyloxy, insbesondere N-C₁-C₄-Alkanoyloxy, wie Formyloxy, Acetoxy, Propionyloxy, Butyryloxy oder Isobutyryloxy, kann aber auch C₅-C₇-Alkanoyloxy, wie Pivaloyloxy, sein.

Niederalkanoyloxyniederalkoxycarbonyl ist beispielsweise N-C₁-C₄-Alkanoyloxy-C₁-C₄-alkoxycarbonyl, wie Acetoxymethoxycarbonyl, Propionyloxymethoxycarbonyl, Tertiärbutyryloxymethoxycarbonyl oder Pivaloyloxymethoxycarbonyl.

Niederalkenylcarbamoyl ist beispielsweise C₂-C₄-Alkenylcarbamoyl, wie Allylcarbamoyl.

Niederalkoxycarbonylniederalkoxycarbonyl ist beispielsweise C₁-C₄-Alkoxycarbonyl-C₁-C₄-alkoxycarbonyl, wie Methoxycarbonylmethoxycarbonyl, Ethoxycarbonylmethoxycarbonyl oder 2-Methoxycarbonylethoxycarbonyl.

Niederalkoxycarbonylniederalkenylcarbamoyl ist beispielsweise C₁-C₄-Alkoxycarbonyl-C₂-C₄-alkenylcarbamoyl, wie Methoxycarbonylvinylcarbamoyl, Ethoxycarbonylvinylcarbamoyl, 3-Methoxycarbonylprop-2-en-2-ylcarbamoyl, 3-Ethoxycarbonylprop-2-en-2-ylcarbamoyl, 3-Methoxycarbonylprop-2-en-1-ylcarbamoyl oder 3-Ethoxycarbonylprop-2-en-1-ylcarbamoyl.

Niederalkoxycarbonylniederalkylcarbamoyl ist beispielsweise C₁-C₄-Alkoxycarbonyl-C₁-C₄-alkylcarbamoyl, wie Methoxycarbonylmethylcarbamoyl, Ethoxycarbonylmethylcarbamoyl, 2-Methoxycarbonylethylcarbamoyl oder 1-Methoxycarbonyl-2,2-dimethyl-propylcarbamoyl.

Niederalkoxyniederalkylcarbamoyl ist beispielsweise C₁-C₄-AlkOxy-C₁-C₄-alkylcarbamoyl, wie Methoxymethylcarbamoyl, 2-Methoxyethylcarbamoyl oder Ethoxymethylcarbamoyl.

Niederalkenyloxy ist beispielsweise C₃-C₄-Alkenyloxy, wie Allyloxy oder Methallyloxy.

N-Niederalkenyloxycarbamoyl ist beispielsweise N-C₂-C₄-alkenyloxycarbamoyl, wie N-Vinyloxycarbamoyl, N-Allyloxycarbamoyl oder N-Methallyloxycarbamoyl.

Niederalkinyloxy ist beispielsweise C₃-C₄-Alkinyloxy, wie Propargyloxy.

Niederalkoxy ist beispielsweise C₁-C₇-Alkoxy, vorzugsweise C₁-C₄-Alkoxy, wie Methoxy, Ethoxy, Propyloxy, Isopropyloxy oder Butyloxy, kann aber auch Isobutyloxy, Sekundärbutyloxy, Tertiärbutyloxy oder eine C₅-C₇-Alkoxy-, wie Pentyloxy-, Hexyloxy- oder Heptyloxygruppe sein.

N-Niederalkenyloxycarbamoyl ist beispielsweise N-C₂-C₄-alkenyloxycarbamoyl, wie N-Vinyloxycarbamoyl, N-Allyloxycarbamoyl oder N-Methallyloxycarbamoyl.

Niederalkoxycarbonyl ist beispielsweise C₁-C₇-Alkoxycarbonyl, vorzugsweise C₁-C₄-Alkoxycarbonyl, wie Methoxycarbonyl, Ethoxycarbonyl, Propyloxycarbonyl, Isopropyloxycarbonyl oder Butyloxycarbonyl, kann aber auch Isobutyloxycarbonyl, Sekundärbutyloxycarbonyl, Tertiärbutyloxycarbonyl oder eine Pentyloxycarbonyl-, Hexyloxycarbonyl- oder Heptyloxycarbonylgruppe sein.

Niederalkyl ist beispielsweise C₁-C₇-Alkyl, vorzugsweise C₁-C₄-Alkyl, wie insbesondere Methyl oder in zweiter Linie Ethyl, Propyl, Isopropyl oder Butyl, kann aber auch Isobutyl, Sekundärbutyl, Tertiärbutyl oder eine C₅-C₇-Alkyl-, wie Pentyl-, Hexyl- oder Heptylgruppe sein.

Niederalkylamino ist beispielsweise N-C₁-C₇-Alkylamino, vorzugsweise N-C₁-C₄-Alkylamino, wie insbesondere Methylamino oder in zweiter Linie Ethylamino, Propylamino, Isopropylamino oder Butylamino, kann aber auch Isobutylamino, Sekundärbutylamino, Tertiärbutylamino oder eine C₅-C₇-Alkylamino-, wie Pentylamino-, Hexylamino-oder Heptylaminogruppe sein.

Niederalkylcarbamoyl ist beispielsweise N-C₁-C₇-Alkylcarbamoyl, vorzugsweise N-C₁-C₄-Alkylcarbamoyl, wie insbesondere Methylcarbamoyl oder in zweiter Linie Ethylcarbamoyl, Propylcarbamoyl, Isopropylcarbamoyl oder Butylcarbamoyl, kann aber auch Isobutylcarbamoyl, Sekundärbutylcarbamoyl, Tertiärbutylcarbamoyl oder eine C₅-C₇-Alkylcarbamoyl-, wie Pentylcarbamoyl-, Hexylcarbamoyl- oder Heptylcarbamoylgruppe sein.

Niederalkylen kann geradkettig oder verzweigt sowie in beliebiger Stellung gebunden sein und ist beispielsweise geradkettiges oder verzweigtes C₁-C₇-Alkylen, vorzugsweise C₁-C₄-Alkylen, wie Methylen, 1,2-Ethylen, 1,3- oder 1,2-Propylen, 1,4-, 1,3- oder 2,3-Butylen oder in zweiter Linie 1,5-, 1,4- oder 2,5-Pentylen.

Niederalkyliden kann geradkettig oder verzweigt sowie geminal in beliebiger Stellung gebunden sein und ist beispielsweise geradkettiges oder verzweigtes C₁-C₇-Alkylen, vorzugsweise C₁-C₄-Alkylen, wie Methylen, 1,1-Ethyliden, 1,1-oder 2,2- Propyliden, 1,1- oder 2,2-Butyliden oder in zweiter Linie 1,1-oder 2,2-Pentyliden.

N-Niederalkyl-N-phenyl-carbamoyl ist beispielsweise N-C₁-C₄-Alkyl-N-phenyl-carbamoyl, wie insbesondere N-Methyl-N-phenyl-carbamoyl oder in zweiter Linie N-Ethyl-N-phenyl-carbamoyl, N-Propyl-N-phenyl-carbamoyl, N-lsopropyl-N-phenyl-carbamoyl oder N-Butyl-N-phenyl-carbamoyl, kann aber auch N-Isobutyl-N-phenyl-carbamoyl, N-Sekundärbutyl-N-phenyl-carbamoyl oder N-Tertiärbutyl-N-phenyl-carbamoyl sein.

Niederalkylphosphono ist beispielsweise C₁-C₇-Alkylphosphono, vorzugsweise C₁-C₄-Alkylphosphono, wie insbesondere Methylphosphono oder in zweiter Linie Ethylphosphono, Propylphosphono, Isopropylphosphono oder Butylphosphono, kann aber auch Isobutylphosphono, Sekundärbutylphosphono, Tertiärbutylphosphono oder eine C₅-C₇-Alkylphosphono-, wie Pentylphosphono-, Hexylphosphono- oder Heptylphosphonogruppe sein.

Oxaniederalkylenaminoniederalkylcarbamoyl ist beispielsweise N-(Morpholino-C₂-C₄-alkyl)carbamoyl, wie insbesondere N-(2-Morpholinoethyl)ethylcarbamoyl.

2-Oxoimidazolidin-1-ylniederalkylencarbamoyl ist beispielsweise N-(2-Oxoimidazolidin-1-yl-C₄-C₅-alkylen)carbamoyl, wie N-[4-(2-Oxoimidazolidin-1-yl)piperidinocarbonyl.

2-Oxoimidazolidin-1-ylniederalkylcarbamoyl ist beispielsweise N-(2-Oxoimidazolidin-1-yl-C₂-C₅-alkyl)carbamoyl, wie N-(2-Oxoimidazolidin-1-yl)ethylcarbamoyl.

Phenylniederalkoxycarbamoyl ist beispielsweise unsubstituiertes oder durch C₁-C₄-Alkyl, C₁-C₄-Alkoxy, Hydroxy, Halogen und/oder Trifluormethyl substituiertes Phenyl-C₁-C₄-alkoxycarbamoyl, wie Benzyloxycarbamoyl oder 1-Phenylethoxycarbamoyl.

Phenyloxyniederalkoxycarbonyl ist beispielsweise unsubstituiertes oder durch C₁-C₄-Alkyl, C₁-C₄-Alkoxy, Hydroxy, Halogen und/oder Trifluormethyl substituiertes Phenyloxy-C₁-C₄-alkoxycarbonyl, wie Phenyloxymethoxycarbonyl oder 2-Phenyloxyethoxycarbonyl.

N-Phenylniederalkenyloxycarbamoyl ist beispielsweise N-Phenyl-C₂-C₄-alkenyloxycarbamoyl, wie N-Phenylvinyloxycarbamoyl oder N-(3-Phenylprop-2-enyloxy)carbamoyl.

Phenylniederalkoxycarbonyl ist beispielsweise unsubstituiertes oder durch C₁-C₄-Alkyl, C₁-C₄Alkoxy, Hydroxy, Halogen und/oder Trifluormethyl substituiertes Phenyl-C₁-C₄-alkoxy carbonyl, wie Benzyloxycarbonyl oder 1-Phenylethoxycarbonyl.

Phenylniederalkylcarbamoyl ist beispielsweise unsubstituiertes oder durch C₁-C₄-Alkyl, C₁-C₄-Alkoxy, Hydroxy, Halogen und/oder Trifluormethyl substituiertes Phenyl-C₁-C₄-alkylcarbamoyl, wie Benzylcarbamoyl oder 2-Phenylethylcarbamoyl.

Triniederalkylphosphono ist beispielsweise Tri-C₁-C₇-Alkylphosphono, vorzugsweise Tri-C₁-C₄-Alkylphosphono, wie insbesondere Trimethylphosphono, oder in zweiter Linie Triethylphosphono, Tripropylphosphono, Triisopropylphosphono oder Tributylphosphono.

Verbindungen der Formel I, die saure Gruppen aufweisen, beispielsweise solche in denen mindestens einer der Reste R₁, R₂ und R₄ Carboxy, Phosphono oder Tetrazolyl ist oder aufweist, können Salze mit Basen bilden. Verbindungen der Formel I können ferner Säureadditionssalze bilden.

Salze von Verbindungen der Formel I mit Basen sind beispielsweise deren Salze mit pharmazeutisch verwendbaren Basen, wie nicht-toxische, von Metallen der Gruppen la, Ib, IIa und IIb abgeleitete Metallsalze, z.B. Alkalimetall-, insbesondere Natrium- oder Kaliumsalze, Erdalkalimetall-, insbesondere Calcium- oder Magnesiumsalze, ebenso Ammoniumsalze mit Ammoniak oder organischen Aminen oder quaternären Ammoniumbasen, wie gegebenenfalls C-hydroxylierten aliphatischen Aminen, insbesondere Mono-, Di- oder Triniederalkylaminen, z.B. Methyl-, Ethyl- oder Diethylamin, Mono-, Di- oder Tri-(hydroxyniederalkyl)aminen, wie Ethanolamin, Diethanolamin oder Triethanolamin, Tris(hydroxymethyl)methylamin oder 2-Hydroxytertiärbutylamin, oder N-(Hydroxyniederalkyl)-N,N-diniederalkyl-aminen bzw. N-(Polyhydroxyniederalkyl)-N-niederalkylaminen, wie 2-(Dimethylamino)ethanol oder D-Glucamin oder Cholin, oder quaternären aliphatischen Ammoniumhydroxiden, z.B. Tetrabutylammoniumhydroxid.

Säureadditionssalze von Verbindungen der Formel I sind beispielsweise deren pharmazeutisch verwendbare Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, oder Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate).

Umfaßt sind sowohl vollständige als auch partielle Salze, d.h. Salze mit 1, 2 oder 3, vorzugsweise 2, Äquivalenten Base pro Mol Säure der Formel I bzw. Salze mit 1, 2 oder 3 Äquivalenten, vorzugsweise 1 Äquivalent, Säure pro Mol Base der Formel I.

Zur Isolierung oder Reinigung können auch pharmazeutisch ungeeignete Salze Verwendung finden. Zur therapeutischen Anwendung gelangen nur die pharmazeutisch verwendbaren, nicht-toxischen Salze, die deshalb bevorzugt sind.

Die Verbindungen der Formel I weisen wertvolle pharmakologische Eigenschaften auf. Sie besitzen eine selektive nicht-kompetetive antagonistische Wirkung gegenüber N-Methyl-D-asparaginsäure-sensitiven (NMDA-sensitiven) excitatorischen Aminosäurerezeptoren von Warmblütern. Insbesondere vermögen sie an Strychnin-insensitive Glycinmodulatoren des NMDA-Rezeptors zu binden. Das Bindungsvermögen der erfindungsgemäss bereitgestellten Verbindungen und ihrer Salze an Strychnin-insensitive Glycinbindungsstellendes NMDA-Rezeptors kann in vitro beispielsweise in der Versuchsanordnung nach Baron et al., Eur. J. Pharmacol., Molec. Pharmacol. Section 206, Seiten 149-154 (1991) und Canton et al., J. Pharm. Pharmacol. 44, Seiten 812-816 (1992) an Rattenkortex- und Rattenhippocampusmembranen ermittelt werden. Dabei wird bestimmt, in welchem Ausmaß [³H]- 5,7-Dichlorkynurensäure (³H-DCKA) verdrängt wird, wobei die prozentuale Hemmung und gegebenenfalls bei mehreren Konzentrationen die für eine 50 %-iger Verdrängung erforderliche Konzentration (IC₅₀) bestimmt wird. Die für eine 50 %-ige Verdrängung erforderliche Konzentration (IC₅₀) liegt im Nano- und unteren Millimolbereich, d.h. bei Konzentrationen von etwa 0.01 bis 10 µMol.

Auf Grund dieser Eigenschaften sind die Verbindungen der Formel I und ihre pharmazeutisch verwendbaren Salze vorzüglich geeignet zur prophylaktischen und therapeutischen Behandlung von pathologischen Zuständen, die auf Glycin-antagonistische Blockierung von NMDA-sensitiven Rezeptoren ansprechen, beispielsweise von neurodegenerativen Erkrankungen, wie solcher im Gefolge von Schlaganfall, Hypoglykämie, Anoxie oder Hirnlähmungserscheinungen, von ischämischen Hirnerkrankungen, wie der cerebralen Ischämie, der cerebralen Ischämie bei Herzchirurgie oder Herzstillstand, perinataler Aphyxie, epileptischer Anfälle, chorea Huntington, Alzheimer'scher und Parkinson'scher Erkrankung, amyotroper Lateralsklerose, Rückenmark- und Hirntrauma sowie Vergiftungserscheinungen durch Neurotoxine oder Suchtmittelmissbrauch, und von ischämischen Erkrankungen des Auges, von Gefäß- und Muskelspasmen, wie von Migräne oder von lokaler oder generaler Spastizität, von Konvulsionen, wie der Epilepsie, und von Angst- und Schmerzzuständen, wie Trigeminusneuralgien.

Die antikonvulsiven Eigenschaften der erfindungsgemäßen Verbindungen können in vivo beispielsweise an der Maus anhand ihrer ausgeprägten Schutzwirkung gegenüber durch Elektroschock oder Metrazol ausgelösten Konvulsionen bestimmt werden, wobei man z.B. das etablierte Elektroschock-Mausmodell bzw. das Mausmodell für Metrazol-induzierte Konvulsionen gemäß Schmutz et al., Naunyn-Schmiedeberg's Arch. Pharmacol. 342, 61-66 (1990) heranziehen kann.

Die Erfindung betrifft beispielsweise Verbindungen der Formel I, worin
A₁ Methylen oder eine Gruppe der Formel >CH-A₄-R₄ (la) darstellt,
A₂ Niederalkyliden und A₃ Oxy, Thio, Sulfinyl, Sulfonyl oder Carbonyl ist,
oder
A₂ Carbonyl und A₃ Oxy, Thio, Sulfinyl oder Sulfonyl bedeutet,
A₄ für Niederalkylen steht,
n für 0 oder 1 steht,
R₁ und R₂ unabhängig voneinander Wasserstoff Amino, Niederalkylamino, Niederalkanoylamino, Diniederalkylamino, N-Niederalkanoyl-N-niederalkyl-amino, Hydroxy, Niederalkoxy, Niederalkenyloxy, Niederalkinyloxy, Niederalkyl, Polyhalogenniederalkyl oder Halogen bedeuten,
R₃ Wasserstoff bedeutet und
R₄ Wasserstoff, Carboxy, Niederalkoxycarbonyl, unsubstituiertes oder durch Niederalkyl, Niederalkoxy, Hydroxy, Halogen und/oder Trifluormethyl substituiertes Phenylniederalkoxycarbonyl, Carbamoyl, Cyano oder Niederalkylcarbamoyl, unsubstituiertes oder durch Niederalkyl, Niederalkoxy, Hydroxy, Halogen und/oder Trifluormethyl substituiertes Phenylcarbamoyl oder Diniederalkylcarbamoyl darstellt,
und ihre Salze.

Die Erfindung betrifft beispielsweise ebenso Verbindungen der Formel I,
worin A₁ Niederalkyliden oder eine Gruppe der Formel >CH-A₄-R₄ (Ia) darstellt,
A₂ Niederalkyliden oder eine Gruppe der Formel >CH-A₄-R₄ (la) oder >C=O (Ib) ist,
A₃ Oxy oder gegebenenfalls oxidiertes Thio bedeutet,
A₄ für Niederalkylen steht,
n für 0 oder 1 steht,
R₁ und R₂ unabhängig voneinander Wasserstoff, gegebenenfalls durch Niederalkyl und/oder Niederalkanoyl substituiertes Amino, gegebenenfalls mit einem Niederalkanol verethertes Hydroxy, gegebenenfalls halogeniertes Niederalkyl oder Halogen bedeuten,
R₃ Wasserstoff, Hydroxy oder gegebenenfalls durch Niederalkanoyl substituiertes Amino bedeutet und
R₄ Wasserstoff, Cyano, Tetrazolyl oder gegebenenfalls verestertes oder amidiertes Carboxy darstellt, wie solche,
worin
A₁ eine Gruppe der Formel >CH-A₄-R₄ (la) darstellt,
A₂ Niederalkyliden bedeutet,
A₃ Oxy, gegebenenfalls oxidiertes Thio oder Carbonyl ist,
A₄ für Niederalkylen steht,
R₁ und R₂ unabhängig voneinander Wasserstoff, gegebenenfalls durch Niederalkyl und/oder Niederalkanoyl substituiertes Amino, gegebenenfalls mit einem Niederalkanol verethertes Hydroxy, gegebenenfalls halogeniertes Niederalkyl oder Halogen bedeuten,
R₃ Wasserstoff oder Hydroxybedeutet und
R₄ Wasserstoff, Cyano oder gegebenenfalls verestertes oder amidiertes Carboxy darstellt, und ihre Salze.

Die Erfindung betrifft in erster Linie Verbindungen der Formel I,
worin
A₁ Niederalkyliden oder eine Gruppe der Formel >CH-A₄-R₄ (Ia) darstellt,
A₂ Niederalkyliden oder eine Gruppe der Formeln >CH-A₄-R₄ (la), >C=O (lb) oder >CH(OH)-A₅-R₄ (Ic) ist,
A₃ Oxy, Thio, Sulfinyl oder Sulfonyl oder eine Gruppe >C(=O) (Ib) bedeutet,
A₄ für Niederalkylen steht,
A₅ Niederalkylen oder eine direkte Bindung darstellt,
n für 0 oder 1 steht,
R₁ und R₂ unabhängig voneinander Wasserstoff, Amino, Niederalkylamino, Niederalkanoylamino, Diniederalkylamino, N-Niederalkanoyl-N-niederalkyl-amino, Nitro, Niederalkanoyl, Hydroxy, Niederalkanoyloxy, Niederalkoxy, Niederalkenyloxy, Niederalkinyloxy, Carboxy, Niederalkoxycarbonyl, Carbamoyl, Cyano, Niederalkyl, Polyhalogenniederalkyl oder Halogen bedeuten,
R₃ Wasserstoff oder Hydroxy bedeutet und
R₄ Wasserstoff, Cyano, Carboxy, Niederalkoxycarbonyl, Carboxyniederalkoxycarbonyl, Niederalkoxycarbonylniederalkoxycarbonyl, Niederalkanoyloxyniederalkoxycarbonyl, unsubstituiertes oder durch Niederalkyl, Niederalkoxy, Hydroxy, Halogen und/oder Trifluormethyl substituiertes Phenyloxycarbonyl, Benzoyloxyniederalkoxycarbonyl oder Phenylniederalkoxycarbonyl, Carbamoyl, Niederalkylcarbamoyl, Niederalkenylcarbamoyl, Dinieder-alkylcarbamoyl, Diniederalkylaminoniederalkylcarbamoyl, Aminoniederalkylaminoniederalkylcarbamoyl, 2-Oxoimidazolidin-1-ylniederalkylcarbamoyl, Aminoniederalkylaminoniederalkylencarbamoyl 2-Oxoimidazolidin-1-ylniederalkylencarbamoyl, Oxaniederalkylenaminoniederalkylcarbamoyl, 3- bis 7-gliedriges Cycloalkylcarbamoyl, 3- bis 7-gliedriges Carboxycycloalkylcarbamoyl, 3- bis 7-gliedriges Niederalkoxycarbonylcycloalkylcarbamoyl, 3- bis 7-gliedriges Cycloalkylniederalkylcarbamoyl, 3-Aza-2-oxo-cycloheptylcarbamoyl., Azaridinocarbonyl, 2-Methylazaridinocarbonyl, 2,3-Dihydoindolin-1-ylcarbamoyl, Pyrrolidinocarbonyl, Piperidinocarbonyl, Morpholinocarbonyl, Thiomorpholinocarbonyl, Piperazinocarbonyl, N'-Niederalkylpiperazinocarbonyl, unsubstituiertes oder durch Niederalkyl, Niederalkoxy, Hydroxy, Halogen und/oder Trifluormethyl substituiertes Phenylniederalkylcarbamoyl, unsubstituiertes oder durch Niederalkyl, Niederalkoxy, Hydroxy, Halogen, Amino, Niederalkoxycarbonylamino, Nitro, Carboxy, Niederalkoxycarbonyl, Phenyl, Phenyloxy und/oder Trifluormethyl substituiertes N-Phenylcarbamoyl oder N-Niederalkyl-N-phenylcarbamoyl, Naphthylcarbamoyl, 5,6-7,8-Tetrahydronaphthylcarbamoyl, N-Indanylcarbamoyl, Furyl-2-carbamoyl, Thien-2-ylcarbamoyl, Thiazol-2-ylcarbamoyl, N-Benzthiazol-2-ylcarbamoyl, Fur-2-ylmethylcarbamoyl, Thien-2-ylmethylcarbamoyl, Thiazol-2-ylmethylcarbamoyl, N-(3-Oxo-2,3-dihydro-4H-1,4-benzoxazin-7-yl)methylcarbamoyl, Hydroxyniederalkylcarbamoyl, Mono- oder Dihydroxyniederalkylcarbamoyl, Mono- oder Diniederalkoxyniederalkylcarbamoyl, Polyhalogenniederalkylcarbamoyl, Carboxyniederalkylcarbamoyl, Niederalkoxycarbonylniederalkylcarbamoyl, Dicarboxyniederalkylcarbamoyl, Diniederalkoxycarbonylniederalkylcarbamoyl, Cyanoniederalkylcarbamoyl, Carboxyniederalkenylcarbamoyl oder Niederalkoxycarbonylniederalkenylcarbamoyl, N-Hydroxycarbamoyl, N-Niederalkoxycarbamoyl, N-Niederalkoxy-N-niederalkyl-carbamoyl, N-Niederalkenyloxycarbamoyl oder jeweils unsubstituiertes oder durch Niederalkyl, Niederalkoxy, Hydroxy, Halogen und/oder Trifluormethyl substituiertes N-Phenyloxycarbamoyl, N-Phenylniederalkoxycarbamoyl oder N-Phenylniederalkenyloxycarbamoyl, Phosphono, Niederalkylphosphono, Diniederalkylphosphono oder Triniederalkylphosphono oder 5-Tetrazolyl darstellt, und ihre Salze.

Die Erfindung betrifft vor allem Verbindungen der Formel I,
worin
A₁ geradkettiges oder verzweigtes C₁-C₇-Alkylen, vorzugsweise C₁-C₄-Alkylen, wie Methylen, 1,1-Ethyliden, 1,1-oder 2,2- Propyliden, 1,1- oder 2,2-Butyliden, oder eine Gruppe der Formel >CH-A₄-R₄ (Ia) darstellt,
A₂ geradkettiges oder verzweigtes C₁-C₇-Alkylen, vorzugsweise C₁-C₄-Alkylen, wie Methylen, 1,1-Ethyliden, 1,1-oder2,2-Propyliden, 1,1-oder 2,2-Butyliden, oder eine Gruppe der Formel >CH-A₄-R₄ (Ia) oder >CH(OH)-A₅-R₄ (Ic) ist,
A₃ Oxy, Thio, Sulfinyl oder Sulfonyl bedeutet,
A₄ für geradkettiges oder verzweigtes C₁-C₇-Alkylen, vorzugsweise C₁-C₄-Alkylen, wie Methylen, 1,1-Ethyliden, 1,1- oder 2,2- Propyliden, 1,1- oder 2,2-Butyliden, steht,
A₅ C₁-C₄-Alkylen, wie Methylen, 1,1-Ethyliden, 1,1- oder 2,2- Propyliden, 1,1- oder 2,2-Butyliden, oder eine direkte Bindung darstellt,
n für 0 oder 1 steht,
R₁ und R₂ unabhängig voneinander Wasserstoff, Amino, N-C₁-C₄-Alkylamino, wie Methylamino, Ethylamino, Propylamino, Isopropylamino oder Butylamino, N-C₁-C₇-Alkanoylamino, wie Formylamino, Acetylamino, Propionylamino, Butyrylamino oder Isobutyrylamino, N,N-Di-C₁-C₄-Alkylamino, wie Dimethylamino, Diethylamino, Dipropylamino, Diisopropylamino oder Dibutylamino, N-C₁-C₇-Alkanoyl-N-C₁-C₄-alkylamino, wie N-Formyl-N-methylamino, N-Acetyl-N-methyl-amino, N-Acetyl-N-ethyl-amino, N-Ethyl-N-propionyl-amino, N-Methyl-N-propionylamino, N-Butyryl-N-methyl-amino oder N-lsobutyryl-N-methyl-amino, Nitro, N-C₁-C₄-Alkanoyl, wie Formyl, Acetyl, Propionyl, Butyryl oder Isobutyryl, Pivaloyl, Hydroxy, N-C₁-C₄-Alkanoyloxy, wie Formyloxy, Acetoxy, Propionyloxy, Butyryloxy oder Isobutyryloxy, Pivaloyloxy, C₁-C₄-Alkoxy, wie Methoxy, Ethoxy, Propyloxy, Isopropyloxy, Butyloxy, Isobutyloxy, Sekundärbutyloxy oder Tertiärbutyloxy, C₃-C₄-Alkenyloxy, wie Allyloxy oder Methallyloxy, C₃-C₄-Alkinyloxy, wie Propargyloxy, Carboxy, C₁-C₄-Alkoxycarbonyl, wie Methoxycarbonyl, Ethoxycarbonyl, Propyloxycarbonyl, Isopropyloxycarbonyl, Butyloxycarbonyl, Isobutyloxycarbonyl, Sekundärbutyloxycarbonyl oder Tertiärbutyloxycarbonyl, Carbamoyl, Cyano, C₁-C₄-Alkyl, wie Methyl, Ethyl, Propyl, Isopropyl Butyl, Isobutyl, Sekundärbutyl oder Tertiärbutyl, 17
Trifluormethyl oder Halogen bedeuten,
R₃ Wasserstoff oder Hydroxy bedeutet und
R₄ Wasserstoff, Cyano, Carboxy, C₁-C₇-Alkoxycarbonyl, wie Methoxycarbonyl, Ethoxycarbonyl, Propyloxycarbonyl, Isopropyloxycarbonyl, Butyloxycarbonyl, Isobutyloxycarbonyl, Sekundärbutyloxycarbonyl oder Tertiärbutyloxycarbonyl, Carboxy-C₁-C₄-alkoxycarbonyl, wie Carboxymethoxycarbonyl, 2-Carboxyethoxycarbonyl, 3-Carboxypropyloxycarbonyl oder 4-Carboxybutyloxycarbonyl, C₁-C₄-Alkoxycarbonyl-C₁-C₄-alkoxycarbonyl, wie Methoxycarbonylmethoxycarbonyl, Ethoxycarbonylmethoxycarbonyl oder 2-Methoxycarbonylethoxycarbonyl, N-C₁-C₄-Alkanoyloxy-C₁-C₄-alkoxycarbonyl, wie Acetoxymethoxycarbonyl, Propionyloxymethoxycarbonyl, Tertiärbutyryloxymethoxycarbonyl oder Pivaloyloxymethoxycarbonyl, unsubstituiertes oder durch C₁-C₄-Alkyl, C₁-C₄-Alkoxy, Hydroxy, Halogen und/oder Trifluormethyl substituiertes Phenyloxycarbonyl, Benzoyloxy-C₁-C₄-alkoxycarbonyl oder Phenyl-C₁-C₄-alkoxycarbonyl, wie Benzyloxycarbonyl oder Benzoyloxymethoxycarbonyl, Carbamoyl, N-C₁-C₄-Alkylcarbamoyl, wie Methylcarbamoyl, Ethylcarbamoyl, Propylcarbamoyl, Isopropylcarbamoyl, Butylcarbamoyl, Isobutylcarbamoyl, Sekundärbutylcarbamoyl oder Tertiärbutylcarbamoyl, C₂-C₄-Alkenylcarbamoyl, wie Allylcarbamoyl, N,N-Di-C₁-C₄-Alkylcarbamoyl, wie Dimethylcarbamoyl, oder in zweiter Linie Diethylcarbamoyl, Dipropylcarbamoyl, Diisopropylcarbamoyl oder Dibutylcarbamoyl,, C₃-C₇-Cycloalkylcarbamoyl, wie Cyclopropyl- oder Cyclohexylcarbamoyl, Carboxy-C₃-C₇-cycloalkylcarbamoyl, wie 1-Carboxycarbamoyl, C₁-C₄-Alkoxycarbonyl-C₃-C₇-cycloalkylcarbamoyl, wie 1-Methoxycarbonyl- oder 1-Ethoxycarbonylcyclopropylcarbamoyl, 3- bis 7-gliedriges N-(C₃-C₆-Cycloalkyl)-C₁-C₄-alkylcarbamoyl, wie N-(Cyclopropylmethyl)carbamoyl, N-(Cyclobutylmethyl)carbamoyl, N-(Cyclopentylmethyl)carbamoyl oder N-(Cyclohexylmethyl)carbamoyl, 3-Aza-2-oxo-cycloheptylcarbamoyl., Azaridinocarbonyl, 2-Methylazaridinocarbonyl, 2,3-Dihydoindolin-1-ylcarbamoyl, Pyrrolidinocarbonyl, Piperidinocarbonyl, Morpholinocarbonyl, Thiomorpholinocarbonyl, Piperazinocarbonyl, N'- C₁-C₄-Alkyl-, wie N'-Methylpiperazinocarbonyl, unsubstituiertes oder C₁-C₄-Alkyl, C₁-C₄-Alkoxy, Hydroxy, Halogen und/oder Trifluormethyl substituiertes Phenyl-C₁-C₄-alkylcarbamoyl, wie Benzylcarbamoyl oder 2-Phenylethylcarbamoyl, unsubstituiertes oder durch C₁-C₄-Alkyl, C₁-C₄-Alkoxy, Hydroxy, Halogen, Trifluormethyl, Amino, C₁-C₄-Alkoxycarbonylamino, wie Methoxy- oder Ethoxycarbonylamino, Nitro, Carboxy, C₁-C₄-Alkoxycarbonyl, wie Methoxy- oder Ethoxycarbonyl, Phenyl, Phenyloxy und/oder Trifluormethyl substituiertes N-Phenylcarbamoyl oder N-C₁-C₄-Alkyl-N-phenylcarbamoyl, wie N-Methyl-N-phenylcarbamoyl, Naphthylcarbamoyl, 5,6-7,8-Tetrahydronaphthylcarbamoyl, Indanylcarbamoyl, Furyl-2-carbamoyl, Thien-2-ylcarbamoyl, Thiazol-2-ylcarbamoyl, N-Benzthiazol-2-ylcarbamoyl, Fur-2-ylmethylcarbamoyl, Thien-2-ylmethylcarbamoyl, Thiazol-2-ylmethylcarbamoyl, N-(3-Oxo-2,3-dihydro-4H-1,4-benzoxazin-7-yl)methylcarbamoyl, N-(Hydroxy-C₂-C₄-alkyl)carbamoyl, wie Hydroxymethylcarbamoyl oder 2-Hydroxyethylcarbamoyl, N,N-Di(hydroxy-C₂-C₄-alkyl)carbamoyl, wie N,N-Di(2-hydroxyethyl)carbamoyl, C₁-C₄-Alkoxy-C₁-C₄-alkylcarbamoyl, wie Methoxymethylcarbamoyl, 2-Methoxyethylcarbamoyl oder Ethoxymethylcarbamoyl, Di-C₁-C₄-alkoxy-C₁-C₄-alkylcarbamoyl, wie Dimethoxymethylcarbamoyl oder Diethoxymethylcarbamoyl, Polyhalogen-C₂-C₄-alkylcarbamoyl, wie 2,2,2,-Trifluorethylcarbamoyl, Carboxy-C₁-C₄-alkylcarbamoyl, wie Carboxymethylcarbamoyl, 2-Carboxyethylcarbamoyl oder 1-Carboxy-2,2-dimethylpropylcarbamoyl, C₁-C₄-Alkoxycarbonyl-C₁-C₄-alkylcarbamoyl, wie Methoxycarbonylmethylcarbamoyl, Ethoxycarbonylmethylcarbamoyl, 2-Methoxycarbonylethylcarbamoyl oder 1-Methoxycarbonyl-2,2-dimethyl-propylcarbamoyl, Dicarboxy-C₁-C₄-alkylcarbamoyl, wie Dicarboxymethylcarbamoyl, oder Di-C₁-C₄-alkoxycarbonyl-C₁-C₄-alkylcarbamoyl, wie Dimethoxycarbonylmethylcarbamoyl oder Diethoxycarbonylmethylcarbamoyl,, Cyano-C₁-C₄-alkylcarbamoyl, insbesondere Cyanomethylcarbamoyl, Carboxy-C₂-C₄-alkenylcarbamoyl, wie Carboxyvinylcarbamoyl, 3-Carboxyprop-2-en-2-ylcarbamoyl oder 3-Carboxyprop-2-en-1-ylcarbamoyl, C₁-C₄-Alkoxycarbonyl-C₂-C₄-alkenylcarbamoyl, wie Methoxycarbonylvinylcarbamoyl, Ethoxycarbonylvinylcarbamoyl, 3-Methoxycarbonylprop-2-en-2-ylcarbamoyl, 3-Ethoxycarbonylprop-2-en-2-ylcarbamoyl, 3-Methoxycarbonylprop-2-en-1-ylcarbamoyl oder 3-Ethoxycarbonylprop-2-en-1-ylcarbamoyl, N-Hydroxycarbamoyl, N-C₁-C₄-Alkoxycarbamoyl, wie Methoxycarbamoyl, Ethoxycarbamoyl, Propyloxycarbamoyl, Iso-propyloxycarbamoyl, Butyloxycarbamoyl oder insbesondere Tertiärbutyloxycarbamoyl, N-C₂-C₄-alkenyloxycarbamoyl, wie N-Vinyloxycarbamoyl, N-Allyloxycarbamoyl oder N-Methallyloxycarbamoyl oder unsubstituiertes oder jeweils durch C₁-C₄-Alkyl, C₁-C₄Alkoxy, Hydroxy, Halogen und/oder Trifluormethyl substituiertes N-Phenyloxycarbamoyl, N-Phenyl-C₁-C₄-alkoxycarbamoyl, wie Benzyloxycarbamoyl oder 1-Phenylethoxycarbamoyl, oder N-Phenyl-C₂-C₄-alkenyloxycarbamoyl, wie N-Phenylvinyloxycarbamoyl oder N-(3-Phenylprop-2-enyloxy)carbamoyl, Phosphono, C₁-C₄-Alkylphosphono, wie Methylphosphono, Ethylphosphono, Propylphosphono, Isopropylphosphono oder Butylphosphono, Di-C₁-C₄-Alkylphosphono, wie insbesondere Dimethylphosphono, Diethylphosphono, Dipropylphosphono, Diisopropylphosphono oder Dibutylphosphono, Tri-C₁-C₄-Alkylphosphono, wie insbesondere Trimethylphosphono, oder in zweiter Linie Triethylphosphono, Tripropylphosphono, Triisopropylphosphono oder Tributylphosphono, oder 5-Tetrazolyl darstellt,
und ihre Salze.

Die Erfindung betrifft vor allem beispielsweise Verbindungen der Formel I,
worin
A₁ Methylen oder eine Gruppe der Formel >CH-A₄-R₄ (Ia) darstellt,
A₂ geradkettiges oder verzweigtes C₁-C₇-Alkyliden, wie Methylen, 1,1-Ethyliden, 1,1- oder 2,2- Propyliden, 1,1- oder 2,2-Butyliden oder in zweiter Linie 1,1- oder 2,2-Pentyliden ist,
A₃ Oxy, Thio, Sulfinyl oder Sulfonyl bedeutet,
A₄ für geradkettiges oder verzweigtes C₁-C₇-Alkylen, wie Methylen, 1,2-Ethylen, 1,3- oder 1,2-Propylen, 1,4-, 1,3- oder 2,3-Butylen oder 1,5-, 1,4- oder 2,5-Pentylen, steht,
n für 0 oder 1 steht,
R₁ und R₂ unabhängig voneinander Wasserstoff Amino, N-C₁-C₇-Alkylamino, wie Methylamino, Ethylamino, Propylamino, Isopropylamino oder Butylamino, ferner Isobutylamino, Sekundärbutylamino, Tertiärbutylamino oder eine C₅-C₇-Alkylamino-, wie Pentylamino-, Hexylamino-oder Heptylaminogruppe, N-C₁-C₇-Alkanoylamino, wie Formylamino, Acetylamino, Propionylamino, Butyrylamino oder Isobutyrylamino, ferner C₅-C₇-Alkanoylamino, wie Pivaloylamino, N,N-Di-C₁-C₇-Alkylamino, wie Dimethylamino, Diethylamino, Dipropylamino, Diisopropylamino oder Dibutylamino, N-C₁-C₇-Alkanoyl-N-C₁-C₄-alkylamino, wie N-Formyl-N-methyl-amino, N-Acetyl-N-methyl-amino, N-Acetyl-N-ethyl-amino, N-Ethyl-N-propionyl-amino, N-Methyl-N-propionyl-amino, N-Butyryl-N-methyl-amino oder N-lsobutyryl-N-methyl-amino, Hydroxy, C₁-C₇-Alkoxy, wie Methoxy, Ethoxy, Propyloxy, Isopropyloxy oder Butyloxy, ferner Isobutyloxy, Sekundärbutyloxy oder Tertiärbutyloxy, C₃-C₄-Alkenyloxy, wie Allyloxy oder Methallyloxy, C₃-C₄-Alkinyloxy, wie Propargyloxy, C₁-C₇-Alkyl, wie Methyl, Ethyl, Propyl, Isopropyl oder Butyl, ferner Isobutyl, Sekundärbutyl, Tertiärbutyl oder eine C₅-C₇-Alkyl-, wie Pentyl-, Hexyl- oder Heptylgruppe, Trifluormethyl oder Halogen bedeuten,
R₃ Wasserstoff oder Hydroxy bedeutet und
R₄ Wasserstoff, Carboxy, C₁-C₇-Alkoxycarbonyl, wie Methoxycarbonyl, Ethoxycarbonyl, Propyloxycarbonyl, Isopropyloxycarbonyl oder Butyloxycarbonyl, aber auch Isobutyloxycarbonyl, Sekundärbutyloxycarbonyl, Tertiärbutyloxycarbonyl oder eine Pentyloxycarbonyl-, Hexyloxycarbonyl- oder Heptyloxycarbonylgruppe, unsubstituiertes oder durch C₁-C₄-Alkyl, C₁-C₄-Alkoxy, Hydroxy, Halogen und/oder Trifluormethyl substituiertes Phenyl-C₁-C₄-alkoxycarbonyl, Carbamoyl, Tetrazolyl, Cyano, C₁-C₄-Alkylcarbamoyl, unsubstituiertes oder durch C₁-C₄-Alkyl, C₁-C₄-Alkoxy, Hydroxy, Halogen und/oder Trifluormethyl substituiertes Phenylcarbamoyl oder N,N-Di-C₁-C₇-Alkylcarbamoyl, wie Dimethylcarbamoyl, Diethylcarbamoyl, Dipropylcarbamoyl, Diisopropylcarbamoyl oder Dibutylcarbamoyl, darstellt, und ihre Salze.

Die Erfindung betrifft insbesondere Verbindungen der Formel I,
worin
einer der Rest A₁ und A₂ eine Gruppe der Formel >CH-A₄-R₄ (la) und der andere geradkettiges oder verzweigtes C₁-C₄-Alkylen, wie Methylen, 1,1-Ethyliden, 1,1-oder 2,2-Propyliden, 1,1- oder 2,2-Butyliden, darstellt,
A₃ Oxy, Thio, Sulfinyl oder Sulfonyl bedeutet,
A₄ für geradkettiges oder verzweigtes C₁-C₄-Alkylen, wie Methylen, 1,1-Ethyliden, 1,1- oder 2,2- Propyliden, 1,1- oder 2,2-Butyliden, steht,
n für 0 steht,
R₁ und R₂ unabhängig voneinander Wasserstoff, Nitro, N-C₁-C₄-Alkanoyl, wie Formyl, Acetyl, Propionyl, Butyryl oder Isobutyryl, Pivaloyl, Hydroxy, C₁-C₄-Alkoxy, wie Methoxy, Carboxy, C₁-C₄-Alkoxycarbonyl, wie Methoxycarbonyl oder Ethoxycarbonyl, Carbamoyl, Cyano, C₁-C₄-Alkyl, wie Methyl oder Ethyl, Halogen, Trifluormethyl oder Halogen bedeuten,
R₃ Wasserstoff bedeutet und
R₄ Wasserstoff, Carboxy, C₁-C₇-Alkoxycarbonyl, wie Methoxycarbonyl oder Ethoxycarbonyl, C₁-C₄-Alkoxycarbonyl-C₁-C₄-alkoxycarbonyl, wie Methoxycarbonylmethoxycarbonyl, 1-Ethoxycarbonylmethoxycarbonyl oder 2-Methoxycarbonylethoxycarbonyl, N-C₁-C₄-Alkanoyloxy-C₁-C₄-alkoxycarbonyl, wie Tertiärbutyryloxymethoxycarbonyl, jeweils unsubstituiertes oder durch C₁-C₄-Alkyl, C₁-C₄-Alkoxy, Hydroxy, Halogen und/oder Trifluormethyl substituiertes Phenyloxycarbonyl, Benzoyloxy-C₁-C₄-alkoxycarbonyl oder Phenyl-C₁-C₄-alkoxycarbonyl, wie Benzyloxycarbonyl oder Benzoyloxymethoxycarbonyl, Carbamoyl, N-C₁-C₄-Alkylcarbamoyl, wie Methylcarbamoyl, Butylcarbamoyl oder Tertiärbutylcarbamoyl, C₂-C₄-Alkenylcarbamoyl, wie Allylcarbamoyl, N,N-Di-C₁-C₄-Alkylcarbamoyl, wie Dimethylcarbamoyl, Diethylcarbamoyl oder Dibutylcarbamoyl, C₃-C₇-Cycloalkylcarbamoyl, wie Cyclopropylcarbamoyl, C₁-C₄-Alkoxycarbonyl-C₃-C₇-cycloalkylcarbamoyl, wie 1-Methoxycarbonyl- oder 1-Ethoxycarbonylcyclopropylcarbamoyl, 3- bis 7-gliedriges N-(C₃-C₆-Cycloalkyl)-C₁-C₄-alkylcarbamoyl, wie N-(Cyclopropylmethyl)carbamoyl, 3-Aza-2-oxo-cycloheptylcarbamoyl., Azaridinocarbonyl, 2-Methylazaridinocarbonyl, 2,3-Dihydoindolin-1-ylcarbamoyl, Pyrrolidinocarbonyl, Piperidinocarbonyl, unsubstituiertes oder C₁-C₄-Alkyl, C₁-C₄-Alkoxy, Hydroxy, Halogen und/oder Trifluormethyl substituiertes Phenyl-C₁-C₄-alkylcarbamoyl, wie Benzylcarbamoyl, unsubstituiertes oder durch C₁-C₄-Alkyl, C₁-C₄-Alkoxy, Hydroxy, Halogen, Nitro, Carboxy, C₁-C₄-Alkoxycarbonyl, wie Methoxy- oder Ethoxycarbonyl, Phenyl, Phenyloxy und/oder Trifluormethyl substituiertes N-Phenylcarbamoyl oder N-C₁-C₄-Alkyl-N-phenylcarbamoyl, wie N-Methyl-N-phenyl-carbamoyl, Naphthylcarbamoyl, 5,6-7,8-Tetrahydronaphthylcarbamoyl, Furyl-2-carbamoyl, Thien-2-ylcarbamoyl, Thiazol-2-ylcarbamoyl, Fur-2-ylmethylcarbamoyl, Thien-2-ylmethylcarbamoyl oder Thiazol-2-ylmethylcarbamoyl, C₁-C₄-Alkoxy-C₁-C₄-alkylcarbamoyl, wie Methoxymethylcarbamoyl, 2-Methoxyethylcarbamoyl oder Ethoxymethylcarbamoyl, Di-C₁-C₄-alkoxy-C₁-C₄-alkylcarbamoyl, wie Dimethoxymethylcarbamoyl oder Diethoxymethylcarbamoyl, Carboxy-C₁-C₄-alkylcarbamoyl, wie Carboxymethylcarbamoyl, 2-Carboxyethylcarbamoyl oder 1-Carboxy-2,2-dimethyl-propylcarbamoyl, C₁-C₄-Alkoxycarbonyl-C₁-C₄-alkylcarbamoyl, wie Methoxycarbonylmethylcarbamoyl, Ethoxycarbonylmethylcarbamoyl, 2-Methoxycarbonylethylcarbamoyl oder 1-Methoxycarbonyl-2,2-dimethyl-propylcarbamoyl, Dicarboxy-C₁-C₄-alkylcarbamoyl, wie Dicarboxymethylcarbamoyl, oder Di-C₁-C₄-alkoxycarbonyl-C₁-C₄-alkylcarbamoyl, wie Dimethoxycarbonylmethylcarbamoyl, Carboxy-C₂-C₄-alkenylcarbamoyl, wie 3-Carboxyprop-2-en-2-ylcarbamoyl, C₁-C₄-Alkoxycarbonyl-C₂-C₄-alkenylcarbamoyl, wie 3-Methoxycarbonylprop-2-en-2-ylcarbamoyl, 3-Ethoxycarbonylprop-2-en-2-ylcarbamoyl, 3-Methoxycarbonylprop-2-en-1-ylcarbamoyl oder 3-Ethoxycarbonylprop-2-en-1-ylcarbamoyl, N-Hydroxycarbamoyl, N-C₁-C₄-Alkoxycarbamoyl, wie Methoxycarbamoyl, Ethoxycarbamoyl, Propyloxycarbamoyl, Isopropyloxycarbamoyl, Butyloxycarbamoyl oder insbesondere Tertiärbutyloxycarbamoyl, N-C₂-C₄-Alkenyloxycarbamoyl, wie N-Vinyloxycarbamoyl, N-Allyloxycarbamoyl oder N-Methallyloxycarbamoyl oder unsubstituiertes oder durch C₁-C₄-Alkyl, C₁-C₄Alkoxy, Hydroxy, Halogen und/oder Trifluormethyl substituiertes N- Phenyl-C₁-C₄-alkoxycarbamoyl, wie Benzyloxycarbamoyl oder 1-Phenylethoxycarbamoyl, N-Phenyl-C₂-C₄-alkenyloxycarbamoyl, wie N-Phenylvinyloxycarbamoyl oder N-(3-Phenylprop-2-enyloxy)carbamoyl, Phosphono oder 5-Tetrazolyl darstellt,
und ihre Salze.

Die Erfindung betrifft insbesondere beispielsweise Verbindungen der Formel I, worin
A₁ Methylen oder eine Gruppe der Formel >CH-A₄-R₄ (la) darstellt,
A₂ eine Gruppe der Formel >CH-A₄-R₄ (la) oder, sofern A₁ eine Gruppe der Formel >CH-A₄-R₄ (la) darstellt, geradkettiges oder verzweigtes C₁-C₄-Alkylen, wie Methylen, 1,1-Ethyliden, 1,1-oder 2,2- Propyliden, 1,1- oder 2,2-Butyliden, bedeutet,
A₃ Oxy, Thio, Sulfinyl oder Sulfonyl ist,
A₄ für geradkettiges oder verzweigtes C₁-C₄-Alkylen, wie Methylen, 1,2-Ethylen, 1,3- oder 1,2-Propylen oder 1,4-, 1,3- oder 2,3-Butylen, steht,
n für 0 steht,
R₁ und R₂ unabhängig voneinander Wasserstoff, Hydroxy, C₁-C₄-Alkoxy, wie Methoxy, C₁-C₄-Alkyl, wie Methyl oder Ethyl, Trifluormethyl oder Halogen bedeuten,
R₃ Wasserstoff oder Hydroxy bedeutet und
R₄ Wasserstoff, Carboxy, C₁-C₄-Alkoxycarbonyl, wie Methoxycarbonyl, Ethoxycarbonyl, Propyloxycarbonyl, Isopropyloxycarbonyl oder Butyloxycarbonyl, ferner lsobutyloxycarbonyl, Sekundärbutyloxycarbonyl oder Tertiärbutyloxycarbonyl, unsubstituiertes oder durch C₁-C₄-Alkyl, C₁-C₄-Alkoxy, Hydroxy, Halogen und/oder Trifluormethyl substituiertes Phenyl-C₁-C₄-alkoxycarbonyl, Carbamoyl, C₁-C₄-Alkylcarbamoyl, unsubstituiertes oder durch C₁-C₄-Alkyl, C₁-C₄-Alkoxy, Hydroxy, Halogen und/oder Trifluormethyl substituiertes Phenylcarbamoyl N,N-Di-C₁-C₄-Alkylcarbamoyl, wie Dimethylcarbamoyl, Diethylcarbamoyl, Dipropylcarbamoyl, Diisopropylcarbamoyl oder Dibutylcarbamoyl, oder 5-Tetrazolyl darstellt, und ihre Salze.

Die Erfindung betrifft vorzugsweise Verbindungen der Formel I, worin
einer der Rest A₁ und A₂ eine Gruppe der Formel >CH-A₄-R₄ (la) und der andere Methylen darstellt,
A₃ Thio bedeutet,
A₄ für Methylen steht,
n für 0 steht,
R₁ und R₂ unabhängig voneinander Wasserstoff, C₁-C₄-Alkyl, wie Methyl oder Ethyl,
Trifluormethyl oder Halogen bedeuten,
R₃ Wasserstoff bedeutet und
R₄ Wasserstoff, Carboxy, C₁-C₄-Alkoxycarbonyl, wie Methoxycarbonyl, Ethoxycarbonyl, Propyloxycarbonyl, Isopropyloxycarbonyl oder Butyloxycarbonyl, unsubstituiertes oder durch C₁-C₄-Alkyl, C₁-C₄-Alkoxy, Hydroxy, Halogen und/oder Trifluormethyl substituiertes Benzoyloxy-C₁-C₄-alkoxycarbonyl, wie Benzoyloxymethyoxycarbonyl, unsubstituiertes oder durch C₁-C₄-Alkyl, C₁-C₄-Alkoxy, Hydroxy, Halogen und/oder Trifluormethyl, Nitro, Carboxy, C₁-C₄-Alkoxycarbonyl, wie Methoxy- oder Ethoxycarbonyl, und/oder Trifluormethyl substituiertes N-Phenylcarbamoyl, Naphthylcarbamoyl, 5,6-7,8-Tetrahydronaphthylcarbamoyl, Furyl-2-carbamoyl, Thien-2-ylcarbamoyl, Thiazol-2-ylcarbamoyl, Bicyclo[2,2,1]heptyl-, Bicyclo[2,2,2]octyl- oder Adamantylcarbamoyl, 3-Aza-2-oxo-cycloheptylcarbamoyl, N-C₁-C₄-Alkoxycarbamoyl, wie Methoxycarbamoyl oder Tertiärbutyloxycarbamoyl, N-C₂-C₄-alkenyloxycarbamoyl, wie N-Allyloxycarbamoyl oder N-Methallyloxycarbamoyl oder unsubstituiertes oder durch C₁-C₄-Alkyl, C₁-C₄Alkoxy, Hydroxy, Halogen und/oder Trifluormethyl substituiertes N- Phenyl-C₁-C₄-alkoxycarbamoyl, wie Benzyloxycarbamoyl oder 1-Phenylethoxycarbamoyl, N-Phenyl-C₂-C₄-alkenyloxycarbamoyl, wie N-Phenylvinyloxycarbamoyl oder N-(3-Phenylprop-2-enyloxy)carbamoyl, oder 5-Tetrazolyl darstellt,
und ihre Salze.

Die Erfindung betrifft vorzugsweise beispielsweise Verbindungen der Formel I, worin einer der Reste A₁ und A₂ Methylen oder eine Gruppe der Formel >CH-A₄-R₄ (la) und der andere Methylen darstellt,
A₃ Thio bedeutet,
n für 0 steht,
R₁ und R₂ unabhängig voneinander Wasserstoff, C₁-C₄-Alkyl, wie Methyl oder Ethyl,
Trifluormethyl oder Halogen bedeuten,
R₃ Wasserstoff darstellt und
R₄ Carboxy, C₁-C₄-Alkoxycarbonyl, wie Methoxycarbonyl, Ethoxycarbonyl, Propyloxycarbonyl, Isopropyloxycarbonyl oder Butyloxycarbonyl, 5-Tetrazolyl oder unsubstituiertes oder durch C₁-C₄-Alkyl, C₁-C₄-Alkoxy, Hydroxy, Halogen und/oder Trifluormethyl substituiertes Phenylcarbamoyl darstellt,
und ihre Salze.

Die Erfindung betrifft in allererster Linie Verbindungen der Formel I' 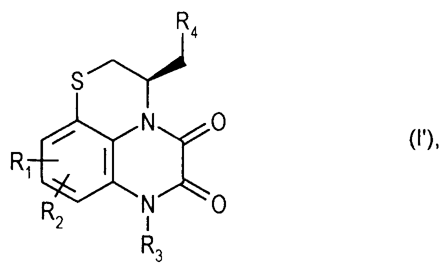
worin
R₁ und R₂ unabhängig voneinander Wasserstoff, C₁-C₄-Alkyl, wie Methyl oder Ethyl, Trifluormethyl oder Halogen bedeuten,
R₃ Wasserstoff bedeutet und
R₄ Wasserstoff, Carboxy, C₁-C₄-Alkoxycarbonyl, wie Methoxycarbonyl, Ethoxycarbonyl, Propyloxycarbonyl, Isopropyloxycarbonyl oder Butyloxycarbonyl, unsubstituiertes oder durch C₁-C₄-Alkyl, C₁-C₄-Alkoxy, Hydroxy, Halogen und/oder Trifluormethyl substituiertes Benzoyloxy-C₁-C₄-alkoxycarbonyl, wie Benzoyloxymethyoxycarbonyl, Bicyclo[2,2,1]heptyl-, Bicyclo[2,2,2]octyl- oder Adamantylcarbamoyl, 3-Aza-2-oxo-cycloheptylcarbamoyl, unsubstituiertes oder durch C₁-C₄-Alkyl, C₁-C₄-Alkoxy, Hydroxy, Halogen, Nitro, Carboxy, C₁-C₄-Alkoxycarbonyl, wie Methoxy- oder Ethoxycarbonyl, und/oder Trifluormethyl substituiertes N-Phenylcarbamoyl, Naphthylcarbamoyl, 5,6-7,8-Tetrahydronaphthylcarbamoyl, Furyl-2-carbamoyl, Thien-2-ylcarbamoyl, Thiazol-2-ylcarbamoyl, Bicyclo[2,2,1]heptyl-, Bicyclo[2,2,2]octyl- oder Adamantylcarbamoyl, 3-Aza-2-oxo-cycloheptylcarbamoyl, N-C₁-C₄-Alkoxycarbamoyl, wie Methoxycarbamoyl oder Tertiärbutyloxycarbamoyl, N-C₂-C₄-alkenyloxycarbamoyl, wie N-Allyloxycarbamoyl oder N-Methallyloxycarbamoyl oder unsubstituiertes oder durch C₁-C₄-Alkyl, C₁-C₄Alkoxy, Hydroxy, Halogen und/oder Trifluormethyl substituiertes N- Phenyl-C₁-C₄-alkoxycarbamoyl, wie Benzyloxycarbamoyl oder 1-Phenylethoxycarbamoyl, N-Phenyl-C₂-C₄-alkenyloxycarbamoyl, wie N-Phenylvinyloxycarbamoyl oder N-(3-Phenylprop-2-enyloxy)carbamoyl, oder 5-Tetrazolyl darstellt,
und ihre Salze.

Die Erfindung betrifft namentlich die in den Beispielen genannten Verbindungen der Formel I und ihre Salze, insbesondere ihre pharmazeutisch verwendbaren Salze.

Das Verfahren zur Herstellung der neuen Verbindungen der Formel I beruht auf an sich bekannten Methoden und ist beispielsweise dadurch gekennzeichnet, daß man
a) eine Verbindung der Formel II 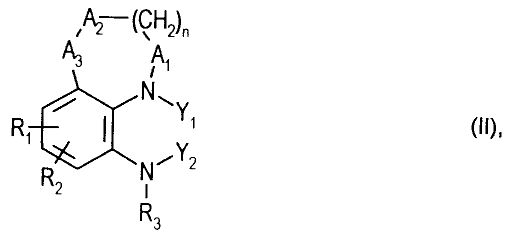 worin
   einer der Reste Y₁ und Y₂ für eine Gruppe der Formel -C (=O) -Y₃ (IIa) steht, in der Y₃ eine funktionell abgewandelte Carboxygruppe darstellt, und der andere Wasserstoff bedeutet, oder jeweils ein Salz davon intramolekular cyclisiert oder
b) eine Verbindung der Formel III 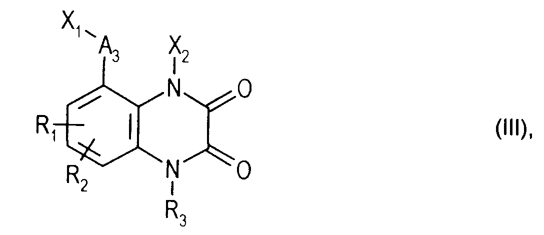 X₁ eine Gruppe der Formel -A₂-(CH₂)ₙ-CH(Y₃)-A₄-R₄ (IIIa) oder-A₂-(CH₂)ₙ-CH=A'₄-R₄ (IIIb) darstellt, wobei Y₃ eine nukleofuge Abgangsgruppe und A'₄ eine der Gruppe A₄ entsprechende Niederalkanylylidengruppe bedeutet, X₂ für Wasserstoff steht und R₁, R₂, R₃, A₁, A₂, A₃ und A₄ die angebenen Bedeutungen haben,
   intramolekular cyclisiert oder
c) in einer Verbindung der Formel IV 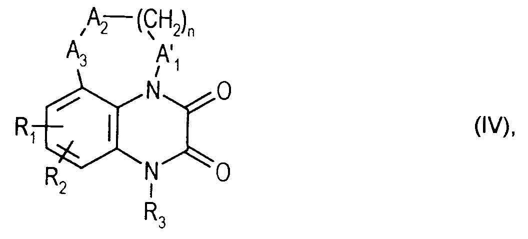 worin A'₁ eine Gruppe der Formel >C=A'₄-R₄ (IIIa) darstellt, in der A'₄ für Niederalkanylyliden bedeutet, die extracyclische Doppelbindung zu eine Einfachbindung reduziert und jeweils gewünschtenfalls eine erhaltene Verbindung in eine andere Verbindung der Formel I überführt, ein verfahrensgemäß erhältliches Isomerengemisch in die Komponenten auftrennt und das jeweils bevorzugte Isomere abtrennt und/oder eine verfahrensgemäß erhältliche freie Verbindung in ein Salz oder eine verfahrensgemäß erhältliches Salz in die entsprechende freie Verbindung überführt.

Die intramolekulare Cyclisierung von Verbindungen der Formel II gemäß der Verfahrensvariante a) erfolgt in üblicher Weise, erforderlichenfalls in einem inerten organischen Lösungsmittel, wie Aceton, Tetrahydrofuran, Dioxan oder Dimethylformamid, gegebenenfalls im Gemisch mit Wasser, und/oder in Gegenwart eines basischen Kondensationsmittels, wie eines tertiären aliphatischen Amins, wie Triethylamin, oder einer tertiären aromatischen Stickstoffbase, wie Pyridin, oder einer Metallbase, wie einem Alkalimetallhydroxid, Alkalimetallcarbonat oder Alkalimetallamid, z.B. Natrium- oder Kaliumhydroxid, Natrium- oder Kaliumcarbonat oder Natrium- oder Kaliumamid, vorteilhaft unter Erwärmen, z.B. im Temperaturbereich von etwa 25° bis 120°, vorzugsweise zwischen 50° und 100°.

Ausgangsstoffe der Formel II werden vorzugsweise *in situ* hergestellt und ohne Isolierung intramolekular cyclisiert. So erhält man Verbindungen der Formel II, worin Y₁ für eine Gruppe der Formel -C (=O) -Y₃ (IIa) und Y₂ für Amino steht, indem man eine Verbindung der Formel IIb 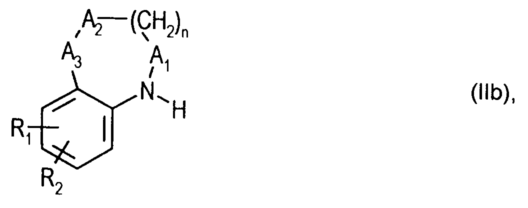
in üblicher Weise, beispielsweise in Gegenwart eines tertiären Amins, wie Triethylamin in Dichlormethan, mit einem reaktiven Oxalsäurederivat, beispielsweise mit einer Verbindungen der Formel Y₃-C(=O)-C (=O) -Y'₃ (IIc), worin Y₃ und Y'₃ gleiche oder verschiedene nukleophile Abgangsgruppen bedeuten und Y₃ vorzugsweise für Halogen und Y'₃ vorzugsweise für verethertes Hydroxy, wie Niederalkoxy steht, kondensiert, das Reaktionsprodukt der Formel IId 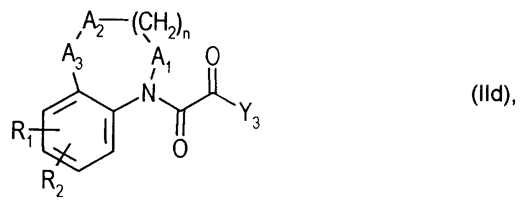
in üblicher Weise, beispielsweise mittels Salpetersäure und Schwefelsäure, nitriert und anschließend reduziert. Dabei wird intermediär eine Verbindung der Formel IIe 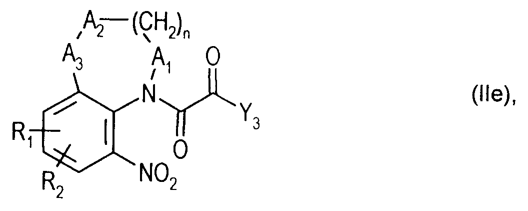
gebildet, die unter den Bedingungen ihrer Bildung *in situ* erfindungsgemäß cyclisiert.

Zwischenprodukte der Formel IIb, worin A₁ eine Gruppe der Formel >CH-A₄-R₄ (Ia) ist,
A₂ und A₄ Methylen ist, A₃ vorzugsweise Thio ist, R₁ und R₂ die angegebenen Bedeutungen haben,
R₃ Wasserstoff ist, R₄ eine veresterte Carboxygruppe und n 0 darstellt,
werden beispielsweise hergestellt, indem man
eine Verbindung der Formel 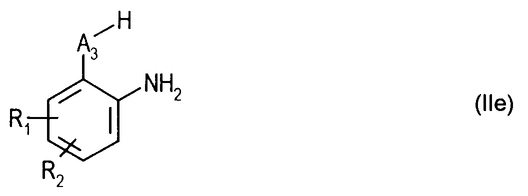
in üblicher Weise mit einem ω-Halogenacetessigsäureester der Formel Hal-A₂-C(=O)-CH₂-R₄ (IIf) kondensiert oder in einer Verbindung der Formel IIg 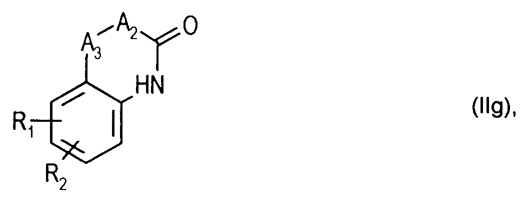
die Carbonylgruppe in üblicher Weise, beispielsweise nach Lawesson, in Thiocarbonyl überführt, das Reaktionsprodukt der Formel IIh 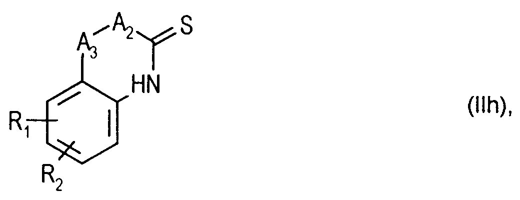
mit einem Halogenessigsäureester der Formel Hal-CH₂-COOR (IIi; R = Niederalkyl) kondensiert, das Reaktionsprodukt der Formel IIj 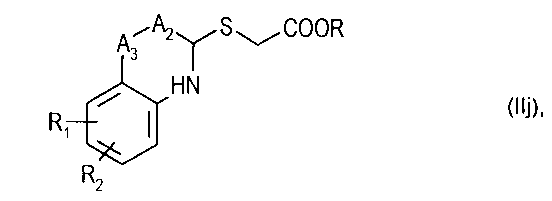
mit Triphenylphosphin behandelt und in dem Reaktionsprodukt der Formel IIk 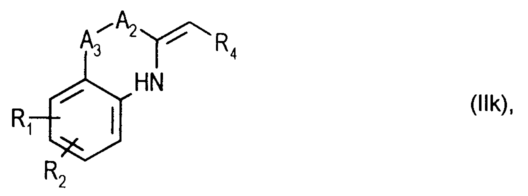
die extracyclische Doppelbindung in üblicher Weise, beispielsweise durch Umsetzung mit Natriumcyanoborhydrid, oder einem stereoselektiv wirkenden homogenen Rhodiumkatalysator, zur Einfachbindung reduziert.

Gemäß einer alternativen Verfahrensweise kann man das Zwischenprodukt der Formel IIe mit einem 3-Halogenprop-2-ensäureester, direkt zur entsprechenden Verbindung der Formel IIb umsetzen.

In einer weiteren Abwandlung dieses Verfahrens kann man die Verbindung der Formel IIe in üblicher Weise, beispielsweise in Tetrahydrofuran, mit (S)-Epichlorhydrin umsetzen und die erhaltene Verbindungen der Formel IIv 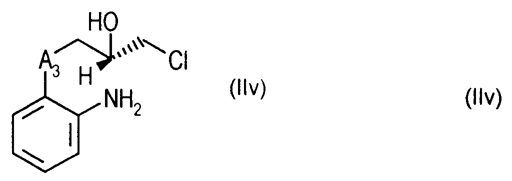
in üblicher Weise. beispielsweise durch Behandeln mit Kaiumhydroxid in Ethanl, zur entsprechenden Verbindungen der Formel IIw 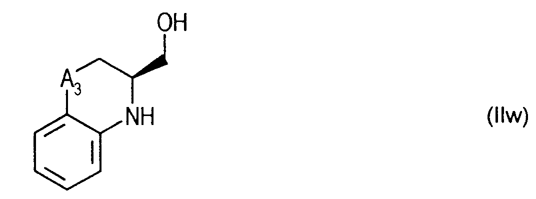
cyclisiert, deren Hydroxygruppe dann nach üblichen Methoden durch eine der genannten Gruppen R₄, beispielsweise durch Cyano, gegebenenfalls verestertes oder amidiertes Carboxy, Tetrazolyl oder gegebenenfalls verestertes Phosphono ersetzt werden kann.

Verbindungen der Formel IIe ihrerseits werden beispielsweise durch Hydrolyse entsprechender Verbindungen der Formel IIm 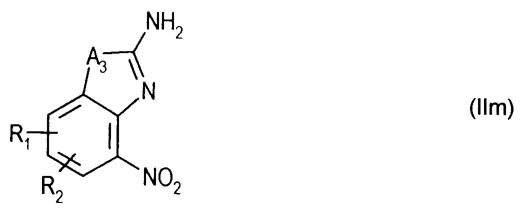
bzw. durch Reduktion, beispielsweise mittel Natriumthiosulfat, entsprechenderDisulfide der Formel IIn 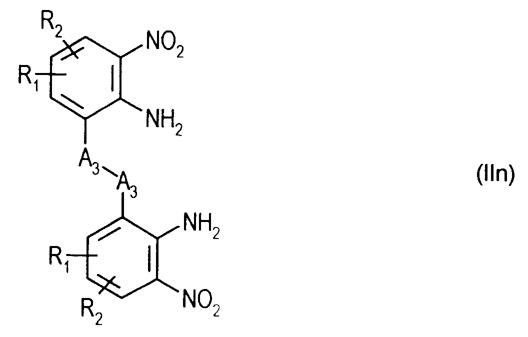
erhalten werden.

In analoger Weise können auch Zwischenprodukte der Formel IIb, worin A₁ und A₂ Methylen bedeuten und A₃ Thio ist, erhalten werden, indem man die Verbindung der Formel IIe mit einer wäßrigen Halogenessigsäure, beispielsweise mit Chloressigsäure, umsetzt.

Verbindungen der Formel II, worin A₁ für eine Gruppe >CH-A₄-R₄ (Ia), A₂ für Methylen, A₃ für Oxy, A₄ für Methylen, n für 0, Y₁ für eine Gruppe der Formel -C (=O) -Y₃(IIa) und Y₂ für Amino steht kann man vorteilhaft ferner herstellen, indem man eine Verbindung der Formel IIo 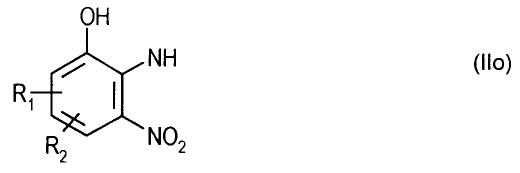
in üblicher Weise mit einem ω-Halogenacetessigsäureester der Formel Hal-CH₂-C(=O)-CH₂-R₄ (IIp, Hal = Halogen) kondensiert und aus dem Reaktionsprodukt der Formel llq 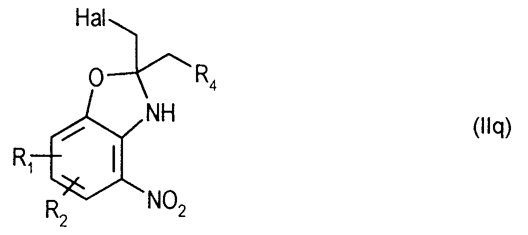
in üblicher Weise, beispielsweise durch Behandlung mit Natriumhydrid in Tetrahydrofuran, unter Ringerweiterung die Gruppe Hal abspaltet, in dem erhaltenen Produkt der Formel IIr 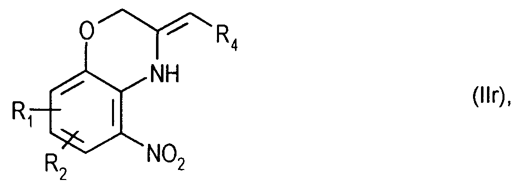
die Nitrogruppe in üblicher Weise, beispielsweise durch katalytische Hydreirung mit Raney-Nickel in Ethanol, zu Amino und anschiießend, beispielsweise mittels Natriumcyanoborhydrid in Ethanol, die extracyclische Doppelbindung zur Einfachbindung reduziert und das erhaltene Produkt der Formel IIs 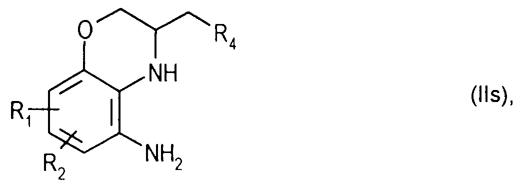
in üblicher Weise, beispielsweise in Gegenwart eines tertiären Amins, wie Triethylamin in Dichlormetahn, mit einem reaktiven Oxalsäurederivat, beispielsweise mit einer Verbindungen der Formel Y₃-C(=O)-C (=O) -Y'₃(IIc), worin Y₃ und Y'₃ gleiche oder verschiedene nukleophile Abgangsgruppen bedeuten und Y₃ vorzugsweise für Halogen und Y'₃ vorzugsweise für verethertes Hydroxy, wie Niederalkoxy steht, kondensiert. Dabei wird intermediär die entsprechende Verbindung der Formel II gebildet, die unter den Bedingungen ihrer Bildung *in situ* erfindungsgemäß cyclisiert.

In analoger Weise kann man auch Verbindungen der Formel II, worin A₁ für Methylen oder eine Gruppe >C(=O) (Ib), A₂ für Methylen, A₃ für Oxy, n für 0, Y₁ für eine Gruppe der Formel -C (=O) -Y₃(IIa) und Y₂ für Amino steht kann man vorteilhaft ferner herstellen, indem man eine Verbindung der Formel IIt 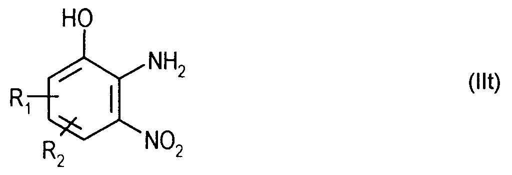
mit einer wäßrigen Halogenessigsäure, beispielsweise mit Chloressigsäure, umsetzt, in der ehaltenen Verbindung der Formel IIu 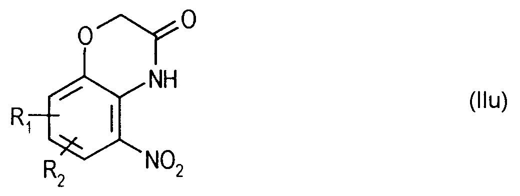
in üblicher Weise die Nitrogruppe zu Amino und gewünschtenfalls die Carbonylgruppe zu Methylen reduziert und das Reaktionsprodukt mit einem reaktiven Oxalsäurederivat, beispielsweise mit einer Verbindungen der Formel Y₃-C(=O)-C (=O) -Y'₃(IIc), worin Y₃ und Y'₃ gleiche oder verschiedene nukleophile Abgangsgruppen bedeuten und Y₃ vorzugsweise für Halogen und Y'₃ vorzugsweise für verethertes Hydroxy, wie Niederalkoxy steht, kondensiert. Dabei wird intermediär die entsprechende Verbindung der Formel II gebildet, die unter den Bedingungen ihrer Bildung *in situ* erfindungsgemäß cyclisiert.

Zur Herstellung von Verbindungen der Formeln II, worin A₃ für oxidiertes Thio, d.h. Sulfinyl oder Sulfonyl steht, wird in einem der vorstehend genannten Zwischenprodukte, in dem A₃ für Thio steht, die Thiogruppe in üblicher Weise oxidiert, vorteilhaft auf der Stufe der Herstellung der jeweiligen Nitroverbindung gemäß IId ----> IIe.

Zur Herstellung von Verbindungen der Formel II, worin R₁ bzw. R₂ von Wasserstoff verschieden sind und R₁ beispielsweise in 3-Stellung zur Gruppe A₃ gebundenes Halogen bedeutet, nimmt man die Einführung solcher Substituenten vorzugsweise auf der Stufe der Zwischenprodukte der Formeln IIb, IIn oder IIs vor.

In Ausgangsstoffen der Formel III gemäß Verfahrensvariante b) sind nukleophile Abgangsgruppen beispielsweise reaktionsfähige veresterte Hydroxygruppen, wie mit einer Mineralsäure oder Sulfonsäure veresterte Hydroxygruppen, insbesondere Halogenatome, beispielsweise Chlor, Brom oder Jod, oder mit einer aliphatischen oder einer gegebenenfalls substituierten aromatischen Sulfonsäure veresterte Hydroxygruppen, beispielsweise Niederalkansulfonyloxy, wie Methansulfonyloxy, oder gegebenenfalls substituiertes Benzolsulfonyloxy, wie Benzolsulfonyloxy, Brombenzolsulfonyloxy oder Toluolsulfonyloxy, ferner tertiäre Aminogruppen, wie Diniederalkylamino oder gegebenenfalls durch Sauerstoff, Schwefel oder Stickstoff unterbrochenes Niederalkylenamino, beispielsweise Pyrrolidino, Piperidino, Morpholin oder Thiomorpholino.

Funktionell abgewandelte Carboxygruppen sind beispielsweise gegebenenfalls veresterte oder anhydridisierte Carboxygruppen, wie Carboxy, Niederalkoxycarbonyl, insbesondere Methoxy- oder Ethoxycarbonyl, Isopropyloxycarbonyl oder Tertiaäbutyloxycarbonyl, gegebenenfalls durch Niederalkyl, Niederalkoxy, Halogen und/oder Nitro substituiertes Phenyloxycarbonyl, Halogencarbonyl, wie Chlorcarbonyl, oder Niederalkanoyloxycarbonyl, insbesondere Formyloxycarbonyl, Acetoxycarbonyl oder Pivaloyloxycarbonyl.

Die intramolekulare Cyclisierung von Verbindungen der Formel III erfolgt in üblicher Weise, erforderlichenfalls in einem inerten organischen Lösungsmittel, wie Tetrahydrofuran, Dioxan oder Dimethylformamid, und/oder in Gegenwart eines basischen Kondensationsmittels, wie eines tertiären aliphatischen Amins, wie Triethylamin, oder einer tertiären aromatischen Stickstoffbase, wie Pyridin, oder einer Metallbase, wie einem Alkalimetallhydroxid, Alkalimetallcarbonat oder Alkalimetallamid, z.B. Natrium- oder Kaliumhydroxid, Natrium- oder Kaliumcarbonat oder Natrium- oder Kaliumamid, vorteilhaft unter Erwärmen, z.B. im Temperaturbereich von etwa 25° bis 120°, vorzugsweise zwischen 50° und 100°.

Die Ausgangsstoffe der Formeln III können nach an sich bekannten Methoden hergestellt werden.

So erhält man Verbindungen der Formel III, worin n für 0 steht, A₂ Niederalkyliden bedeutet und A₃ Oxy oder Thio ist, indem man eine Verbindung der Formel IIIa 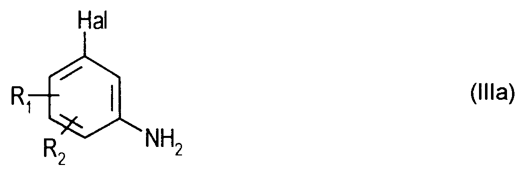
worin Hal für ein Halogen-, wie Chlor oder Bromatom steht, mit einem reaktionsfähigen Derivat der Kohlensäure, wie einem Halogenameisensäureniederalkylester der Formel Hal - C(=O) -COOR (IIIb; R= Alkyl, gegebenenfalls substituiertes Phenyl), kondensiert, das Reaktionsprodukt der Formel IIIc 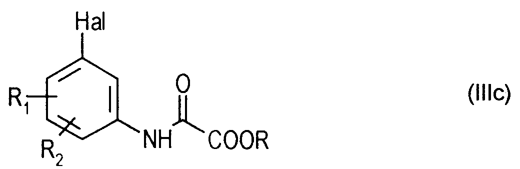
in üblicher Weise, beispielsweise mittels Kaliumnitrat und Schwefelsäure, in o-Stellung zur Hal und zur Aminogruppe nitriert, das Reaktionsprodukt der Formel IIId 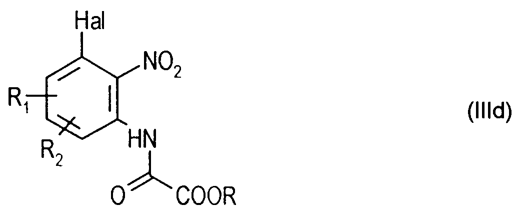
mit einer Verbindung der Formel H-A₃-A₂-(CH₂)ₙ-C(H)=A'₄-R₄ kondensiert und in der erhaltenen Verbindung der Formel IIIe 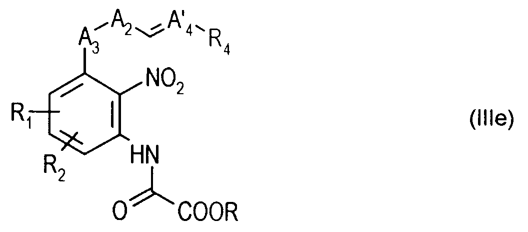
Die Nitrogruppe in üblicher Weise, beispielsweise mittels Zinn-II-chlorid, zu Amino reduziert, wobei das Reaktionsprodukt unter den Bedingungen seiner Bildung spontan zum entsprechenden Chinoxalindion der Formel III cyclisiert.

Die Reduktion der extracyclischen Doppelbindung in Verbindungen der Formel IV gemäß der Verfahrensvariante c) erfolgt in üblicher Weise, beispielsweise durch Umsetzung mit einem Dileichtmetallhydrid, wie Natriumcyanoborhydrid, oder durch katalytische Hydrierung, beispielsweise in Gegenwart von Raney-Nickel.

Ausgangsstoffe der Formel IV können beispielsweise erhalten werden, indem man eine Verbindung der Formel 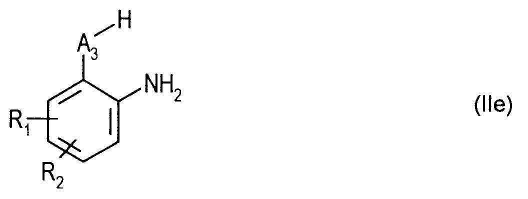
in üblicher Weise mit einem ω-Halogenacetessigsäureester der Formel Hal-A₂-C(=O)-CH₂-R₄ (IIf) kondensiert, das Reaktionsprodukt der Formel IVa 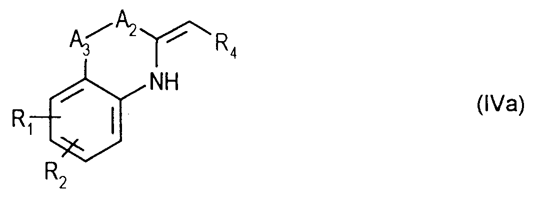
in üblicher Weise, beispielsweise in Gegenwart eines tertiären Amins, wie Triethylamin in Dichlormethan, mit einem reaktiven Oxalsäurederivat, beispielsweise mit einer Verbindungen der Formel Y₃-C(=O)-C (=O) -Y'₃(IIc), worin Y₃ und Y'₃ gleiche oder verschiedene nukleophile Abgangsgruppen bedeuten und Y₃ vorzugsweise für Halogen und Y'₃ vorzugsweise für verethertes Hydroxy, wie Niederalkoxy steht, kondensiert, das Reaktionsprodukt der Formel IVb 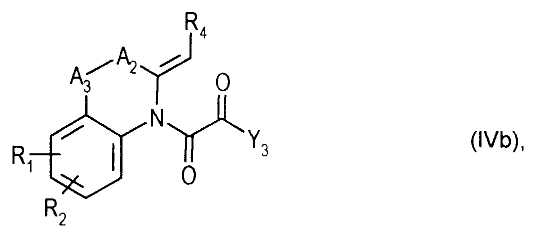
in üblicher Weise, beispielsweise mittels Salpetersäure und Schwefelsäure, nitriert und anschließend reduziert. Dabei wird intermediär eine Verbindung der Formel IVc 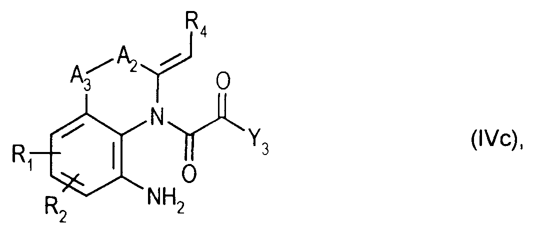
gebildet, die unter den Bedingungen ihrer Bildung *in situ* zur entsprechenden Verbindungen der Formel IV, worin n für 0 steht und A'₄ Methin bedeutet, cyclisiert.

Verbindungen der Formel IIe ihrerseits werden beispielsweise durch Hydrolyse entsprechender Verbindungen der Formel IIh 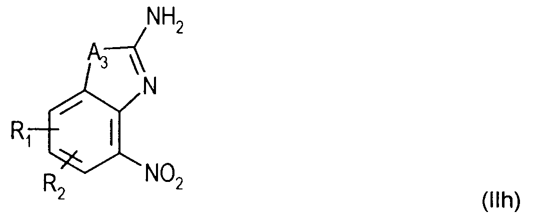
bzw. durch Reduktion, beispielsweise mittel Natriumthiosulfat, entsprechenderDisulfide der Formel IIi 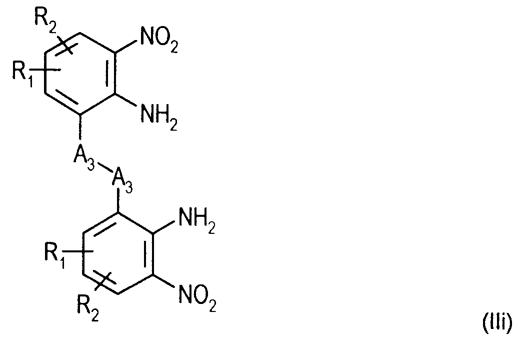
erhalten werden.

Zur Herstellung von Verbindungen der Formel IV, worin A₃ für Oxy, A₂ für Methylen A'₄ für Methin und n für 0 steht, kann man ferner eine Verbindung der Formel IIj 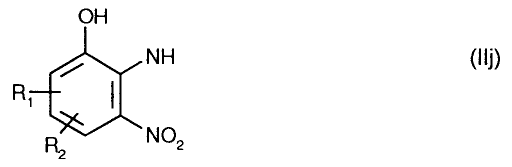
in üblicher Weise mit einem ω-Halogenacetessigsäureester der Formel Hal-CH₂-C(=O)-CH₂-R₄ (IIk, Hal = Halogen) kondensieren, aus dem Reaktionsprodukt der Formel IIk 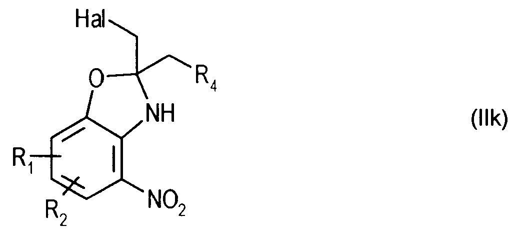
in üblicher Weise, beispielsweise durch Behandlung mit Natriumhydrid in Tetrahydrofuran, unter Ringerweiterung die Gruppe Hal abspalten, in dem erhaltenen Produkt der Formel IIm 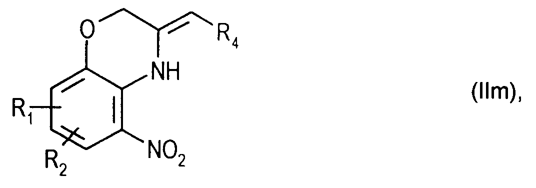
die Nitrogruppe in üblicher Weise, beispielsweise durch katalytische Hydreirung mit Raney-Nickel in Ethanol, zu Amino redzuziert und das Reaktionsprodukt in üblicher Weise, beispielsweise in Gegenwart eines tertiären Amins, wie Triethylamin in Dichlormethan, mit einem reaktiven Oxalsäurederivat, beispielsweise mit einer Verbindungen der Formel Y₃-C(=O)-C (=O) -Y'₃ (IIc), worin Y₃ und Y'₃ gleiche oder verschiedene nukleophile Abgangsgruppen bedeuten und Y₃ vorzugsweise für Halogen und Y'₃ vorzugsweise für verethertes Hydroxy, wie Niederalkoxy steht, kondensieren, wobei intermediär ebenfalls wird eine Verbindung der Formel IVc gebildet wird, die unter den Bedingungen ihrer Bildung *in situ* zur entsprechenden Verbindungen der Formel IV, worin n für 0 steht und A'₄ Methin bedeutet, cyclisiert.

Zur Herstellung von Verbindungen der Formel IV, worin A₃ für Oxy oder insbesondere Thio, A₂ für Methylen A'₄ für Methin und n für 0 steht, kann man ferner in einer Verbindung der Formel IIg 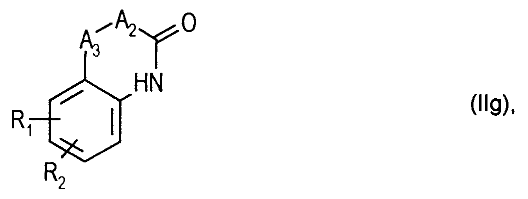
die Carbonylgruppe in üblicher Weise, beispielsweise nach Lawesson, in Thiocarbonyl überführen, das Reaktionsprodukt der Formel IIh 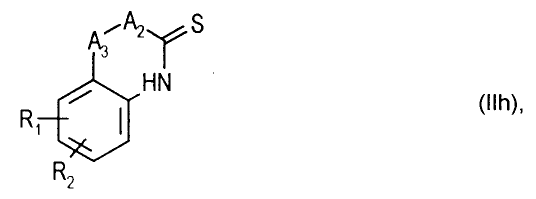
mit einem Halogenessigsäureester der Formel Hal-CH₂-COOR (IIi; R = Niederalkyl) kondensieren und das Reaktionsprodukt der Formel llj 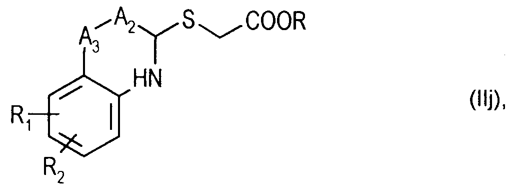
mit Triphenylphosphin behandeln.

Verfahrensgemäss erhältliche Verbindungen können in üblicher Weise in andere Verbindungen der Formel I überführt werden.

So kann man Verbindungen der Formel I, worin R₄ für Carboxy steht, zu der entsprechenden Verbindung der Formel I verestern, in der R₄ für verestertes Carboxy steht. Ebenso kann man Verbindungen der Formel I, worin R₄ für gegebenenfalls verestertes Carboxy steht, zu der entsprechenden Verbindung der Formel I amidieren, in der R₄ für amidiertes Carboxy steht. Umgekehrt kann man Verbindungen der Formel I, worin R₄ für Cyano oder verestertes oder amidiertes Carboxy steht, zu der entsprechenden Verbindung der Formel I hydrolysieren, in der R₄ und gegebenenfalls R₅ für Carboxy steht. Ferner kann man eine Cyanogruppe R₅ zu Carbamoyl hydrolysieren.

Ferner kann man in erhaltenen Verbindungen der Formel I, worin A₃ für Thio steht, die Thiogruppe in üblicher Weise, beispielsweise. durch Umsetzung mit einer geeigneten Peroxyverbindung, wie m-Chlorperbenzoesäure oder Permonophthalsäure, zu Sulfinyl oder Sulfonyl oxidieren.

Ferner kann man in Verbindung der Formel I, worin R₁ und/oder R₂ Wasserstoff bedeutet, das Wasserstoffatom durch einen von Wasserstoff verschiedenen Rest R₁ und/oder R₂ ersetzen.

So kann man in üblicher Weise Niederalkanoyl einführen, beispielsweise durch Umsetzung mit einem reaktionsfähigen Niederalkansäurederivat, wie einem Niederalkansäurechlorid oder Niederalkansäurenitril, in Gegenwart von Aluminiumtrichlorid, bei Umsetzung mit einem Niederalkansäurenitril erforderlichenfalls in Gegenwart von Bortrichlorid, vorzugsweise in einem halogenierten Kohlenwasserstoff, erforderlichenfalls in der Siedehitze.

Erhaltene Salze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formel I, einschließlich ihrer Salze, können auch in Form von Hydraten erhalten werden oder das zur Kristallisation verwendete Lösungsmittel einschließen.

Infolge der engen Beziehung zwischen den neuen Verbindungen in freier Form und in Form ihrer Salze sind vorstehend und nachfolgend unter den freien Verbindungen und ihren Salzen sinn- und zweckgemäß gegebenenfalls auch die entsprechenden Salze bzw. freien Verbindungen zu verstehen.

Erhaltene Diastereomerengemische und Racematgemische können auf Grund der physikalisch-chemischen Unterschiede der Bestandteile in bekannter Weise in die reinen Diastereomeren bzw. Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung des erhaltenen Diastereomerengemisches bzw. Racemates mit einer optisch aktiven Hilfsverbindung, z.B. entsprechend der in Verbindungen der Formel I enthaltenen sauren, basischen oder funktionell abwandelbaren Gruppen mit einer optisch aktiven Säure, Base oder einem optisch aktiven Alkohol, in Gemische diastereomerer Salze bzw. funktioneller Derivate, wie Ester, Trennung derselben in die Diastereomeren, aus denen das jeweils gewünschte Enantiomere in der jeweils üblichen Weise freigesetzt werden kann. Dafür geeignete Basen, Säuren bzw. Alkohole sind beispielsweise optisch aktive Alkaloidbasen, wie Strychnin, Cinchonin oder Brucin, oder D- oder L-(1-Phenyl)ethylamin, 3-Pipecolin, Ephedrin, Amphetamin und ähnliche synthetisch zugängliche Basen, optisch aktive Carbon- oder Sulfonsäuren, wie Chinasäure oder D- oder L-Weinsäure, D- oder L-Di-o-toluylweinsäure, D- oder L-Äpfelsäure, D- oder L-Mandelsäure, oder D- oder L-Camphersulfonsäure, bzw. optisch aktive Alkohole, wie Borneol oder D- oder L-(1-Phenyl)ethanol.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemäßen Verbindungen entwickelt wurden, insbesondere die zu den eingangs als bevorzugt gekennzeicheten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Die Erfindung betrifft gleichfalls pharmazeutische Präparate, die erfindungsgemäßen Verbindungen oder pharmazeutisch verwendbare Salze derselben als Wirkstoffe enthalten, sowie Verfahren zu ihrer Herstellung.

Bei den erfindungsgemäßen pharmazeutischen Präparaten, welche die erfindungsgemäßen Verbindung oder pharmazeutisch verwendbare Salze davon enthalten, handelt es sich um solche zur enteralen, wie oralen, ferner rektalen, und parenteralen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffes hängt von dem Alter und dem individuellen Zustand sowie von der Applikationsweise ab.

Die neuen pharmazeutischen Präparate enthalten z.B. von etwa 10 % bis etwa 80 %, vorzugsweise von etwa 20 % bis etwa 60 %, des Wirkstoffs. Erfindungsgemäße pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Traganth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fließ-, Fließregulierund Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaft-resistenten Überzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Ölen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetisch Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung eignen sich in erster Linie wäßrige Lösungen eines Wirkstoffs in wässerlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat oder Triglyceride, verwendet oder wäßrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die Dosierung des Wirkstoffes hängt von der Warmblüter-Spezies, dem Alter und dem individuellen Zustand sowie der Applikationsweise ab. Im Normalfall ist für einen etwa 75 kg schweren Patienten bei oraler Applikation eine ungefähre Tagesdosis von etwa 10 mg bis etwa 500 mg zu veranschlagen.

Die nachfolgen Beispiele dienen zur Illustration der Erfindung; Temperaturen sind in Celsiusgraden, Drucke in mbar angegeben. Die Bezifferung der Ringatome von erfindungsgemäßen 4,5-Dioxy-2,3,5,6-tetrahydro 4H-1-X-3a,6-diazaphenalenen entspricht der nachstehenden Formel

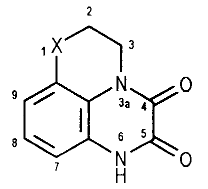

### Beispiel 1: 3,3-Dihydro-6H-1-oxa-3a,6-diaza-phenalen-4,5-dion

Man löst 1.03 g (6.86 mMol) 3,4-Dihydro-2*H*-benzo[1,4]oxazin-5-ylamin in 23 ml (167.7 mMol) Diethyloxalat bund rotiert bei 80° und 20 mbar 22 Stunden am Rotationsverdampfer. Die so entstandene Suspension wird filtriert, der Rückstand mit Ether gewaschen und unter vermindertem Druck bei 60° getrocknet. Man erhält 1.05 g (5.14 mMol = 75%) der Titelverbindung in Form brauner Kristalle; Smp. 288° nach Sublimation bei 200° und 0.13 mbar; ¹H-NMR (D₆-DMSO, 300 MHz): 4.03 (t, J=4.8 Hz, 2H, H₂C-N); 4.32 (t, J=5.0 Hz, 2H, H₂C-O); 4.03 (t, J=4.8 Hz, 2H, H₂C-N); 6.69-6.77 (m, 2H, H(arom.)C(4, 6)); 7.00-7.05 (m, 1H, H(arom.)C(5)); 12.02 (s br, 1H, HN); ¹³C-NMR (D₆-DMSO, 74 MHz): 40.35 (H₂C-N); 63.61 (H₂C-O); 108.08 (HC); 110.66 (HC); 114.06 (C); 123.97 (HC); 126.62 (C); 143.88 (C(1)); 153.29 (C=O); 154.22 (C=O).

### Beispiel 2:

In analoger Weise wie in Beispiel 1 bzw. in der Beschreibung beschrieben kann man auch folgende Verbindungen der Formel I herstellen:

### 8-Chlor-4,5-dioxo-2,3,5,6-tetrahydro-4H-1-thia-3a,6-diaza-phenalen-3-ylessigsäureethylester;

### 8-Chlor-4,5-dioxo-2,3,5,6-tetrahydro-4H-1-thia-3a,6-diaza-phenalen-3-ylessigsäure;

### 8-Chlor-4,5-dioxo-2,3,5,6-tetrahydro-4H-1-thia-3a,6-diaza-phenalen-3-yl-(N-phenyl)acetamid;

### 8-Chlor-6H-1-thia-3a,6-diaza-phenalen-3,4,5-trion;

### 8-Chlor-1,4,5-triioxo-2,3,5,6-tetrahydro-1H,4H-1-thia-3a,6-diaza-phenalen-3-ylessigsäureethylester;

### 8-Brom-1,4,5-trioxo-2,3,5,6-tetrahydro-1H,4H-1-thia-3a,6-diaza-phenalen-3-ylessigsäureethylester;

### 8-Chlor-1,4,5-triioxo-2,3,5,6-tetrahydro-1H,4H-1-thia-3a,6-diaza-phenalen-3-ylessigsäure;

### 8-Brom-1,4,5-trioxo-2,3,5,6-tetrahydro-1H,4H-1-thia-3a,6-diaza-phenalen-3-ylessigsäure;

### 8-Chlor-1,4,5-triioxo-2,3,5,6-tetrahydro-1H,4H-1-thia-3a,6-diaza-phenalen-3-yl-(N-phenyl)acetamid;

### 8-Brom-1,4,5-trioxo-2,3,5,6-tetrahydro-1H,4H-1-thia-3a,6-diaza-phenalen-3-yl-(N-phenyl)acetamid;

### 4,5-Dioxo-2,3,5,6-tetrahydro-4H-1-oxa-3a,6-diaza-phenalen-3-ylessigsäureethylester;

### 4,5-Dioxo-2,3,5,6-tetrahvdro-4H-1-oxa-3a,6-diaza-phenalen-3-ylessigsäure;

### 4,5-Dioxo-2,3,5,6-tetrahydro-4H-1-oxa-3a,6-diaza-phenalen-3-yl-(N-phenyl)acetamid;

### 8-Brom-4,5-dioxo-2,3,5,6-tetrahydro-4H-1-oxa-3a,6-diaza-phenalen-3-ylessigsäureethylester;

### 8-Brom-4,5-dioxo-2,3,5,6-tetrahydro-4H-1-oxa-3a,6-diaza-phenalen-3-ylessigsäure und

### 8-Brom-4,5-dioxo-2,3,5,6-tetrahydro-4H-1-oxa-3a,6-diaza-phenalen-3-yl-(N-phenyl)acetamid.

### Beispiel 3:

In analoger Weise wie in Beispiel 1 bzw. in der Beschreibung beschrieben kann man auch folgende Verbindungen der Formel I herstellen:

### 8-Brom-4,5-dioxo-2,3,5,6-tetrahydro-4H-1-thia-3a,6-diaza-phenalen-2-ylessigsäureethylester;

### 8-Brom-4,5-dioxo-2,3,5,6-tetrahydro-4H-1-thia-3a,6-diaza-phenalen-2-ylessigsäure;

### 8-Brom-4,5-dioxo-2,3,5,6-tetrahydro-4H-1-thia-3a,6-diaza-phenalen-2-ylessigsäure-N-phenylamid;

### 8-Brom-4,5-dioxo-2,3,5,6-tetrahydro-4H-1-thia-3a,6-diaza-phenalen-2-ylacetamid;

### 8-Brom-4,5-dioxo-2,3,5,6-tetrahydro-4H-1-thia-3a,6-diaza-phenalen-2-ylacetonitril;

### 8-Brom-2(1H-tetrazol-5-ylmethyl)-2,3-dihydro-6H-1-thia-3a,6-diaza-phenalen-4,5-dion.

### Beispiel 4: 8-Brom-4,5-dioxo-2,3,5,6-tetrahydro-4H-1-thia-3a,6-diaza-phenalen-3-yl-essigsäureethylester

4.58 g (11 mMol) 7-Brom-3,4-dihydro-2H-benzo(1,4)thiazin-3-yl-essigsäureethylester, gelöst in 12 ml konzentrierter Schwefelsäure wird bei -10° mit 12 ml rauchender Salpetersäure nitriert. Man lässt über Nacht bei 0° ausrühren, gießt auf Eiswasser, extrahiert dreimal mit Essigsäureethylester, wäscht die organischen Phasen mit Wasser, gesättigter Natriumhydrogencarbonatlösung und gesättigter Kochsalzlösung, trocknet über Magnesiumsulfat und zieht das Lösungsmittel ab. Das rohe, nitrierte Zwischenprodukt wird in 50 ml Aceton gelöst bei 0° zu einer Mischung von 140ml 15% TiCl₃ in ca. 10 %iger Salzsäure,75 ml Wasser, und 110 ml Aceton langsam zugetropft und 18 Stunden bei 0° gerührt. Die entstandene weisse Fällung wird abfiltriert, mit viel Wasser neutralgewaschen und am Hochvakuum bei 60° getrocknet. Man erhält 2.51 g (6.52 mMol) = 59% 8-Brom-4,5-dioxo-2,3,5,6-tetrahydro-4H-1-thia-3a,6-diaza-phenalen-3-yl-essigsäureethylester als beiges Pulver; ¹H-NMR (CDCl₃, 300MHz): 1.25 (t, 3H); 2.69 (dd, 1H); 2.99 (dd, 1H); 3.26 (dd, 1H); 3.38 (dd, 1H); 4.11 (q. 2H); 5.70 (m, 1H); 7.11 (d,1H); 7.22 (d, 1H); 11.78 (sbr, 1H); ¹³C-NMR (CDCl₃, 75MHz, APT): 15.2 (CH₃); 28.7 (CH₂); 36.6 (CH₂); 48.1(C); 62.2 (CH₂); 117.8 (C); 117.9 (CH); 121.2 (C); 124.0 (C); 126.5 (CH); 127.9 (C); 155.0 (NC=O); 155.6 (NC=O); 171.0 (C=O); FD-MS: 284, 286 (M⁺); Analyse: C 43.98% (43.65); H 3.72% (3.40); N 7.13% (7.27); DC: (Dichlormethan-Methanol = 9:1) R_{f}= 0.53.

Die Herstellung der Ausgangsprodukte ist im Folgenden beschrieben:

### a) 3,4-Dihydro-2H-benzo(1,4)thiazin-3-yl-essigsäureethylester

8 g (34 mMol) (4H-Benzo(1,4)thiazin-3-yliden)-essigsäureethylster wird in 50 ml Ethanol gelöst, 0.5 ml 0.1%-ige ethanolische Bromkresolgrün-Lösung und 0.5 ml 5N HCl/Ethanol zugegeben. Man fügt 2.14 g (34 mMol) Natriumcyanoborhydrid in 4 Portionen bei Raumtemperatur zu und hält das Reaktionsgemisch durch tropfenweise Zugabe von HCl/Ethanol sauer. Nach 2 Stunden ausrühren bei Raumtemperatur zeiht man das Lösungsmittel am RV ab, nimmt in Essigsäureethylester auf und wäscht mit Wasser und gesättigter Kochsalzlösung. Die organischen Phasen werden über Magnesiumsulfat getrocknet, eingeengt und auf einer Kieselgelsäule gereinigt (Laufmittel: Petrolether - Ethylacetat = 8:2). Man erhält 6.1 g (25.6 mMol) = 75% (3.4-Dihydro-2H-benzo(1,4)thiazin-3-yl)-essigsäureethylester als gelbes Öl; ¹H-NMR (CDCl₃, 200MHz): 1.28 (t, 3H); 2.51-2.89 (m, 3H); 3.05 (dd, 1H); 4.07 (m, 1H); 4.17 (q, 2H); 4.60 (sbr, 1H, NH); 6.48 (dd, 1H); 6.62 (m, 1H); 6.85-7.02 (m, 2H); ¹³C-NMR (CDCl₃, 50MHz): 15.3; 31.4; 41.6; 48.4; 61.8; 116.7 (2x); 119.1; 126.9; 128.6; 142.1; 172.8; FD-MS: 237 (M+); DC: (Petrolether-Essigsäureethylester = 9:1) R_{f} = 0.24.

### b) 7-Brom-3,4-dihydro-2H-benzo(1,4)thiazin-3-yl-essigsäureethylester

4.9 g (20.6 mMol) (3,4-Dihydro-2H-benzo(1,4)thiazin-3-yl)-essigsäureethylester wird in 45 ml Dimethylformamid bei 0° tropfenweise mit einer Lösung von 3.7 g (20.6 mMol) N-Bromsuccinimid in 45 ml Dimethylformamid versetzt, 18 Stunden bei Raumtemperatur gerührt, danach am Hochvakuum bei 50° vom Lösungsmittel befreit. Das resultierende Öl wird in Dichlormethan aufgenommen, mit Wasser und gesättigter Kochsalzlösung gewaschen, die organischen Phasen über Magnesiumsulfat getrocknet, eingeengt und an Kieselgel (Petrolether-Essigsäureethylester = 9:1) chromatographiert. Man erhält 6,3 g (97%) 7-Brom-3,4-dihydro-2H-benzo(1,4)thiazin-3-yl-essigsäureethylester als gelbes Öl; ¹H-NMR (CDCl₃, 300MHz): 1.28 (t, 3H); 2.54-2.84 (m, 3H); 3.00 (dd,1H); 4.06 (m, 1H); 4.18 (q, 2H); 4.68 (sbr, 1H, NH); 6.36 (d, 1H); 6.97 (dd, 1H); 7.11 (d, 1H): ¹³C-NMR (CDCl₃, 75MHz): 15.2; 31.1; 41.5; 48.3; 61.9; 110,3: 117.9: 118.4; 129.6: 130.6: 141.1; 172.7; FD-MS: 315, 317 (M⁺); DC: (Petrolether-Essigsäureethylester = 9:1) R_{f} = 0.09.

### c) 2-(7-Brom-3-ethoxycarbonylmethyl-2,3-dihydro-benzo(1,4)thiazin-4-yl)-2-oxo-essigsäureethylester

6.66 g (21.06 mMol) (7-Brom-3,4-dihydro-2H-benzo(1,4)thiazin-3-yl-essigsäureethylester und 5.9 ml (42.12 mMol) Triethylamin in 5 ml Dichlormethan werden bei 0° unter Argon mit 2.8 ml (25.27 mMol) Oxalsäuremonoethylesterchlorid versetzt, 2 Stunden bei Raumtemperatur gerührt, dann mit 40 ml Dichlormethan verdünnt und mit gesättigter Kochsalzlösung, 2N Salzsäure, Wasser und nochmals gesättigter Kochsalzlösung gewaschen. Die organischen Phasen werden über Magnesiumsulfat getrocknet, eingeengt und an Kieselgel (Petrolether-Essigsäureethylester = 17:3) chromatographiert. Man erhält 8.1 g (19.5 mMol) (96.4%) (7-Brom-3-ethoxycarbonylmethyl-2,3-dihydro-benzo(1,4)thiazin-4-yl)-oxo-essigsäureethylester als gelbes-oranges Öl; ¹H-NMR (CDCl₃, 200MHz): vermutlich Gemisch der Rotamere: 1.10-1.30 (2xt,3H); 2.55 (dbr, 2H); 3.00 (ddbr, 1H); 3.49 (ddbr, 1H); 4.02-4.18 (quart,m, 4H); 5.49 (m, 1H); 6.89 (d, 1H); 7.18 (dd, 1H); 7.40 (d, 1H). FD-MS: 415, 417 (M⁺); DC: (Petrolether-Essigsäureethylester = 7:3) R_{f} = 0.28.

### Beispiel 5: 8-Brom-4,5-dioxo-2,3,5,6-tetrahydro-4H-1-thia-3a,6-diaza-phenalen-3-yl-essigsäure

4.1 g (10.64 mMol) 8-Brom-4,5-dioxo-2,3,5,6-tetrahydro-4H-1-thia-3a,6-diaza-phenalen-3-yl-essigsäureethylester wird in 180 ml Methanol bei Raumtemperatur 4 Stunden mit 26.6 ml 2N wässr. Natronlauge gerührt, danach mit 48 ml 2N Salzsäure sauergestellt. Die entstandene Fällung wird abfiltriert, mit viel Wasser neutralgewaschen und Hochvakuum-getrocknet (60°). Man erhält 3.62 g (10.12 mMol) = 95% 8-Brom-4,5-dioxo-2,3,5,6-tetrahydro-4H-1-thia-3a,6-diaza-phenalen-3-yl-essigsäure als weisses Pulver; Schmelzpunkt: >300°; ¹H-NMR (d₆-DMSO, 300MHz): 2.55 (dd, 1H); 2.76 (dd, 1H); 3.27 (m, 2H); 5.48 (m, 1H); 7.06 (d, 1H); 7.22 (d, 1H); 12.11 (sbr, 1H); 12.59 (sbr, 1H); ¹³C-NMR (CDCl₃, 75MHz, APT): 28.0 (CH₂); 36.5 (CH₂); 46.9 (C); 62.2 (CH₂); 115.6 (C); 115.7 (CH); 121.6 (C); 123.4 (C); 124.1 (CH); 129.0 (C); 154.1 (NC=O); 154.9 (NC=O); 172.4 (C=O); FD-MS: 356, 358 (M⁺); Analyse: C 40.69% (40.35); H 2.67% (2.54); N 7.88% (7.84).

### Beispiel 6: 8-Brom-4,5-dioxo-2,3,5,6-tetrahydro-4H-1-thia-3a,6-diaza-phenalen-3-yl-essigsäure Natriumsalz

Eine Suspension von 200 mg (560 mMol) 8-Brom-4,5-dioxo-2,3,5,6-tetrahydro-4H-1-thia-3a,6-diaza-phenalen-3-yl-essigsäure in 40 ml Wasser wird mit 5.6 ml 0.1N Natriumhydrogencarbonatlösung versetzt, 1 Stunde unter Rückfluss gekocht, filtriert und Iyophilisiert.

### Beispiel 7: 8-Brom-4,5-dioxo-2,3,5,6-tetrahydro-4H-1-thia-3a,6-diaza-phenalen-3-yl-essigsäure Kaliumsalz

Herstellung analog Beispiel 6.

### Beispiel 8: 8-Brom-4,5-dioxo-2,3,5,6-tetrahydro-4H-1-thia-3a,6-diaza-phenalen-3-yl-essigsäuretriethylammoniumsalz

Eine Suspension von 200 mg (560 mMol) 8-Brom-4,5-dioxo-2,3,5,6-tetrahydro-4H-1-thia-3a,6-diaza-phenalen-3-yl-essigsäure in 40 ml 1N Triethylammonium-hydrogencarbonat wird während 1 Stunde auf 60° erhitzt, filtriert, am RV/Hochvakuum eingeengt und aus 8 ml Wasser Iyophilisiert.

### Beispiel 9: 2-(8-Brom-4,5-dioxo-2,3,5,6-tetrahydro-4H-1-thia-3a,6-diaza-phenalen-3-yl)-N-phenyl-acetamid

u einer Lösung von 714.4 mg (2 mMol) 8-Brom-4,5-dioxo-2,3,5,6-tetrahydro-4H-1-thia-3a,6-diaza-phenalen-3-yl-essigsäure, 540.5 mg (4 mMol) 1-Hydroxybenzotriazol und 766.8 mg (4 mMol) N-Ethyl-N'-(3-dimethylaminopropyl)-carbodiimid-hydrochlorid (EDC) in 10 ml Dimethylformamid wird bei Raumtemperatur unter Argon 274 µl (3 mMol) Anilin zugefügt und 24 Stunden gerührt. Man gießt auf Eiswasser, filtriert die Fällung ab, wäscht mit viel Wasser und trocknet am Hochvakuum (60°). Man erhält 741.5 mg (1.72 mMol) = 86% 2-(8-Brom-4,5-dioxo-2,3,5,6-tetrahydro-4H-1-thia-3a,6-diaza-phenalen-3-yl)-N-phenyl-acetamid als beiges Pulver; Smp: 285-290°; ¹H-NMR (d₆-DMSO, 200MHz): 2.62 (dd,1H); 2.90 (dd, 1H); 3.30 (m, 2H); 5.68 (m, 1H); 7.06 (m, 2H); 7.30 (m, 3H); 7.55 (m, 2H); 10.00 (sbr,1H); 12.10 (sbr, 1H); El-MS: 433, 431 (M⁺); 313, 311; 299, 297; Analyse: enthält 0.41% Wasser: C 50.00% (49.80); H 3.35% (3.30); N 9.49% (9.68).

### Beispiel 10:

In analoger Weise wie in Beispiel 9 beschrieben können folgende Amide mit den entsprechenden freien Aminen (primär. sekundär, Aniline) als Kupplungspartner hergestellt werden.

### 2-(8-Brom-4,5-dioxo-2,3,5,6-tetrahydro-4H-1-thia-3a,6-diaza-phenalen-3-yl)-N-methyl-N-phenyl-acetamid;

Smp.: 148-152°; ¹H-NMR (d₆-DMSO, 200MHz): 2.27-2.85 (m, ca. 2H); 3.10-3.40 (m, ca. 2H); 3.18 (s, 3H); 5.55 (m, 1H); 7.00-7.48 (m, 7H); 12.05 (sbr, 1H); FD-MS: 445, 447 (M⁺); Analyse: C 49.69% (51.13); H 3.78% (3.61); N 10.43% (9.41);

### 2-(8-Brom-4,5-dioxo-2,3,5,6-tetrahydro-4H-1-thia-3a,6-diaza-phenalen-3-yl)-N-thiazol-2-yl-acetamid;

Ausbeute: 92%; Smp.: 260-261°; ¹H-NMR (d₆-DMSO, 300MHz): 2.72 (dd, 1H); 3.05 (dd, 1H); 3.27 (s, 2H); 5.67 (m, 1H); 7.09 (d, 1H): 7.22 (d, 1H); 7.28 (d, 1H); 7.46 (d, 1H); 12.14 (sbr, 1H) 12.26 (sbr, 1H); FAB-MS: 439, 414 (M⁺); Analyse: C 39.03% (41.01); H 3.19% (2.52); N 12.00% (12.75);

### 2-(8-Brom-4,5-dioxo-2,3,5,6-tetrahydro-4H-1-thia-3a,6-diaza-phenalen-3-yl)-N-cyclopropylmethyl-acetamid;

Ausbeute: 82%; Smp.: 250-253°; ¹H-NMR (d₆-DMSO, 300MHz): 0.12-0.14 (m, 2H); 0.36-0.40 (m, 2H); 0.84-0.89 (m, 1H); 2.35-2.68 (m, 2H); 2.89-2.95 (m, 2H); 3.19-3.29 (m, 2H); 5.55 (m, 1H); 7.07 (d, 1H); 7.25 (d, 1H); 8.04-8.08 (m, 1H) 12.11 (sbr, 1H); FD-MS: 409, 411 (M⁺); Analyse: C 45.19% (46.84); H 4.00% (3.93); N 9.78% (10.24);

### 2-(8-Brom-4,5-dioxo-2,3,5,6-tetrahydro-4H-1-thia-3a,6-diaza-phenalen-3-yl)-N-cyclopropylacetamid;

Ausbeute: 60%; Smp.: 180-185°; ¹H-NMR (d₆-DMSO, 300MHz): 0.34-0.39 (m, 2H); 0.55-0.61 (m, 2H); 0.84-0.89 (m, 1H); 2.29-2.61 (m, ca. 3H); 3.20 (sbr, 2H); 5.53 (m, 1H); 7.07 (d, 1H); 7.24 (d, 1H); 8.04 (d, 1H) 12.01 (sbr, 1H); FD-MS: 395, 397 (M⁺);

### 2-[2-(8-Brom-4,5-dioxo-2,3,5,6-tetrahydro-4H-1-thia-3a,6-diaza-phenalen-3-yl)-acetylamino]-3,3-dimethyl-buttersäure-tert.-butyl-ester;

Ausbeute: 96%; Smp.: 149-154°; ¹H-NMR (d₆-DMSO, 300MHz): 0.92 (d, 9H); 1.40 (d, 9H); 2.80-2.92 (m, 1H); 3.13-3.20 (m, 2H); 4.01 (dd, 1H); 5.55 (m, 1H); 7.07 (m, 1H); 7.26 (m, 1H); 8.08 (dd, 1H); 12.09 (sbr, 1H); FAB-MS: 527, 529 (M⁺+1); 492, 494 (M-tBu); Analyse: C 50.19% (49.32); H 3.35% (5.36); N 7.36% (7.98); DC: (Dichlormethan-Methanol = 9:1) R_{f} = 0.26;

### N-Adamantan-1-yl-2-(8-brom-4,5-dioxo-2,3,5,6-tetrahydro-4H-1-thia-3a,6-diaza-phenalen-3-yl)-acetamid;

Smp. 210-215°>; FD-MS: 489, 491 (M⁺); Analyse: C 53.51% (53.88); H 5.77% (4.93); N 10.00% (8.57).

### 2-(8-Brom-4,5-dioxo-2,3,5,6-tetrahydro-4H-1-thia-3a,6-diaza-phenalen-3-yl)-N,N-dibutylacetamid;

Ausbeute: 77%; ¹H-NMR (d₆-DMSO, 300MHz): 0.81-0.91 (m, 6H); 1.17-1.27 (m, 4H); 1.39-1.43 (m, 4H); 2.44-2.86 (m, 2H); 3.15-3.30 (m, 6H); 5.50 (m, 1H); 7.08 (m, 1H); 7.25 (m, 1H); 12.13 (sbr, 1H); FAB-MS: 468, 470 (M⁺+1); Analyse: C 50.67% (51.28); H 5.44% (5.59); N 9.07% (8.97).

### 2-(8-Brom-4,5-dioxo-2,3,5,6-tetrahydro-4H-1-thia-3a,6-diaza-phenalen-3-yl)-N-(2-oxo-azepan-3-yl)-acetamid;

Ausbeute: 45%; Smp.: 305-307°; ¹H-NMR (d₆-DMSO, 300MHz): 1.17-1.89 (m, 6H); 2.18-3.30 (m, 6H);4.38 (m, 1H); 5.53 (m, 1H); 7.07 (m, 1H); 7.25 (m, 1H); 7.77 (m, 1H); 7.97 (m, 1H); 12.11 (sbr, 1H); FAB-MS: 467, 469 (M⁺+1); Analyse: C 50.26% (50.67); H 4.62% (4.48); N 9.86% (9.33);

### N-Allyl-2-(8-brom-4,5-dioxo-2,3,5,6-tetrahydro-4H-1-thia-3a,6-diaza-phenalen-3-yl)-acetamid;

Ausbeute: 49%; Smp.: 150-153°; ¹H-NMR (d₆-DMSO, 300MHz): 2.42 (dd, 1H); 2.58 (dd, 1H); 3.21 (d, 2H); 3.69 (m, 2H); 5.03-5.15 (m, 2H); 5.56 (m, 1H); 5.74-5.84 (m, 1H); 7.07 (m, 1H); 7.24 (m, 1H); 7.77 (m, 1H); 8.16 (t, 1H); 12.11 (sbr, 1H).; EI-MS: 395, 397 (M⁺); Analyse: C 44.83% (45.47); H 3.69% (3.56); N 10.33% (10.60);

### 8-Brom-3-(2-(2,3-dihydro-indol-1-yl)-2-oxo-ethyl)-2,3-dihydro-6H-1-thia-3a,6-diazaphenalen-4,5-dion;

Ausbeute: 99% (beige Kristalle); Smp.: 270-273°; ¹H-NMR (d₆-DMSO, 300MHz): 3.00-3.62 (m, ca. 6H); 4.02 (d, 2H); 5.64 (m, 1H); 7.00-7.28 (m, 5H); 8.08 (d, 1H); 12.15 (sbr, 1H); FD-MS: 457,459 (M⁺); Analyse: C 51.20% (52.41); H 4.18% (3.52); N 10.20% (9.71);

### 8-Brom-3-(2-oxo-2-piperidin-1-yl-ethyl)-2,3-dihydro-6H-1-thia-3a,6-diaza-phenalen-4,5-dion;

Ausbeute: 89% beige Kristalle; Smp.: 170-172°; ¹H-NMR (d₆-DMSO, 300MHz): 1.40-1.60 (m, 8H); 2.30-3.60 (m, 6H); 5.52 (m, 1H); 7.07 (d. 1H); 7.24 (d, 1H); 12.10 (sbr, 1H); FD-MS: 423, 425 (M⁺); Analyse: C 47.60% (48.12); H 4.36% (4.28); N 9.80% (9.90);

### 8-Brom-3-(2-(2-methyl-aziridin-1-yl)-2-oxo-ethyl)-2,3-dihydro-6H-1-thia-3a,6-diazaphenalen-4,5-dion;

Ausbeute: 62% (beige Kristalle); Smp.: 193-195° ¹H-NMR (d₆-DMSO, 300MHz): 1.08 (m, 3H); 2.62 (m, 2H); 3.21-3.72 (m, ca. 5H); 3.90-4.41 (m , 1H); 5.57 (m, 1H); 7.07 (d, 1H); 7.24 (d, 1H); 12.10 (sbr, 1H); FD-MS: 395, 397 (M⁺); Analyse: C 43.81% (45.47); H 3.77% (3.56); N 11.66% (11.60).

### 2-(8-Brom-4,5-dioxo-2,3,5,6-tetrahydro-4H-1-thia-3a,6-diaza-phenalen-3-yl)-N-(2,2-dimethoxy-ethyl)-acetamid;

Ausbeute: 69% (beige Kristalle); Smp.: 190-192°; ¹H-NMR (d₆-DMSO, 300MHz): 2.28-2.72 (m, 2H); 3.10-3.20 (m, 4H); 3.21 (2xs, 6H); 4.32 (t, 1H); 5.58 (m, 1H); 7.07 (d, 1H); 7.24 (d, 1H); 8.09 (t, 1H); 12.10 (sbr, 1H); FD-MS: 443, 445 (M⁺); Analyse: C 41.74% (43.25); H 4.06% (4.08); N 9.10% (9.46);

### 4-[2-(8-Brom-4,5-dioxo-2,3,5,6-tetrahydro-4H-1-thia-3a,6-diaza-phenalen-3-yl)-acetylamino]-benzoesäuremethylester;

Ausbeute: 59% beige Kristalle: Smp,: 145-147°; ¹H-NMR (d₆-DMSO, 300MHz): 2.60-2.95 (m, 2H): 3.22 (m, 2H); 3.80 (s, 3H); 5.68 (m, 1H); 7.07 (d, 1H); 7.23 (d, 1H); 7.70 (d, 2H); 7.91 (d, 2H); 10.20 (s, 1H); 12.10 (sbr, 1H); FD-MS: 489, 491 (M⁺);

### 2-(8-Brom-4,5-dioxo-2,3,5,6-tetrahydro-4H-1-thia-3a,6-diaza-phenalen-3-yl)-N-(5,6,7,8-tetrahydro-naphthalen-1-yl)-acetamid;

Ausbeute: 97% beige Kristalle; Smp.: 323-325°; ¹H-NMR (d₆-DMSO, 300MHz): 1.62-1.73 (m, 4H); 2.60-2.95 (m, 6H); 3.22 (m, 2H); 5.65 (m, 1H); 6.90-7.30 (m, 5H); 9.25 (s, 1H); 12.15 (sbr, 1H); FD-MS: 485, 487 (M⁺); Analyse: C 54.28% (54.33); H 4.18% (4.14); N 9.51% (8.64);

### 2-(8-Brom-4,5-dioxo-2,3,5,6-tetrahydro-4H-1-thia-3a,6-diaza-phenalen-3-yl)-N-furan-2-ylmethyl-acetamid;

Ausbeute: 82% beige Kristalle; Smp.: 202-203°
¹H-NMR (d₆-DMSO, 300MHz): 2.20-2.71 (m, 2H); 3.21 (m, 2H); 4.23 (m, 2H); 5.68 (m, 1H); 6.22 (d, 1H); 6.39 (d, 1H); 7.07 (d, 1H); 7.22 (d, 1H); 7.58 (d, 1H); 8.48 (t, 1H); 12.10 (sbr, 1H); FD-MS: 435, 437 (M⁺); Analyse: C 46.31% (46.80); H 3.33% (3.23); N 9.66% (9.63).

### 2-(8-Brom-4,5-dioxo-2,3,5,6-tetrahydro-4H-1-thia-3a,6-diaza-phenalen-3-yl)-N-(2-methoxyethyl)-acetamid;

Ausbeute: 38% beige Kristalle; Smp.: 135-137°
¹H-NMR (d₆-DMSO, 300MHz): 2.20-2.71 (m, 2H); 3.21 (m, 6H); 3.23 (s, 3H): 5.68 (m, 1H); 7.07 (d, 1H); 7.22 (d, 1H); 8.08 (m, 1H); 12.12 (sbr, 1H); FD-MS: 413, 415 (M⁺); Analyse: C 42.96% (43.49); H 3.92% (3.89); N 10.01% (10.14).

### Beispiel 11: 1-[2-(8-Brom-4,5-dioxo-2,3,5,6-tetrahydro-4H-1-thia-3a,6-diaza-phenalen-3-yl)-acetylamino]-cyclopropan-carbonsäure-methylester

Zu einer Lösung von 259.6 mg (727 mMol) 8-Brom-4,5-dioxo-2,3,5,6-tetrahydro-4H-1-thia-3a,6-diaza-phenalen-3-yl-essigsäure, 196.4 mg (1454 mMol) 1-Hydroxybenzotriazol, 279 mg (1454 mMol) N-Ethyl-N'-(3-dimethylaminopropyl)-carbodiimid-hydrochlorid (EDC) und 153 µl (1090 mMol) Triethylamin in 10 ml Dimethylformamid wird bei Raumtemperatur unter Argon 165.2 mg (1090 mMol) 1-Aminocyclopropan-1-carbonsäuremethylester-hydrochlorid zugefügt und 48h gerührt. Man gießt auf angesäuertes Eiswasser, filtriert die Fällung ab, wäscht mit viel Wasser und trocknet am Hochvakuum (60°). Man erhält 220.3 mg (485 mMol) = 67% 1-(2-(8-Brom-4,5-dioxo-2,3,5,6-tetrahydro-4H-1-thia-3a,6-diaza-phenalen-3-yl)-acetylamino)-cyclopropan-carbonsäure-methylester als beiges Pulver;
Smp: 186-188°; ¹H-NMR (d₆-DMSO, 300MHz): 1.02 (m, 2H); 1.34 (m, 2H); 2.37-2.59 (m, ca. 3H); 3.21 (sbr. 2H); 3.58 (s,3H); 5.54 (m, 1H); 7.07 (m, 1H); 7.24 (m, 1H); 8.65 (s, 1H); 12.11 (sbr, 1H), FAB-MS: 454, 456 (M⁺); Analyse: C 44.95% (44.23); H 3.82% (3.55); N 9.24% (9.25).

### Beispiel 12:

Gemäss Beispiel 11 können folgende Amide in analoger Weise hergestellt werden mit Amin-hydrochloriden als Kupplungspartner:

### N-Adamantan-2-yl-2-(8-brom-4,5-dioxo-2,3,5,6-tetrahydro-4H-1-thia-3a,6-diaza-phenalen-3-yl)-acetamid;

Ausbeute: 91%: Smp.: 183-185°: ¹H-NMR (d₆-DMSO, 300MHz): 1.40-2.00 (m, ca. 15H): 2.74 (m, 1H): 3.19 (m, 2H); 3.82 (m, 1H); 5.57 (m, 1H); 7.07 (d, 1H); 7.26 (d, 1H); 7.84 (d, 1H); 12.10 (sbr, 1H); FAB-MS: 490, 492 (M⁺+1).
Analyse: C 53.58% (53.88); H 5.08% (4.93); N 8.67% (8.57);

### N-Bicyclo[2.2.1]hept-2-yl-2-(8-brom-4,5-dioxo-2,3,5,6-tetrahydro-4H-1-thia-3a,6-diazaphenalen-3-yl)-acetamid;

Ausbeute: 88%: Smp.: 215-222°; ¹H-NMR (d₆-DMSO, 300MHz): 0.8 - 1.8 (m, ca. 7H); 2.00-2.40 (m, ca. 3H); 2.65 (m, 1H); 3.16 (m, 2H); 3.87 (m, 1H); 5.54 (m, 1H); 7.07 (d, 1H); 7.25 (d, 1H); 7.91 (m, 1H); 12.10 (sbr, 1H); FAB-MS: 450, 452 (M⁺+1); Analyse: C 45.56% (46.26); H 4.26% (4.10); N 11.88% (11.99);

### 2-(8-Brom-4,5-dioxo-2,3,5,6-tetrahydro-4H-1-thia-3a,6-diaza-phenalen-3-yl)-N-tert.-butoxyacetamid;

Ausbeute: 29%; Smp.: 175-180°; ¹H-NMR (d₆-DMSO, 300MHz): 1.14 (s, 9H); 2.25-3.40 (m, 4H); 5.55 (m, 1H); 7.07 (m, 1H); 7.27 (m, 1H); 10.45 (sbr, 1H); 12.11 (sbr, 1H); FAB-MS: 428, 430 (M⁺+1);

### N-Benzyloxy-2-(8-brom-4,5-dioxo-2,3,5,6-tetrahydro-4H-1-thia-3a,6-diaza-phenalen-3-yl)-acetamid;

Ausbeute: 93%; Smp.: 140-148°; ¹H-NMR (d₆-DMSO, 300MHz): 2.28-2.45 (m, 2H); 3.12-3.23 (m, 2H): 4.77 (q, 2H); 5.55 (m, 1H); 7.07 (m, 1H); 7.24 (m, 1H); 7.38 (sbr, 5H); 11.14 (sbr, 1H); 12.12 (sbr, 1H); FAB-MS: 462, 464 (M⁺+1); Analyse: C 49.14% (49.36); H 3.77% (3.49); N 9.72% (9.09).

### 2-(8-Brom-4,5-dioxo-2,3,5,6-tetrahydro-4H-1-thia-3a,6-diaza-phenalen-3-yl)-N-prop-2-ynyl-acetamid;

Ausbeute: 73%; Smp.: 181-182°; ¹H-NMR (d₆-DMSO, 300MHz): 2.40 (dd, 1H); 2.68 (dd, 1H); 3.13 (s,1H); 3.20 (d, 2H); 3.85 (m, 2H); 5.54 (m, 1H); 7.07 (m, 1H); 7.24 (m, 1H); 8.46 (m, 1H); 12.11 (sbr, 1H); FAB-MS: 394, 396 (M⁺+1).
Analyse: C 44.92% (45.70); H 3.33% (3.07); N 10.43% (10.66);

### 2-[2-(8-Brom-4,5-dioxo-2,3,5,6-tetrahydro-4H-1-thia-3a,6-diaza-phenalen-3-yl)-acetylamino]-malonsäuredimethylester;

Ausbeute: 51% beige Kristalle; Smp.: 143-145°; ¹H-NMR (d₆-DMSO, 300MHz): 2.38 (m, 1H); 2.80 (m, 1H); 3.20 (m, 2H); 3.72 (s, 6H); 5.17 (d, 1H); 5.57 (m, 1H); 7.07 (m, 1H); 7.24 (m, 1H); 9.01 (t, 1H); 12.11 (sbr, 1H); FD-MS: 485, 487 (M⁺); Analyse: C 40.68% (41.99); H 3.47% (3.32); N 8.64% (8.64).

### 2-(8-Brom-4,5-dioxo-2,3,5,6-tetrahydro-4H-1-thia-3a,6-diaza-phenalen-3-yl)-N-cyanomethylacetamid;

Ausbeute: 54% beige Kristalle; Smp.: 212-214°
¹H-NMR (d₆-DMSO, 300MHz): 2.40-2.50 (m, 1H); 2.70 (m, 1H); 3.20 (m, 2H); 3.72 (s, 6H); 4.13 (d, 1H); 5.57 (m, 1H); 7.07 (m, 1H); 7.24 (m, 1H); 8.75 (t, 1H); 12.11 (sbr, 1H); FD-MS: 394, 396 (M⁺);

### 3-[2-(8-Brom-4,5-dioxo-2,3,5,6-tetrahydro-4H-1-thia-3a,6-diaza-phenalen-3-yl)-acetylamino]-propionsäuremethylester;

Ausbeute: 43% braune Kristalle;
Smp.: 240-242°; ¹H-NMR (d₆-DMSO, 300MHz): 2.25-2.75 (m, ca. 4H); 3.18 (s, 2H); 3.28 (t, 2H); 3.60 (s, 3H); 5.57 (m, 1H); 7.07 (m, 1H); 7.23 (m, 1H); 8.13 (t, 1H); 12.11 (sbr, 1H). FD-MS: 441, 443 (M⁺);

### N-Allyloxy-2-(8-brom-4,5-dioxo-2,3,5,6-tetrahydro-4H-1-thia-3a,6-diaza-phenalen-3-yl)-acetamid;

Ausbeute: 77% beige Kristalle; Smp.: 162-165°
¹H-NMR (d₆-DMSO, 300MHz): 2.23-2.40 (m, 2H); 3.18 (m, 2H): 4.23 (m, 2H): 5.25 2xd, 2H); 5.57 (m, 1H); 5.90 (m, 1H); 7.07 (m, 1H); 7.24 (m, 1H); 11.09 (sbr, 1H); 12.11 (sbr, 1H); FD-MS: 411, 413 (M⁺); Analyse: C 43.53% (43.70); H 3.72% (3.42): N 10.47% (10.19);

### 2-(8-Brom-4,5-dioxo-2,3,5,6-tetrahydro-4H-1-thia-3a,6-diaza-phenalen-3-yl)-N-methoxyacetamid;

Ausbeute: 90% beige Kristalle; Smp.: 182-185°
¹H-NMR (d₆-DMSO, 300MHz): 2.23-2.40 (m, 2H); 3.19 (m, 2H): 3.58 (s, 3H); 5.57 (m, 1H); 5.90 (m, 1H); 7.07 (m, 1H); 7.24 (m, 1H); 11.14 (sbr, 1H); 12.10 (sbr, 1H); FD-MS: 385, 387 (M⁺); Analyse: C 39.03% (40.43); H 3.50% (3.13); N 10.24% (10.88);

### Beispiel 13: 2-[2-(8-Brom-4,5-dioxo-2,3,5,6-tetrahydro-4H-1-thia-3a,6-diaza-phenalen-3-yl)-acetylamino]-benzoesäuremethylester

Eine Lösung von 754.5 mg (2.11 mMol) 8-Brom-4,5-dioxo-2,3,5,6-tetrahydro-4H-1-thia-3a,6-diaza-phenalen-3-yl-essigsäure, 590.6 mg (2.32 mMol) N,N-Bis-(2-oxo-3-oxazolidinyl)-phosphinsäurechlorid (BOP-CI), 705.3 µl Triethylamin und 300.5 µl Anthranilsäuremethylester in 10 ml Dimethylformamid wird unter Argon während 3h auf 60° erwärmt, dann 590.6 mg (2.32 mMol) BOP-CI (= Bis-(oxo-3-oxazolodinyl)-phosphinsäurechlorid)und 705.3 µl Triethylamin zugefügt und weitere 2h bei 60° und 15h bei Raumtemperatur gerührt. Man gießt auf angesäuertes Eiswasser, filtriert die entstandene Fällung, wäscht sie mit viel Wasser neutral und trocknet am Hochvakuum (60°). Man erhält 987.2 mg (2.01 mMol) = 95% 2-(2-(8-Brom-4,5-dioxo-2,3,5,6-tetrahydro-4H-1-thia-3a,6-diaza-phenalen-3-yl)-acetylamino)-benzoesäuremethylester als beiges Pulver; Smp.: 165-175°; ¹H-NMR (d₆-DMSO, 300MHz): 2.72-2.97 (m, 2H); 3.24-3.33 (m, 2H); 3.83 (s, 3H); 5.63 (m, 1H); 7.09 (d, 1H); 7.22 (t, 1H); 7.28 (d, 1H); 7.61 (t, 1H); 7.89 (d, 1H); 8.12 (d, 1H); 10.58 (sbr, 1H) 12.13 (sbr, 1H); FAB-MS: 490, 492 (M⁺+1); Analyse: C 46.98% (48.99); H 3.51% (3.29); N 8.70% (8.57).

### Beispiel 14: Analog Beispiel 13 kann auch 3-[2-(8-Brom-4,5-dioxo-2,3,5,6-tetrahydro-4H-1-thia-3a,6-diaza-phenalen-3-yl)-acetylamino]-but-2-en-säuremethylester;

Ausbeute: 47% beiges Pulver; Smp.: 160-167°; FD-MS: 453, 455 (M⁺); Analyse: C 45.38% (44.95); H 3.80% (3.55); N 9.35% (9.25) hergestellt werden

### Beispiel 15: 2-[2-(8-Brom-4,5-dioxo-2,3,5,6-tetrahydro-4H-1-thia-3a,6-diaza-phenalen-3-yl)-acetylamino]-benzoesäure

Eine Aufschlämmung von 149 mg (303.9 mMol) 2-(2-(8-Brom-4,5-dioxo-2,3,5,6-tetrahydro-4H-1-thia-3a,6-diaza-phenalen-3-yl)-acetylamino)-benzoesäuremethylester in 80 ml Methanol wird mit 5 ml 2N Natronlauge versetzt und 4.5h bei Raumtemperatur gerührt. Man gibt 8 ml 2N Salzsäure zu, zieht das Methanol am RV ab, filtriert die entstandene Fällung ab, wäscht sie mit viel Wasser neutral und trocknet am Hochvakuum (60°). Man erhält 105 mg (220 mMol) = 73% 2-(2-(8-Brom-4,5-dioxo-2,3,5,6-tetrahydro-4H-1-thia-3a,6-diazaphenalen-3-yl)-acetylamino)-benzoesäure als beiges Pulver; Smp.: 205-212°; ¹H-NMR (d₆-DMSO, 300MHz): 2.72-2.97 (m, 2H); 3.23-3.33 (m, 2H); 5.63 (m, 1H); 7.09 (d, 1H); 7.17 (t, 1H); 7.27 (d, 1H); 7.60 (t, 1H); 7.86 (d, 1H); 8.39 (d, 1H); 11.21 (sbr, 1H) 12.12 (sbr, 1H); FD-MS: 475, 477 (M⁺); Analyse: C 45.04% (47.91): H 3.03% (2.96); N 8.35% (8.82).

### Beispiel 16:

In analoger Weise wie in Beispiel 15 beschrieben können auch folgende Verbindungen hergestellt werden.

### 3-[2-(8-Brom-4,5-dioxo-2,3,5,6-tetrahydro-4H-1-thia-3a,6-diaza-phenalen-3-yl)-acetylamino]-propionsäure;

Ausbeute: 79% beiges Pulver;
Smp.: 284-286°; ¹H-NMR (d₆-DMSO, 300MHz): 2.25-2.75 (m, ca. 4H); 3.18-3.28 (m, ca. 4H); 5.59 (m, 1H); 7.07 (d, 1H); 7.23 (d, 1H); 8.08 (t, 1H); 12.15 (sbr, 2H); FD-MS: 427, 429 (M⁺);

### [2-(8-Brom-4,5-dioxo-2,3,5,6-tetrahydro-4H-1-thia-3a,6-diaza-phenalen-3-yl)-acetylamino]-essigsäure;

( unter Decarboxylierung); Ausbeute: 42% weisse Kristalle; Smp.: 193-195°; ¹H-NMR (d₆-DMSO, 300MHz): 2.20-3.75 (m, ca. 6H); 5.57 (m, 1H); 7.07 (d, 1H); 7.24 (d, 1H); 8.18 (t, 0.5H); 8.67 (d, 0.5H); 12.13 (sbr, 1H).
FD-MS: 413, 415 (M⁺);

### Beispiel 17: 1-[2-(8-Brom-4,5-dioxo-2,3,5,6-tetrahydro-4H-1-thia-3a,6-diaza-phenalen-3-yl)-acetylamino]-cyclopropan-carbonsäure

Eine Lösung von 84.3 mg (186 mMol) 1-(2-(8-Brom-4,5-dioxo-2,3,5,6-tetrahydro-4H-1-thia-3a,6-diaza-phenalen-3-yl)-acetylamino)-cyclopropan-carbonsäuremethylester in 40 ml Dioxan wird mit 10 ml 2N Salzsäure versetzt 18h bei Raumtemperatur gerührt. Man gibt 20 ml Salzsäure zu, rührt bei Raumtemperatur 24h und engt am RV ein. Die entstandene Fällung wird mit viel Wasser neutralgewaschen und am Hochvakuum (60°) getrocknet. Man erhält 53.2 mg (121 mMol) = 65% 1-(2-(8-Brom-4,5-dioxo-2,3,5,6-tetrahydro-4H-1-thia-3a,6-diaza-phenalen-3-yl)-acetylamino)-cyclopropan-carbonsäure als beiges Pulver; Smp.: 300-302°; ¹H-NMR (d₆-DMSO,300MHz): 0.97 (m, 2H); 1.30 (m, 2H); 2.36-2.63 (m, ca. 3H); 3.21 (sbr, 2H); 5.52 (m, 1H); 7.07 (m, 1H); 7.24 (m, 1H); 8.55 (s, 1H); 12.09 (sbr, 1H); 12.36 (sbr, 1H); FAB-MS: 440, 442 (M⁺)

### Beispiel 18: 2-[2-(8-Brom-4,5-dioxo-2,3,5,6-tetrahydro-4H-1-thia-3a,6-diaza-phenalen-3-yl)-acetylamino]-3,3-dimethyl-buttersäure

Eine Lösung von 113 mg (214.6 mMol) 2-(2-(8-Brom-4,5-dioxo-2,3,5,6-tetrahydro-4H-1-thia-3a,6-diaza-phenalen-3-yl)-acetylamino)-3,3-dimethyl-buttersäuretert.-butyl-ester in 5 ml Dichlormethan wird mit 2 ml Trifluoressigsäure versetzt und 5h bei Raumtemperatur gerührt. Die Lösungsmittel werden am RV abgezogen, der Rückstand in 1 ml Dimethylformamid gelöst und in 50 ml Eiswasser eingerührt. Das ausgefallene Produkt wird abfiltriert, mit viel Wasser gewaschen und Hochvakuum getrocknet (60°). Man erhält 32.8 mg (70 mMol) = 33% 2-(2-(8-Brom-4,5-dioxo-2,3,5,6-tetrahydro-4H-1-thia-3a,6-diaza-phenalen-3-yl)-acetylamino)-3,3-dimethyl-buttersäure als weisses Pulver; Smp.: 195-198°: ¹H-NMR (d₆-DMSO, 300MHz): 0.93 (d, 9H); 2.85-2.96 (m, 1H); 3.15-3.19 (m, 2H); 4.10 (dd, 1H); 5.55 (m, 1H); 7.07 (m, 1H); 7.26 (m, 1H); 8.10 (dd, 1H); 12.12 (sbr, 1H); 12.55 (sbr, 1H); FAB-MS: 470, 472 (M⁺+1); DC: (Dichlormethan-Methanol = 9:1) R_{f} = 0.07.

### Beispiel 19: 2,2-Dimethyl-propionsäure-(8-brom-4,5-dioxo-2,3,5,6-tetrahydro-4H-1-thia-3a,6-diaza-phenalen-3-yl)-acetoxymethylester

Eine Gemisch von 201.4 mg (563.9 mMol) 8-Brom-4,5-dioxo-2,3,5,6-tetrahydro-4H-1-thia-3a,6-diaza-phenalen-3-yl-essigsäure, 42.9 mg (310.1 mMol) K₂CO₃ und 98.3 µg (676.6 mMol) Pivalsäurechlormethylester in 5 ml Dimethylformamid wird über Nacht bei Raumtemperatur gerührt. Man gießt auf Eiswasser, engt bei 50° leicht ein, filtriert das ausgefallene Produkt ab, wäscht mit Wasser und trocknet am Hochvakuum (60°). Man erhält 56.5 mg (120 mMol) = 21% 2,2-Dimethyl-propionsäure-(8-brom-4,5-dioxo-2,3,5,6-tetrahydro-4H-1-thia-3a,6-diaza-phenalen-3-yl)-acetoxymethylester als weisses Pulver; Smp.: 163-167°; ¹H-NMR (d₆-DMSO, 300MHz): 1.07-1.24 (m, 9H); 2.65-2.93 (m, 2H); 3.20 (m, 2H); 5.48 (m, 1H); 5.70 (m, 2H); 7.06 (d, 1H); 7.25 (d, 1H); 12.14 (sbr, 1H); FD-MS: 470, 472 (M⁺).

### Beispiel 20: In analoger Weise wie in Beispiel 19 beschrieben kann auch Benzoesäure-(8-brom-4,5-dioxo-2,3,5,6-tetrahydro-4H-1-thia-3a,6-diaza-phenalen-3-yl)-acetoxymethylester;

Ausbeute: 71%; Smp.: 140-148°; ¹H-NMR (d₆-DMSO, 300MHz): 2.65-2.98 (m, 2H); 3.20 (m, 2H); 5.52 (m, 1H); 5.96 (m, 2H); 7.07 (d, 1H); 7.24 (m, 1H); 7.57 (m, 2H); 7.70 (m, 1H); 8.00 (m, 2H); 12.11 (sbr, 1H); FD-MS: 490, 492 (M⁺); Analyse: C 48.89% (48.89); H 3.23% (3.08); N 5.96% (5.70) hergestellt werden.

### Beispiel 21: In analoger Weise wie in Beispiel 9 beschrieben kann auch 8-Brom-4,5-dioxo-2,3,5,6-tetrahydro-4H-1-thia-3a,6-diaza-phenalen-3-yl-essigsäuremethoxycarbonyl-methylester;

Ausbeute: 56%.; Smp.: 185-191°; ¹H-NMR (d₆-DMSO, 300MHz): 2.65-2.98 (m, 2H); 3.24 (m, 2H): 3.62 (s, 3H); 4.73 (s, 2H); 5.53 (m, 1H); 7.08 (d, 1H): 7.26 (d, 1H); 12.13 (sbr, 1H); FD-MS: 428,430 (M⁺); Analyse: C 41.16% (41.97); H 3.14% (3.05); N 7.69% (6.53) hergestellt werden.

### Beispiel 22: 2-(8-Brom-4,5-dioxo-2,3,5,6-tetrahydro-4H-1-thia-3a,6-diaza-phenalen-3-yl)-acetamid

In einem verschraubbaren Bombenrohr wird 1.0 g (2.6 mMol) 8-Brom-4,5-dioxo-2,3,5,6-tetrahydro-4H-1-thia-3a,6-diaza-phenalen-3-yl-essigsäureethylester in 30 ml 4N methanolischer Ammoniaklösung während 8h auf 100° erhitzt. Die ausgefallene Produkt wird abfiltriert, mit Ethanol gewaschen und Hochvakuum-getrocknet (60°). Man erhält 370 mg (1.04 mMol) = 40% 2-(8-Brom-4,5-dioxo-2,3,5,6-tetrahydro-4H-1-thia-3a,6-diaza-phenalen-3-yl)-acetamid als graues Pulver; Smp.: >320°; ¹H-NMR (d₆-DMSO, 300MHz): 2.32-2.70 (m, 2H); 3.17-3.30 (m, 2H); 5.52 (m, 1H); 6.99 (sbr); 7.06 (d, 1H); 7.24 (d, 1H); 7.45 (sbr, 1H); 11.58 (sbr, 1H); FD-MS: 355, 357 (M⁺); Analyse: C 40.55% (40.46); H 3.19% (2.83); N 11.40% (11.80); DC: (Dichlormethan-Methanol = 9:1) R_{f} = 0.07.

### Beispiel 23: 8-Brom-4,5-dioxo-2,3,5,6-tetrahydro-4H-1-thia-3a,6-diaza-phenalen-3-yl-acetonitril

Zu einer auf 90° erwärmten Lösung von 770 mg (2.16 mMol) 2-(8-Brom-4,5-dioxo-2,3,5,6-tetrahydro-4H-1-thia-3a,6-diaza-phenalen-3-yl)-acetamid in 20 ml Dimethylformamid wird unter Argon 396 µl (4.32 mMol) Phosphoroxychlorid zugespritzt und 2h gerührt. Man gießt auf Eiswasser, filtriert den entstandenen Niederschlag ab, wäscht mit viel Wasser und trocknet am Hochvakuum (60°). Das so erhaltene Zwischenprodukt (Iminchlorid) wird über wenig Kieselgel mit Dichlormethan, filtriert. getrocknet, in 30 ml Dioxan gelöst und mit 3.16 ml 1N Natronlauge 2h bei Raumtemperatur hydrolisiert. Man gießt erneut auf Eiswasser, säuert mit wenig Salzsäure an, filtriert die entstandene Fällunf ab, wäscht mit Wasser und trocknet am Hochvakuum (60°). Man erhält 421.5 mg (1.25 mMol) = 58% 8-Brom-4,5-dioxo-2,3,5,6-tetrahydro-4H-1-thia-3a,6-diaza-phenalen-3-yl-acetonitril als ockergelbes Pulver; Smp.: 235-250°; ¹H-NMR (d₆-DMSO, 300MHz): 2.95-2.99 (m, 2H); 3.27 (m, 2H); 5.55 (m, 1H); 7.09 (d, 1H); 7.29 (d, 1H); 12.19 (s, 1H); FD-MS: 337, 339 (M⁺)
Analyse: (enthält ca. 1 eq. Wasser): C 40.53% (42.62); H 2.67% (2.38); N 11.52% (12.43); DC: (Dichlormethan-Methanol = 19:1) R_{f} = 0.11.

### Beispiel 24: 8-Brom-3-(2H-tetrazol-5-ylmethyl)-2,3-dihydro-6H-1-thia-3a,6-diaza-phenalen-4,5-dion

Eine Lösung von 200 mg (591 mMol) 8-Brom-4,5-dioxo-2,3,5,6-tetrahydro-4H-1-thia-3a,6-diaza-phenalen-3-yl-acetonitril, 115.3 mg (1.774 mMol) Natriumazid und 122.1 mg (887 mMol) Triethylamin-hydrochlorid in 5 ml N-Methyl-2-pyrrolidon wird während 4h unter N₂ auf 150° erhitzt. Man lässt abkühlen, gießt dann auf 100 ml Eiswasser, säuert mit Salzsäure an und extrahiert 2x mit je 150 ml Essigsäureethylester, wäscht die organischen Phasen mit gesättigter Kochsalzlösung, trocknet über Magnesiumsulfat, zieht das Lösungsmittel am RV ab und trocknet am Hochvakuum (60°). Das resultierende Öl wird in 5 ml Dimethylformamid aufgenommen und das Produkt durch einrühren in 100 ml angesäuertes Eiswasser gefällt. Die Fällung wird mit Wasser gewaschen und Hochvakuum-getrocknet. Man erhält 101.3 mg (266.4 mMol) = 45% 8-Brom-3-(2H-tetrazol-5-ylmethyl)-2,3-dihydro-6H-1-thia-3a,6-diaza-phenalen-4,5-dion als braunes Pulver; Smp.: 238-242°; ¹H-NMR (d₆-DMSO, 300MHz): 3.20-3.40 (m, 4H); 5.58 (m, 1H); 7.10 (d, 1H); 7.30 (d, 1H); 12.14 (s, 1H) 16.20 (sbr, 1H); FD-MS: 380, 382 (M⁺)

### Beispiel 25: 3-(S)-8-Brom-4,5-dioxo-2,3,5,6-tetrahydro-4H-1-thia-3a,6-diaza-phenalen-3-yl-essigsäureethylester

Enantiomerenreinner 3-(S)-8-Brom-4,5-dioxo-2,3,5,6-tetrahydro-4H-1-thia-3a,6-diaza-phenalen-3-yl-essigsäureethylester wird erhalten durch HPL-chromatographische Trennung des Racemates. Säule: Chiracel OJ 10x50cm; Hexan-Ethanol = 7:3; 150 ml/min; ca. 45 bar: Det.: 220 nm. Nachreinigung der enantiomerenreinen Fraktionen (Entfernung von Phthalaten) mittels Flash-Säulenchromatographie: Kieselgel; Dichlormethan-Methanol = 9:1.; (α)_{D} = + 104.4° (c = 0.25, Dimethylformamid); ¹H-NMR (CDCl₃, 300MHz): identisch mit Racemat (Beispiel 4); EI-MS: 384, 386 (M⁺); Analyse: C 44.42% (43.65); H 3.73% (3.40); N 7.06% (7.27).

### Beispiel 26: 3-(R)-8-Brom-4,5-dioxo-2,3,5,6-tetrahydro-4H-1-thia-3a,6-diaza-phenalen-3-yl-essigsäureethylester

Herstellung analog aus Racematspaltung gemäß Beispiel 25 (α)_{D} = - 111.2° (c = 0.25, Dimethylformamid); ¹H-NMR (CDCl₃ 300MHz): identisch mit Racemat (Beispiel 1). EI-MS: 384, 386 (M+); Analyse: C 43.61% (43.65); H 3.59% (3.40); N 6.93% (7.27).

### Beispiel 27: 3-(S)-8-Brom-4,5-dioxo-2,3,5,6-tetrahvdro-4H-1-thia-3a,6-diaza-phenalen-3-yl-essigsäure

Herstellung analog Beispiel 5 aus enantiomerenreinem 3-(S)-8-Brom-4,5-dioxo-2,3,5,6-tetrahydro-4H-1-thia-3a,6-diaza-phenalen-3-yl-essigsäureethylester: (α)_{D} = + 94.4° (c = 0.25, Dimethylformamid); ¹H-NMR (CDCl₃, 300MHz): identisch mit Racemat (Beispiel 2); EI-MS: 356, 358 (M⁺); Analyse: C 40.38% (40.35); H 3.15% (2.54); N 7.36% (7.84).

### Beispiel 28: 3-(R)-8-Brom-4,5-dioxo-2,3,5,6-tetrahydro-4H-1-thia-3a,6-diaza-phenalen-3-yl-essigsäure

Herstellung analog Beispiel 5 aus enantiomerenreinem 3-(R)-8-Brom-4,5-dioxo-2,3,5,6-tetrahydro-4H-1-thia-3a,6-diaza-phenalen-3-yl-essigsäureethylester: (α)_{D} = - 87.6° (c = 0.25, Dimethylformamid); ¹H-NMR (CDCl₃, 300MHz): identisch mit Racemat (Beispiel 2); EI-MS: 356, 358 (M⁺); Analyse: C 40.35% (40.35); H 3.09% (2.54); N 7.23% (7.84).

### Beispiel 29: 2-(3-(S)-8-Brom-4,5-dioxo-2,3,5,6-tetrahydro-4H-1-thia-3a,6-diaza-phenalen-3-yl)-N-phenyl-acetamid

Herstellung analog Beispiel 5 aus enantiomerenreiner 3-(S)-8-Brom-4,5-dioxo-2,3,5,6-tetrahydro-4H-1-thia-3a,6-diaza-phenalen-3-yl-essigsäure; α)_{D} = + 128.0° (c = 0.25, Dimethylformamid); ¹H-NMR (CDCl₃, 300MHz): identisch mit Racemat (Beispiel 5); EI-MS: 431, 433 (M⁺); Analyse: C 49.95% (50.01); H 3.64% (3.26); N 9.19% (9.72).

### Beispiel 30: 2-(3-(R)-8-Brom-4,5-dioxo-2,3,5,6-tetrahvdro-4H-1-thia-3a,6-diaza-phenalen-3-yl)-N-phenyl-acetamid

Herstellung analog Beispiel 5 aus enantiomerenreiner 3-(R)-8-Brom-4,5-dioxo-2,3,5,6-tetrahydro-4H-1-thia-3a,6-diaza-phenalen-3-yl-essigsäure; (α)_{D} = - 124.4° (c = 0.25, Dimethylformamid; ¹H-NMR (CDCl₃, 300MHz): identisch mit Racemat (Beispiel 5); EI-MS: 431,433 (M⁺); Analyse: C 49.96% (50.01); H 3.69% (3.26); N 9.36% (9.72).

### Beispiel 31: 8-Chlor-2,3,5,6-dihydro-6H-1-thia-3a,6-diaza-phenalen-4,5-dion

Herstellung aus (7-Chlor-2,3-dihydro-benzo[1,4]thiazin-4-yl)-oxo-essigsäureethylester analog Beispiel 4. Ausbeute: 24%. Eine Lösung von 1.0 g (2.6 mMol) 8-Brom-4,5-dioxo-2,3,5,6-tetrahydro-4H-1-thia-3a,6-diaza-phenalen-3-yl-essigsäureethylester in 30 ml Eisessig und 25 ml 30%-iger Wasserstoffperoxidlösung wird während 18 Stunden auf 100° erhitzt, dann fast zur Trochne eingeengt und mit 300 ml Eiswasser versetzt. Das ausgefallene Produkt wîrd abfiltriert, mit Wasser neutralgewaschen und Hochvakuum-getrocknet (60°). Man erhält 670 mg (1.61 mMol) = 62% 8-Brom-1,1,4,5-tetraoxo-2,3,5,6-tetrahydro-1H,4H-6-thia-3a,6-diaza-phenalen-3-yl-essigsäureethylester als weisses Pulver; Smp.: >330°; ¹H-NMR (d₆-DMSO, 300MHz): 3.20-3.23 (m, 2H); 4.25-4.29 (m, 2H); 6.93 (d, 1H); 7.12 (d, 1H); 12.11 (sbr, 1H); EI-MS: 254,256 (M⁺+1); Analyse: C 47.15% (47.16); H 2.77% (2.81); N 10.92% (11.00); DC: (Dichlormethan-Methanol = 19:1) R_{f}= 0.32.

Die Herstellung des Ausgangsproduktes ist im Folgenden beschrieben:

### a) (7-Chlor-2,3-dihydro-benzo[1,4]thiazin-4-yl)-oxo-essigsäureethylester

Herstellung aus 7-Chlor-3,4-dihydro-2H-benzo(1,4)thiazin (CAN: 106016-84-6) analog Beispiel 4b; ¹H-NMR (CDCl₃, 300MHz): 1.14 (t, 3H); 3.21 (t, 2H); 4.03 (t, 2H); 4.16 (quart, 2H); 6.97 (m, 2H); 7.22 (d, 1H); FD-MS: 285, 287 (M⁺); DC: (Dichlormethan-Petrolether = 1:1) R_{f} = 0.10.

### Beispiel 32: 8-Chlor-4,5-dioxo-2,3,5,6-tetrahydro-4H-1-thia-3a,6-diaza-phenalen-3-yl-essigsäureethylester

Herstellung analog Beispiel 4; Ausbeute: 33%; Smp.: 222-226°; ¹H-NMR (CDCl₃, 200MHz): 1.25 (t, 3H); 2.69 (dd, 1H); 3.00 (dd, 1H); 3.26 (dd, 1H); 3.93 (dd, 1H); 4.18 (q, 2H); 5.78 (m, 1H); 7.08 (dd, 1H); 7.12 (dd, 1H); 12.65 (sbr, 1H); FD-MS: 340, 342 (M⁺)
DC: (Dichlormethan-Methanol = 9:1) R_{f} = 0.56.

Die Herstellung der Ausgangsprodukte ist im Folgenden beschrieben:

### a) (7-Chlor-3,4-dihydro-2H-benzo[1,4]thiazin-3-yl-essigsäureethylester

Hergestellt analog Beispiel 4a ausgehend von (7-Chlor-4H-benzo(1,4)-3-yliden)-essigsäureethylester; Ausbeute: 78%; ¹H-NMR (CDCl₃, 300MHz): 1.28 (t, 3H); 2.57 (dd, 1H); 2.70 (dd, 1H); 2.81 (dd, 1H); 3.01 (dd, 1H); 4.06 (m, 1H); 4.18 (q, 2H); 4.67 (sbr, 1H, NH); 6.40 (d, 1H); 6.84 (dd, 1H); 6.98 (dd, 2H); ¹³C-NMR (CDCl₃, 75MHz): 15.2; 31.2; 41.5; 48.3; 61.9; 117.5, 117.9; 123.4; 126.7; 127.8; 140.6; 172.7; DC: (Petrolether-Essigsäureethylester = 9:1) R_{f} = 0.12.

### b) (7-Chlor-3-ethoxycarbonylmethyl-2,3-dihydro-benzo(1,4)thiazin-4-yl)-oxo-essigsäureethylester

Herzustellen analog Beispiel 4c; ¹H-NMR (CDCl₃, 300MHz): vermutlich Gemisch der Rotamere: 1.09-1.25 (2xt, 3H); 2.56 (m, 2H); 3.00 (ddbr, 1H); 3.48 (ddbr, 1H); 4.11 (quart, 4H); 5.49 (m, 1H); 6.94 (d, 1H); 7.02 (dd, 1H); 7.25 (d, 1H); FD-MS: 371, 373 (M⁺)
DC: (Hexan-Essigsäureethylester = 4:1) R_{f} = 0.21.

### Beispiel 33: 8-Chlor-4,5-dioxo-2,3,5,6-tetrahydro-4H-1-thia-3a,6-diaza-phenalen-3-yl-essigsäure

Herstellung analog Beispiel 5; Ausbeute: 82%; Schmelzpunkt: >300°; ¹H-NMR (d₆-DMSO, 300MHz): 2.56 (dd, 1H); 2.77 (dd, 1H); 3.28 (m, 2H); 5.50 (m, 1H); 6.95 (d, 1H); 7.15 (d, 1H); 12.13 (sbr, 1H); 12.58 (sbr, 1H); FD-MS: 312, 314 (M⁺).

### Beispiel 34: 2-(8-Chlor-4,5-dioxo-2,3,5,6-tetrahydro-4H-1-thia-3a,6-diaza-phenalen-3-yl)-N-phenyl-acetamid

Herstellung analog Beispiel 9; Ausbeute: 64%; Schmelzpunkt: >300°; ¹H-NMR (d₆-DMSO, 300MHz): 2.62 (dd, 1H); 2.90 (dd, 1H); 3.28 (m, 2H); 5.67 (m, 1H); 6.96 (d, 1H); 7.04 (m, 1H); 7.17 (d, 1H); 7.30 (m, 2H): 7.55 (d, 2H); 10.04 (s, 1H); 12.15 (sbr, 1H).
FD-MS: 387, 389 (M⁺); Analyse: C 55.61% (55.74); H 3.90% (3.64); N 10.94% (10.83).

### Beispiel 35: 8-Brom-1,1,4,5-tetraoxo-2,3,5,6-tetrahydro-1H,4H-6-thia-3a,6-diaza-phenalen-3-yl-essigsäureethylester

Eine Lösung von 1.0 g (2.6 mMol) 8-Brom-4,5-dioxo-2,3,5,6-tetrahydro-4H-1-thia-3a,6-diaza-phenalen-3-ylessigsäureethylester in 30 ml Eisessig und 25 ml 30%-iger Wasserstoffperoxidlösung wird während 18h auf 100° erhitzt, dann fast zur Trochne eingeengt und mit 300 ml Eiswasser versetzt. Das ausgefallene Produkt wîrd abfiltriert, mit Wasser neutralgewaschen und Hochvakuum-getrocknet (60°). Man erhält 670 mg (1.61 mMol) = 62% 8-Brom-1,1,4,5-tetraoxo-2,3,5,6-tetrahydro-1H, 4H-6-thia-3a,6-diaza-phenalen-3-ylessigsäureethylester als weisses Pulver; Smp.: 257-261°; ¹H-NMR (d₆-DMSO, 300MHz): 1.20 (t, 3H); 2.68-2.90 (m, 1H); 3.08-3.25 (m, 1H); 4.05 (m, 2H); 4.11 (q, 2H); 5.62 (m, 1H); 7.50 (d, 1H); 7.73 (d, 1H); 12.50 (sbr, 1H); FD-MS: 416, 418 (M⁺); Analyse: C 38.51% (40.30); H 3.09% (3.14); N 6.84% (6.71); DC: (Dichlormethan-Hexan-Methanol = 6:3:1) R_{f} = 0.21.

### Beispiel 36: 8-Brom-1,1,4,5-tetraoxo-2,3,5,6-tetrahydro-1H,4H-6-thia-3a,6-diaza-phenalen-3-yl-essigsäure

Herstellung analog Beispiel 5; Ausbeute: 83%.; chmelzpunkt: 281-284°; ¹H-NMR (d₆-DMSO, 300MHz): 2.50 (dd, 1H); 3.18 (dd. 1H); 4.05 (m, 2H); 5.58 (m, 1H); 7.50 (d, 1H); 7.72 (d, 1H); 12.50 (sbr, 2H); FD-MS: 388, 390 (M⁺); Analyse: C 37.26% (37.03); H 2.49% (2.33); N 7.19% (7.20).

### Beispiel 37: 2-(8-Brom-1,1,4,5-tetraoxo-2,3,5,6-tetrahydro-1H,4H-6-thia-3a,6-diaza-phenalen-3-yl)-N-phenyl-acetamid

Herstellung analog Beispiel 5; Ausbeute: 88%; Schmelzpunkt: 185-190°; ¹H-NMR (d₆-DMSO, 200MHz): 2.75-2.90 (m, 1H); 3.15-3.25 (m, 1H); 4.05 (m, 2H); 5.80 (m, 1H); 7.08 (m. 1H); 7.30 (m, 2H); 7.56 (m, 3H); 7.77 (m, 1H); 10.09 (s, 1H); 12.50 (sbr, 1H); FD-MS: 463, 465 (M⁺); Analyse: C 46.48% (46.56); H 3.47% (3.04); N 9.87% (9.05); DC: (Dichlormethan-Methanol = 9:1) R_{f}= 0.20.

### Beispiel 38: 8-Chlor-4,5-dioxo-2,3,5,6-tetrahydro-4H-1-oxa-3a,6-diaza-phenalen-3-yl-essigsäureethylester

Eine Lösung von 4.2 g (15.51 mMol) 5-Amino-7-chlor-3,4-dihydro-2H-benzo(1,4)oxazin-3-ylessigsäureethylester in 52 ml Oxalsäurediethylester wird am Rotationsverdampfer während 18h bei 80° und 60 mbar rotiert. Der entstandene Festkörper wird in Et₂O aufgeschlämmt, filtriert, mit 250 ml Et₂O gewaschen und Hochvakuum-getrocknet (80°). Man erhält 3.35 g (10.31 mMol) = 66% 8-Chlor-4,5-dioxo-2,3,5,6-tetrahydro-4H-1-oxa-3a,6-diaza-phenalen-3-ylessigsäureethylester als weisse Kristalle; Smp.: 265°: ¹H-NMR (d₆-DMSO, 300MHz): 1.16 (t, 3H); 2.62 (m, 2H); 4.07 (q, 2H); 4.12 (m, 1H); 4.48 (d, 1H); 4.97 (m, 1H); 6.79 (d, 1H); 6.88 (d, 1H); 12.14 (sbr, 1H); FD-MS: 324, 326 (M⁺).

Die Herstellung der Ausgangsprodukte ist im Folgenden beschrieben:

### a) 2-(2-Chlormethyl-4-nitro-2,3-dihydro-benzo-oxazol-2-yl)-essigsäureethylester

Eine Gemisch von 30 g (194.6 mMol) 2-Hydroxy-6-nitroanilin und 26.5 ml 4-Chlor-acetessigsäureethylester in 130 ml Benzol wird während 12h am Wasserabscheider gekocht. Das Lösungsmittel wird abgezogen und der braune, halbkristalline Rückstand an Kieselgel flash-chromatographiert (Laufmittel: Petrolether-Essigsäureethylester = 9:1). Man erhält 49.7 g (165.3 mMol) = 85% (2-Chlormethyl-4-nitro-2,3-dihydro-benzo-oxazol-2-yl)-essigsäureethylester als leuchtend-orange Kristalle; Smp.: 90-91°; ¹H-NMR (CDCl₃, 300MHz): 1.24 (t, 3H); 3.09 (d, 1H); 3.28 (d, 1H); 3.92 (dd, 2H); 4.19 (q, 2H); 6.63 (t, 1H); 6.82 (d, 1H); 7.09 (sbr, 1H, NH); 7.48 (d, 1H); ¹³C-NMR (CDCl₃, 50MHz): 14.5; 41.6; 48.1; 62.1; 100.6; 112.2; 116.4; 118.7; 135.8; 151.5; 169.2; FD-MS: 300, 302 (M⁺); DC: (Petrolether-Essigsäureethylester = 9:1) R_{f}= 0.23.

### b) 2-(6-Chlor-2-chlormethyl-4-nitro-2,3-dihydro-benzoxazol-2-yl)-essigsäureethylester

Eine Lösung von 45 g (149.7 mMol) (2-Chlormethyl-4-nitro-2,3-dihydro-benzo-oxazol-2-yl)-essigsäureethylester und 24 g (179.6 mMol) N-Chlorsuccinimid in 500 ml Dimethylformamid wird während 18h auf 60° erwärmt, Man engr am RV/Hochvakuum ein, nimmt den braunorangen Rückstand in Dichlormethan auf, wäscht mit 2x Wasser und gesättigter Kochsalzlösung, trocknet über Magnesiumsulfat, und engt am RV (50°) ein. Man erhält 58.2 g (>100%) (6-Chlor-2-chlormethyl-4-nitro-2,3-dihydro-benzoxazol-2-yl)-essigsäureethylester als rot-oranges, NMR-reines Öl; ¹H-NMR (CDCl₃, 200MHz): 1.26 (t, 3H); 3.18 (AB-Syst., 2H); 3.90 (dd, 1H); 4.18 (q, 2H); 6.69 (dd, 1H); 7.12 (sbr, 1H, NH); 7.48 (d, 1H); DC: (Dichlormethan-Hexan = 7:3) R_{f} = 0.27.

### c) (7-Chlor-5-nitro-4H-benzo(1,4) oxazin-3-yliden)-essigsäureethylester

Zu einer Suspension von 4.7 g (107.4 mMol) Natriumhydrid (als 55%-ige ölige Suspension) in 150 ml THF wird bei 0-5° eine Lösung von 30 g (89.51 mMol) 6-Chlor-2-chlormethyl-4-nitro-2,3-dihydro-benzoxazol-2-yl)-essigsäureethylester in 150 ml THF zugetropft, 30 min. gerührt, dann auf RT erwärmt und 1 Stunde gerührt. Man zieht das Lösungsmittel am RV (50°) ab und chromatographiert das halbkristalline, schwarze Öl an Kieselgel (Dichlormethan-Hexan = 7:3) . Nach Kristallisation aus Ether/Hexan erhält man 20.3 g (68 mMol) (76%) (7-Chlor-5-nitro-4H-benzo(1,4)oxazin-3-yliden)-essigsäureethylester als orange Kristalle; Smp.: 162-163°; ¹H-NMR (CDCl₃, 300MHz): 1.33 (t, 3H); 4.28 (q, 2H); 4.67 (d, 2H); 4.98 (s, 1H); 7.18 (dd, 1H); 7.89 (d, 1H); 12.27 (sbr, 1H); DC: (Dichlormethan-Hexan = 7:3) R_{f}= 0.40.

### d) 5-Amino-7-chlor-4H-benzo(1,4)oxazin-3-ylidenessigsäureethylester

7.0 g (23.44 mMol) 7-Chlor-5-nitro-4H-benzo(1,4)oxazin-3-ylidenessigsäureethylester werden in 300 ml Ethanol mit 2 g Raney-Nickel bei Raumtemperatur und Normaldruck während 6h hydriert. Man filtriert das Reaktionsgemisch, engt ein und chromatographiert an Kieselgel (Dichlormethan-Hexan = 7:3) . Nach Kristallisation aus Ethanol/Hexan erhält man 5.67 g (21.1 mMol) (90%) 5-Amino-7-chlor-4H-benzo(1,4)oxazin-3-ylidenessigsäureethylester als feine rosa Nadeln; Smp.: 158-159°
¹H-NMR (CDCl₃, 200MHz): 1.28 (t, 3H); 3.55 (sbr, 2H, NH₂); 4.17 (q, 2H); 4.51 (s, 2H); 4.72 (s, 1H); 6.42 (d, 1H); 6.45 (d, 1H); 9.95 (sbr, 1H, NH); FD-MS: 268,270 (M⁺); DC: (Dichlormethan-Hexan = 7:3) R_{f} = 0.21.

### e) 5-Amino-7-chlor-3,4-dihydro-2H-benzo(1,4)oxazin-3-ylessigsäureethylester

Herstellung analog 4a; Ausbeute 94% gelbliches Öl: ¹H-NMR (CDCl₃, 300MHz): 1.27 (t, 3H); 2.55 (d, 2H); 3.38 (sbr, 3H, NH, NH₂); 3.89-3.95 (m, 2H): 4.14-4.23 (m, 3H); 4.17 (q, 2H); 6.33 (dd, 1H); 6.38 (dd, 1H); ¹³C-NMR (CDCl₃, 50MHz): 14.7; 37.2; 47.3; 61.4; 68.8; 108.8; 109.5; 119.7; 125.0; 137.2; 145.8; 171.9; DC: (Dichlormethan) R_{f} = 0.13.

### Beispiel 39: 8-Chlor-4,5-dioxo-2,3,5,6-tetrahydro-4H-1-oxa-3a,6-diaza-phenalen-3-yl-essigsäure

Herstellung analog Beispiel 5; Ausbeute: 98% weiss-beige Kristalle; Schmelzpunkt: 236-240°; ¹H-NMR (d₆-DMSO, 200MHz): 2.52 (t, 3H); 4.12 (dd, 1H); 4.50 (d, 2H); 4.94 (td, 1H); 6.83 (d, 1H); 6.88 (d, 1H); 12.13 (s, 1H); 12.65 (sbr, 1H); FD-MS: 296, 298 (M⁺); Analyse: C 44.31% (46.36); H 3.43% (3.43); N 8.57% (9.01); Cl 4.56% (4.58).

### Beispiel 40: 8-Chlor-4,5-dioxo-2,3,5,6-tetrahydro-4H-1-oxa-3a,6-diaza-phenalen-3-yl-essigsäurenatriumsalz

Herstellung analog Beispiel 2a.

### Beispiel 41: 2-(8-Chlor-4,5-dioxo-2,3,5,6-tetrahydro-4H-1-oxa-3a,6-diaza-phenalen-3-yl)-N-phenyl-acetamid

Herstellung analog Beispiel 9; Ausbeute: 93% leicht beige Kristalle: Smp.: 263-265°;
¹H-NMR (d₆-DMSO, 200MHz): 2.60-2.73 (m, 2H); 4.15 (dd, 1H); 4.50 (d. 2H); 5.10 (m, 1H); 6.82 (d, 1H); 6.88 (d, 1H); 7.05 (t, 1H); 7.30 (t, 2H); 7.55 (d, 2H); 10.05 (s, 1H); 12.10 (sbr, 1H); FD-MS: 371,373 (M⁺); Analyse: C 55.11% (58.15); H 4.03% (3.80); N 11.11% (11.30); Cl 9.94% (9.54).

### Beispiel 42: In analoger Weise wie in Beispiel 41 beschrieben kann auch 2-(8-Chlor-4,5-dioxo-2,3,5,6-tetrahvdro-4H-1-oxa-3a,6-diaza-phenalen-3-yl)-N-methyl-N-phenyl-acetamid;

Ausbeute: 82% beige Kristalle; ¹H-NMR (d₆-DMSO, 300MHz): 2.32 (d, 2H); 3.15 (s, 3H); 4.07 (d, 1H); 4.45 (d, 1H); 4.97 (m, 1H); 6.70 (d, 1H); 6.78 (d, 1H); 7.28-7.45 (m, 5H); FD-MS: 385, 387 (M⁺); Analyse: C 58.08% (59.15); H 4.34% (4.18); N 12.00% (10.89); DC: (Dichlormethan-Methanol-NH₃ = 700:50:1) R_{f} = 0.37; hergestellt werden.

### Beispiel 43: N-Adamantan-1-yl-2-(8-chlor-4,5-dioxo-2,3,5,6-tetrahydro-4H-1-oxa-3a,6-diaza-phenalen-3-yl)-acetamid

Herstellung analog Beispiel 9, Beiges Pulver; Smp.: 235-238°; ¹H-NMR (d₃-DMSO 200MHz): 1.60-2.05 (m, ca. 15H); 2.32-2.46 (m, 2H); 4.10 (dd, 1H); 4.38 (d, 1H); 4.11 (q, 2H); 4.97 (m, 1H); 6.78 (d, 1H); 6.88 (d, 1H); 7.50 (sbr, 1H); 12.10 (sbr, 1H); FD-MS: 429, 431 (M⁺).

### Beispiel 44: 2-(8-Chlor-4,5-dioxo-2,3,5,6-tetrahydro-4H-1-oxa-3a,6-diaza-phenalen-3-yl)-N,N-bis-(2-hydroxy-ethyl)-acetamid

Herstellung analog Beispiel 9; Ausbeute: 15% weisse Kristalle; Smp.: 220-222°; ¹H-NMR (d₃-DMSO 300MHz): 1.60-1.82 (m, 2H); 3.42 (m, 6H); 4.12 (d, 1H); 4.25 (d, 1H); 4.72 (dbr, 2H); 4.97 (d, 1H); 6.80 (d, 1H); 6.88 (d, 1H); 12.10 (sbr, 1H); FD-MS: 383, 385 (M⁺)

### Beispiel 45: 2,3-Dihydro-6H-1-oxa-3a,6-diaza-phenalen-4,5-dion

Herstellung analog Beispiel 38; Ausbeute 75% gelbe Kristalle; Smp.: 291°; ¹H-NMR (d₆-DMSO, 300MHz): 4.03 (t, 2H); 4.32 (t, 2H,); 4.03 (t, 2H); 6.69-6.77 (m, 2H); 7.00-7.05 (m, 1H); 12.02 (s br, 1H, HN); ¹³C-NMR (d₆-DMSO, 74MHz): 40.35; 63.61; 108.08; 110.66; 114.06; 123.97; 126.62; 143.88; 153.29; 154.22; FD-MS: 204 (M⁺); Analyse: C 58.68% (58.82); H 4.04% (3.95); N 13.33% (13.72).

### Beispiel 46: 4,5-Dioxo-2,3,5,6-tetrahydro-4H-1-oxa-3a,6-diaza-phenalen-3-yl-essigsäure ethylester

Analog Beispiel 38; Ausbeute: 79% weisse Kristalle; Smp.: 256-257°; ¹H-NMR (d₆-DMSO, 300MHz): 1.71 (t, 3H); 2.66 (m, 2H); 4.09 (q, 2H); 4.15 (m, 1H); 4.48 (d, 1H); 5.00 (tbr, 1H); 6.74-6.83 (m, 2H); 7.07 (t, 1H); 12.08 (sbr, 1H); ¹³C-NMR (d₆-DMSO, 50MHz): 14.1; 33.9; 46.7; 60.8; 66.1; 108.5; 110.7; 113.3; 124.1; 126.8; 143.3; 153.0; 154.2; 170.2;
EI-MS: 290 (M⁺); Analyse: C 57.79% (57.93); H 5.03% (4.86); N 9.70% (9.65).

Die Herstellung der Ausgangsprodukte ist im Folgenden beschrieben:

### a) 5-Nitro-4H-benzo(1,4) oxazin-3-ylidenessigsäureethylester

Herstellung analog Beispiel 37c; Ausbeute: 97% gelbe Kristalle; Smp.: 142°; ¹H-NMR (CDCl₃, 300MHz): 1.33 (t, 3H); 4.28 (q, 2H); 4.66 (s, 2H); 4.94 (s, 1H); 7.16 (t, 1H); 7.19 (d, 1H); 7.89 (dd, 1H); 12.25 (sbr, 1H); FD-MS: 264 (M⁺); DC: (Petrolether-Essigsäureethylester = 9:1) R_{f}= 0.43.

### b) 5-Amino-3,4-dihydro-2H-benzo(1,4)oxazin-3-ylessigsäureethylester

1.0g (3.78 mMol) 5-Nitro-4H-benzo(1,4)oxazin-3-ylidenessigsäureethylester wird bei Raumtemperatur bis 50° in 50 ml Ethanol mit 0.2 g 10% Pd/C bei Nornaldruck während 50h hydriert. Man filtriert das Reaktionsgemisch, engt ein und chromatographiert an Kieselgel (Petrolether-Essigsäureethylester = 8:2). Man erhält 753 mg (3.19 mMol) = 84% 5-Amino-3,4-dihydro-2H-benzo(1,4)oxazin-3-yl-essigsäureethylester als oranges Öl; ¹H-NMR (CDCl₃, 200MHz): 1.25 (t, 3H); 2.55 (d, 2H); 3.40 (sbr, 3H); 3.80-3.97 (m, 2H); 4.05-4.15 (m, 1H); 4.18 (q, 2H); 6.31-6.40 (m, 2H); 6.60 (t, 1H); FD-MS: 236 (M⁺); DC: (Essigsäureethylester-Petrolether = 7:3) R_{f} = 0.17.

### Beispiel 47: 7,9-Dibrom-4,5-dioxo-2,3,5,6-tetrahydro-4H-1-oxa-3a,6-diaza-phenalen-3-yl-essigsäureethylester

Herstellung analog Beispiel 4b mit 2 Mol N-Bromsuccinimid. Ausbeute: 92% weisse Kristalle; Smp.: 285-290°; ¹H-NMR (d₃-DMSO 300MHz): 1.18 (t, 3H); 2.61 (d, 2H); 4.08 (q, 2H); 4.15 (d, 1H); 4.60 (d, 1H); 5.02 (m, 1H); 7.68 (s, 1H); 11.28 (sbr, 1H); FD-MS: 446, 448, 450 (M⁺).

### Beispiel 48: 8-Brom-4,5-dioxo-2,3,5,6-tetrahydro-4H-1oxa-3a,6-diaza-phenalen-3-yl-essigsäureethylester

Herstellung analog Beispiel 38; Ausbeute 15% beige Kristalle; Smp.: 222-227°; ¹H-NMR (d₆-DMSO, 200MHz): 1.17 (t, 3H); 2.62 (m, 2H); 4.08 (q, 2H); 4.12 (m, 1H); 4.50 (d, 1H); 4.97 (m, 1H); 6.95 (d, 1H); 7.00 (d, 1H); 12.11 (sbr, 1H); FD-MS: 368, 370 (M⁺).

Die Herstellung der Ausgangsprodukte ist im Folgenden beschrieben:

### a) 5-Nitro-3,4-dihydro-2H-benzo(1,4)oxazin-3-ylessigsäureethylester

145 mg (3.33 mMol) 55% Natriumhydrid-Dispersion in 5 ml THF werden bei 0°-5° mit einer Lösung von 1.0 g (3.33 mMol) (2-Chlormethyl-4-nitro-2,3-dihydro-benzo-oxazol-2-yl)-essigsäureethylester in 5 ml THF versetzt. Man rührt 15 min. bei 0° und 1 Stunde bei Raumtemperatur. Nun wird 2.1 g (33.26 mMol) Natriumcyanoborhydrid zugefügt, auf 0° gekühlt und tropfenweise 15 ml 5N ethanolische Salzsäure zugefügt. Man engt am RV ein, nimmt den Rückstand in Dichlormethan auf, wäscht mit Wasser und gesättigter Kochsalzlösung, trocknet die organischen Phasen über Magnesiumsulfat, engt ein und flash-chromatographiert das orange Rohprodukt. Man erhält 389 mg (1.46 mMol) = 44% (5-Nitro-3,4-dihydro-2H-benzo(1,4)oxazin-3-yl)-essigsäureethylester als oranges Öl; ¹H-NMR (CDCl₃, 200MHz): 1.28 (t, 3H); 2.63 (m, 2H); 3.96-4.20 (m, 3H); 4.21 (q, 2H); 6.56 (t, 1H); 7.00 (d, 1H); 7.78 (d, 1H); 8.21 (sbr, 1H, NH); FD-MS: 266 (M⁺); DC: (Dichlormethan-Hexan = 8:2) R_{f} = 0.28.

### b) 7-Brom-5-nitro-3,4-dihydro-2H-benzo(1,4)oxazin-3-ylessigsäureethylester

Herstellung analog Beispiel 4b; Ausbeute: 53% oranges Öl; ¹H-NMR (CDCl₃, 200MHz): 1.29 (t, 3H); 2.61 (d, 2H); 3.98-4.20 (m, 3H); 4.21 (q, 2H); 7.10 (d, 1H); 7.92 (d, 1H); 8.26 (sbr, 1H, NH); DC: (Dichlormethan-Hexan = 8:2) R_{f} = 0.38;

### c) 5-Amino-7-brom-3,4-dihydro-2H-benzo(1,4)oxazin-3-ylessigsäureethylester

Eine Lösung von 248 mg (0.72 mMol) (7-Brom-5-nitro-3,4-dihydro-2H-benzo(1,4)oxazin-3-yl)-essigsäureethylester in 10 ml Ethanol wird mit 811 mg (3.6 mMol) Zinn-(II)-chloriddihydrat versetzt und bei 80° 5h gerührt. Das Lösungsmittel wird abgezogen, der Rückstand in Dichlormethan aufgenommen und 2x mit Wasser gewaschen. Die organischen Phasen wurden über Magnesiumsulfat getrocknet und eingeengt. Man erhält 225 mg (5-Amino-7-brom-3,4-dihydro-2H-benzo(1,4)oxazin-3-yl)-essigsäureethylester als braunes Öl, das ohne Reinigung weiterverwendet wird.

### Beispiel 49: 8-Chlor-4,5-dioxo-2,3,5,6-tetrahydro-4H-1-oxa-3a,6-diaza-phenalen-3-yl-essigsäurebutylester

3.0 g (10.11 mMol) 8-Chlor-4,5-dioxo-2,3,5,6-tetrahydro-4H-1-oxa-3a,6-diaza-phenalen-3-yl-essig-säure werden in ca. 100 ml CCl₄, 1.02 ml (11.12 mMol) 1-Butanol und in 27.1 µl konzentrierter Schwefelsäure über Nacht am Wasserabscheider unter Rückfluss erhitzt, nochmals 15 ml 1-Butanol und 100 µl konzentrierter Schwefelsäure zugefügt und 6 Stunden erhitzt. Man zieht die Lösungsmittel ab, nimmt in Dichlormethan auf und wäscht mit Wasser, gesättigter Natriumhydrogencarbonatlösung und gesättigter Kochsalzlösung, trocknet über Magnesiumsulfat, zieht das Lösungsmittel und kristallisiert aus Dichlormethan - Petrolether. Man erhält 2.59 g (7.34 mMol) = 73% 8-Chlor-4,5-dioxo-2,3,5,6-tetrahydro-4H-1-oxa-3a,6-diaza-phenalen-3-yl-essigsäurebutylester als braun-beige Kristalle; ¹H-NMR (d₆-DMSO, 200MHz): 0.87 (t, 3H); 1.30 (m, 2H); 1.50 (m, 2H); 2.65 (m, 2H); 4.00 (t, 2H); 4.15 (dd, 1H); 4.50 (dbr, 1H); 4.97 (t(m), 1H); 6.81 (d, 1H); 6.88 (d, 1H); 12.15 (sbr, 1H); FD-MS: 352, 354 (M⁺); DC: (Dichlormethan-Methanol = 19:1) R_{f} = 0.33.

### Beispiel 50: 8-Methyl-4,5-dioxo-2,3,5,6-tetrahydro-4H-1-thia-3a,6-diaza-phenalen-3-yl-essigsäureethylester

Herstellung analog Beispiel 4.

Die Herstellung der Ausgangsprodukte ist im folgenden beschrieben:
a) (7-Methyl-3,4-dihydro-2H-benzo(1,4)thiazin-3-yl-essigsäureethylester
   Herzustellen aus (7-Methyl-4H-benzo(1,4)-3-yliden)-essigsäureethylester analog Beispiel 4a; ¹H-NMR (CDCl₃, 300MHz): 1.28 (t, 3H); 2.57 (dd, 1H); 2.70 (dd, 1H); 2.81 (dd, 1H); 3.01 (dd, 1H); 4.06 (m, 1H); 4.18 (q, 2H); 4.67 (sbr, 1H, NH); 6.40 (d, 1H); 6.84 (dd, 1H); 6.98 (dd, 2H); DC: (Petrolether-Essigsäureethylester = 9:1) R_{f} = 0.12.
b) 2-(7-Methyl-3-ethoxycarbonylmethyl-2,3dihydro-benzo(1,4)thiazin-4-yl)-2-oxo-essigsäureethylester
   Herzustellen analog Beispiel 4c; ¹H-NMR (CDCl₃, 300MHz): vermutlich Gemisch der Rotamere: 1.09-1.25 (2xt, 3H); 2.56 (m, 2H); 3.00 (ddbr, 1H); 3.48 (ddbr, 1H); 4.11 (quart, 4H); 5.49 (m, 1H); 6.94 (d, 1H); 7.02 (dd, 1H); 7.25 (d, 1H); FD-MS: 371, 373 (M⁺);
   DC: (Hexan-Essigsäureethylester = 4:1) R_{f} = 0.21.

### Beispiel 52:

In analoger Weise wie in den Beispielen 4 bis 48 beschrieben kann man auch 8-Methyl-4,5-dioxo-2,3,5,6-tetrahydro-4H-1-thia-3a,6-diaza-phenalen-3-yl-essigsäure herstellen.

### Beispiel 52:

In analoger Weise wie in den Beispielen 1 bis 51 beschrieben kann man auch folgende Verbindungen der Formel I herstellen:
2-(8-Brom-4,5-dioxo-2,3,5,6-tetrahydro-4H-1-thia-3a,6-diaza-phenalen-3-yl)-N-ethoxyacetamid, Smp. 202-204°;
2-(8-Brom-4,5-dioxo-2,3,5,6-tetrahydro-4H-1-thia-3a,6-diaza-phenalen-3-yl)-N-hydroxyacetamid, Smp. 220-221°;
2-(8-Brom-4,5-dioxo-2,3,5,6-tetrahydro-4H-1-thia-3a,6-diaza-phenalen-3-yl)-N-(2,2,2-trifluorethyl)-acetamid, Smp. 270-271°;
2-(8-Brom-4,5-dioxo-2,3,5,6-tetrahydro-4H-1-thia-3a,6-diaza-phenalen-3-yl)-N-methoxy-N-methyl-acetamid, Smp. 201-202°;
2-[2-(8-Brom-4,5-dioxo-2,3,5,6-tetrahydro-4H-1-thia-3a,6-diaza-phenalen-3-yl)acetylomino)-essigsäureethylester, Smp. 200-201°;
2-[2-(8-Brom-4,5-dioxo-2,3,5,6-tetrahydro-4H-1-thia-3a,6-diaza-phenalen-3-yl)acetylamino)-propionsäuremethylester, Smp. 283-285°;
2-(8-Brom-4,5-dioxo-2,3,5,6-tetrahydro-4H-1-thia-3a,6-diaza-phenalen-3-yl)-N-(2,3,4,5,6-pentafluorbenzyloxy)-acetamid, Smp. 147-148°;
2-(8-Brom-4,5-dioxo-2,3,5,6-tetrahydro-4H-1-thia-3a,6-diaza-phenalen-3-yl)-N-phenoxy-acetamid, Smp. 192-193°;
2-(8-Brom-4,5-dioxo-2,3,5,6-tetrahydro-4H-1-thia-3a,6-diaza-phenalen-3-yl)-N-(3-oxo-3,4-dihydro-2H-benzo[1,4]oxazin-7-ylmethyl)-acetamid, Smp. 300-301°;
2-[2-(8-Brom-4,5-dioxo-2,3,5,6-tetrahydro-4H-1-thia-3a,6-diaza-phenalen-3-yl)acetylamino)-5-(tertiärbutyloxycarbonylaminomethyl) benzoesäuremethylester, Smp. 94-95°;
2-[2-(8-Brom-4,5-dioxo-2,3,5,6-tetrahydro-4H-1-thia-3a,6-diaza-phenalen-3-yl)acetylomino)-5-(tertiärbutyloxycarbonylaminomethyl) benzoesäure, Smp. > 300°;
4-[2-(8-Brom-4,5-dioxo-2,3,5,6-tetrahydro-4H-1-thia-3a,6-diaza-phenalen-3-yl)acetylomino)-benzoesäure, Smp. 298-300°;
2-(8-Brom-4,5-dioxo-2,3,5,6-tetrahydro-4H-1-thia-3a,6-diaza-phenalen-3-yl)-N-(3,5-bistrifluormethylphenyl)-acetamid, Smp. > 250°;
2-(8-Brom-4,5-dioxo-2,3,5,6-tetrahydro-4H-1-thia-3a,6-diaza-phenalen-3-yl)-N-(2-chlor-5-trifluormethylphenyl)-acetamid, Smp. 192-198°;
2-(8-Brom-4,5-dioxo-2,3,5,6-tetrahydro-4H-1-thia-3a,6-diaza-phenalen-3-yl)-N-(2-methoxyphenyl)-acetamid, Smp. > 250°;
2-(8-Brom-4,5-dioxo-2,3,5,6-tetrahydro-4H-1-thia-3a,6-diaza-phenalen-3-yl)-N-(2-trifluormethoxyphenyl)-acetamid, Smp. 142-162°;
2-(8-Brom-4,5-dioxo-2,3,5,6-tetrahydro-4H-1-thia-3a,6-diaza-phenalen-3-yl)-N-(4-methoxyphenyl)-acetamid, Smp. > 250°;
2-(8-Brom-4,5-dioxo-2,3,5,6-tetrahydro-4H-1-thia-3a,6-diaza-phenalen-3-yl)-N-(2,5-dimethoxyphenyl)-acetamid, Smp. > 250°;
2-(8-Brom-4,5-dioxo-2,3,5,6-tetrahydro-4H-1-thia-3a,6-diaza-phenalen-3-yl)-N-(2-fluor-5-trifluormethyl-phenyl)-acetamid, Smp. 163-170°;
2-(8-Brom-4,5-dioxo-2,3,5,6-tetrahydro-4H-1-thia-3a,6-diaza-phenalen-3-yl)-N-(4-phenoxyphenyl)-acetamid, Smp. 137-145°;
2-(8-Brom-4,5-dioxo-2,3,5,6-tetrahydro-4H-1-thia-3a,6-diaza-phenalen-3-yl)-N-(4-(4-chlorphenoxy)phenyl]-acetamid, Smp. 104-120°;
2-(8-Brom-4,5-dioxo-2,3,5,6-tetrahydro-4H-1-thia-3a,6-diaza-phenalen-3-yl)-N-(3,4,5-trimethoxyphenyl)-acetamid, Smp. 240-246°;
2-(8-Brom-4,5-dioxo-2,3,5,6-tetrahydro-4H-1-thia-3a,6-diaza-phenalen-3-yl)-N-(4-fluor-2-trifluormethyl-phenyl)-acetamid, Smp. 198-205°;
2-(8-Brom-4,5-dioxo-2,3,5,6-tetrahydro-4H-1-thia-3a,6-diaza-phenalen-3-yl)-N-(3-phenoxyphenyl)-acetamid, Smp. 120-128°;
2-(8-Brom-4,5-dioxo-2,3,5,6-tetrahydro-4H-1-thia-3a,6-diaza-phenalen-3-yl)-N-(1-cyano-1-methylethyl)-acetamid, Smp..> 250°;
2-(8-Brom-4,5-dioxo-2,3,5,6-tetrahydro-4H-1-thia-3a,6-diaza-phenalen-3-yl)-N-(2-methoxy-5-methyl-phenyl)-acetamid, Smp..> 250°;
2-(8-Brom-4,5-dioxo-2,3,5,6-tetrahydro-4H-1-thia-3a,6-diaza-phenalen-3-yl)-N-(3-methoxy-phenyl)-acetamid, Smp. 98-110°;
2-(8-Brom-4,5-dioxo-2,3,5,6-tetrahydro-4H-1-thia-3a,6-diaza-phenalen-3-yl)-N-(3,5-dimethoxy-phenyl)-acetamid, Smp. 130-138°;
8-Methyl-4,5-dioxo-2,3,5,6-tetrahydro-4H-1-thia-3a,6-diaza-phenalen-3-ylessigsäure, Smp. > 250°;
2-(8-Methyl-4,5-dioxo-2,3,5,6-tetrahydro-4H-1-thia-3a,6-diaza-phenalen-3-yl)-N-phenyl-acetamid, Smp. > 250°;
2-(8-Methyl-4,5-dioxo-2,3,5,6-tetrahydro-4H-1-thia-3a,6-diaza-phenalen-3-yl)-N-(tertiärbutyloxy)-acetamid, Smp. > 250°.;

### Beispiel 53:

Tabletten, enthaltend je 50 mg Wirkstoff, beispielsweise 8-Brom-4,5-dioxo-2,3,5,6-tetrahydro-4H-1-thia-3a,6-diaza-phenalen-3-yl-essigsäure oder ein Sallz davon können wie folgt hergestellt werden:

### Zusammensetzung (10000 Tabletten)

| | |
|---|---|
| Wirkstoff | 500.0 g |
| Laktose | 500.0 g |
| Kartoffelstärke | 352.0 g |
| Gelatine | 8.0 g |
| Talk | 60.0 g |
| Magnesiumstearat | 10.0 g |
| Siliciumdioxid (hochdispers) | 20.0 g |
| Ethanol | q.s. |

Der Wirkstoff wird mit der Laktose und 292 g Kartoffelstärke vermischt, die Mischung mit einer ethanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

### Beispiel 54:

Eine sterilfiltrierte wäßrige Gelatine-Lösung mit 20 % Cyclodextrinen als Lösungsvermittler, enthaltend je 3 mg 8-Brom-4,5-dioxo-2,3,5,6-tetrahydro-4H-1-thia-3a,6-diaza-phenalen-3-yl-essigsäure oder ein Salz, z.B. das Natriumsalz, davon als Wirkstoff wird unter Erwärmen mit einer sterilen Gelatinelösung, welche als Konservierungsmittel Phenol enthält, unter aseptischen Bedingungen so vermischt, dass 1.0 ml Lösung die folgende Zusammensetzung hat:

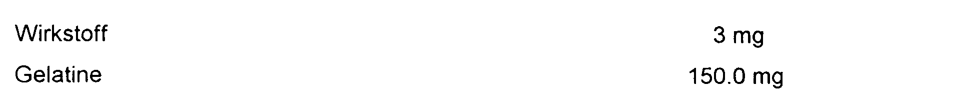

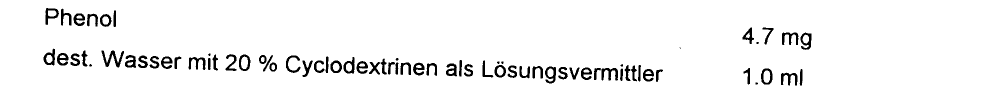

### Beispiel 55:

Zur Herstellung einer sterilen Trockensubstanz zur Injektion, enthaltend je 5 mg 8-Brom-4,5-dioxo-2,3,5,6-tetrahydro-4H-1-thia-3a,6-diaza-phenalen-3-yl-essigsäure oder ein Salz, z.B. das Natriumsalz, davon löst man 5 mg einer der in den vorausgehenden Beispielen genannten Verbindungen der Formel I als Wirkstoff in 1 ml einer wäßrigen Lösung mit 20 mg Mannit und 20 % Cyclodextrinen als Lösungsvermittler. Die Lösung wird sterilfiltriert und unter aseptischen Bedingungen in eine 2 ml-Ampulle gefüllt, tiefgekühlt und lyophilisiert. Vor dem Gebrauch wird das Lyophilisat in 1 ml destilliertem Wasser oder 1 ml physiologischer Kochsalzlösung gelöst. Die Lösung wird intramuskulär oder intravenös angewendet. Diese Formulierung kann auch in Doppelkammerspritzampullen abgefüllt werden.

### Beispiel 56:

Für die Herstellung von 10 000 Lacktabletten, enthaltend je 100 mg 8-Brom-4,5-dioxo-2,3,5,6-tetrahydro-4H-1-thia-3a,6-diaza-phenalen-3-yl-essigsäure oder ein Salz, z.B. das Natriumsalz, davon können wie folgt hergestellt werden:

| | |
|---|---|
| Wirkstoff | 1000 g |
| Maisstärke | 680 g |
| Kolloidale Kieselsäure | 200 g |
| Magnesiumstearat | 20 g |
| Stearinsäure | 50 g |
| Natriumcarboxymethylstärke | 250 g |
| Wasser | q. s. |

Ein Gemisch von einer der in den vorausgehenden Beispielen genannten Verbindungen der Formel I als Wirkstoff, 50 g Maisstärke und der kolloidalen Kieselsäure wird mit Stärkekleister aus 250 g Maisstärke und 2.2 kg entmineralisiertem Wasser zu einer feuchten Masse verarbeitet. Diese wird durch ein Sieb von 3 mm Maschenweite getrieben und bei 45° während 30 Minuten. im Wirbelschichttrockner getrocknet. Das getrocknete Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

### Beispiel 57:

In analoger Weise wie in den Beispielen 53 bis 56 beschrieben kann man ferner pharmazeutische Präparate enthaltend eine andere Verbindung gemäss einem der Beispielen 1 bis 52 herstellen.

## Patentansprüche

**1.** Neue oxa- und thiaaliphatisch überbrückte Chinoxalin-2,3-dione der allgemeinen Formel I 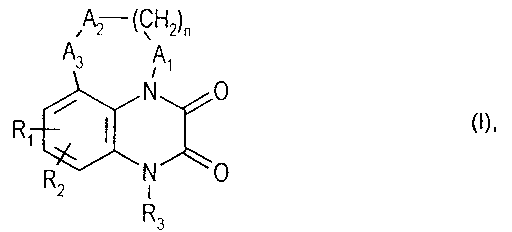 worin
A₁ Niederalkyliden oder eine Gruppe der Formel >CH-A₄-R₄ (la) darstellt,
A₂ Niederalkyliden oder eine Gruppe der Formeln >CH-A₄-R₄ (la), >C=O (Ib) oder >CH(OH)-A₅-R₄ (Ic) ist,
A₃ Oxy, gegebenenfalls oxidiertes Thio oder eine Gruppe >C(=O) (Ib) bedeutet,
A₄ für Niederalkylen steht,
A₅ Niederalkylen oder eine direkte Bindung darstellt,
n für 0 oder 1 steht,
R₁ und R₂ unabhängig voneinander Wasserstoff, gegebenenfalls durch Niederalkyl und/oder Niederalkanoyl substituiertes Amino, Nitro, Niederalkanoyl, gegebenenfalls verethertes oder verestertes Hydroxy, gegebenenfalls verestertes oder amidiertes Carboxy, Cyano, gegebenenfalls halogeniertes Niederalkyl oder Halogen bedeuten,
R₃ Wasserstoff oder Hydroxy bedeutet und
R₄ Wasserstoff, Cyano, gegebenenfalls verestertes oder amidiertes Carboxy, gegebenenfalls verestertes Phosphono oder 5-Tetrazolyl darstellt,
und ihre Salze.

**2.** Verbindungen gemäß Anspruch 1 der Formel I, worin
A₁ Niederalkyliden oder eine Gruppe der Formel >CH-A₄-R₄ (la) darstellt,
A₂ Niederalkyliden oder eine Gruppe der Formel >CH-A₄-R₄ (la) oder >C=O (Ib) ist,
A₃ Oxy oder gegebenenfalls oxidiertes Thio bedeutet,
A₄ für Niederalkylen steht,
n für 0 oder 1 steht,
R₁ und R₂ unabhängig voneinander Wasserstoff, gegebenenfalls durch Niederalkyl und/oder Niederalkanoyl substituiertes Amino, gegebenenfalls mit einem Niederalkanol verethertes Hydroxy, gegebenenfalls halogeniertes Niederalkyl oder Halogen bedeuten,
R₃ Wasserstoff, Hydroxy oder gegebenenfalls durch Niederalkanoyl substituiertes Amino bedeutet und
R₄ Wasserstoff, Cyano, Tetrazolyl oder gegebenenfalls verestertes oder amidiertes Carboxy darstellt, wie solche,worin
A₁ eine Gruppe der Formel >CH-A₄-R₄ (la) darstellt,
A₂ Niederalkyliden bedeutet,
A₃ Oxy, gegebenenfalls oxidiertes Thio oder Carbonyl ist,
A₄ für Niederalkylen steht,
R₁ und R₂ unabhängig voneinander Wasserstoff, gegebenenfalls durch Niederalkyl und/oder Niederalkanoyl substituiertes Amino, gegebenenfalls mit einem Niederalkanol verethertes Hydroxy, gegebenenfalls halogeniertes Niederalkyl oder Halogen bedeuten,
R₃ Wasserstoff oder Hydroxy bedeutet und
R₄ Wasserstoff, Cyano oder gegebenenfalls verestertes oder amidiertes Carboxy darstellt, und ihre Salze.

**3.** Verbindungen gemäß Anspruch 1 der Formel I, worin
A₁ Methylen oder eine Gruppe der Formel >CH-A₄-R₄ (la) darstellt,
A₂ Niederalkyliden und A₃ Oxy, Thio, Sulfinyl, Sulfonyl oder Carbonyl ist oder
A₂ Carbonyl und A₃ Oxy, Thio, Sulfinyl oder Sulfonyl bedeutet,
A₄ für Niederalkylen steht,
n für 0 oder 1 steht,
R₁ und R₂ unabhängig voneinander Wasserstoff Amino, Niederalkylamino, Niederalkanoylamino, Diniederalkylamino, N-Niederalkanoyl-N-niederalkyl-amino, Hydroxy, Niederalkoxy, Niederalkenyloxy, Niederalkinyloxy, Niederalkyl, Polyhalogenniederalkyl oder Halogen bedeuten,
R₃ Wasserstoff bedeutet und
R₄ Wasserstoff, Carboxy, Niederalkoxycarbonyl, unsubstituiertes oder durch Niederalkyl, Niederalkoxy, Hydroxy, Halogen und/oder Trifluormethyl substituiertes Phenylniederalkoxycarbonyl, Carbamoyl, Cyano oder Niederalkylcarbamoyl, unsubstituiertes oder durch Niederalkyl, Niederalkoxy, Hydroxy, Halogen und/oder Trifluormethyl substituiertes Phenylcarbamoyl oder Diniederalkylcarbamoyl darstellt,
und ihre Salze.

**4.** Verbindungen gemäß Anspruch 1 der Formel I, worin
A₁ Niederalkyliden oder eine Gruppe der Formel >CH-A₄-R₄ (Ia) darstellt,
A₂ Niederalkyliden oder eine Gruppe der Formeln >CH-A₄-R₄ (Ia), >C=O (Ib) oder >CH(OH)-A₅-R₄ (Ic) ist,
A₃ Oxy, Thio, Sulfinyl oder Sulfonyl oder eine Gruppe >C(=O) (Ib) bedeutet,
A₄ für Niederalkylen steht,
A₅ Niederalkylen oder eine direkte Bindung darstellt,
n für 0 oder 1 steht,
R₁ und R₂ unabhängig voneinander Wasserstoff, Amino, Niederalkylamino, Niederalkanoylamino, Diniederalkylamino, N-Niederalkanoyl-N-niederalkyl-amino, Nitro, Niederalkanoyl, Hydroxy, Niederalkanoyloxy, Niederalkoxy, Niederalkenyloxy, Niederalkinyloxy, Carboxy, Niederalkoxycarbonyl, Carbamoyl, Cyano, Niederalkyl, Polyhalogenniederalkyl oder Halogen bedeuten,
R₃ Wasserstoff oder Hydroxy bedeutet und
R₄ Wasserstoff, Cyano, Carboxy, Niederalkoxycarbonyl, Carboxyniederalkoxycarbonyl, Niederalkoxycarbonylniederalkoxycarbonyl, Niederalkanoyloxyniederalkoxycarbonyl, unsubstituiertes oder durch Niederalkyl, Niederalkoxy, Hydroxy, Halogen und/oder Trifluormethyl substituiertes Phenyloxycarbonyl, Benzoyloxyniederalkoxycarbonyl oder Phenylniederalkoxycarbonyl, Carbamoyl, Niederalkylcarbamoyl, Niederalkenylcarbamoyl, Diniederalkylcarbamoyl, Diniederalkylaminoniederalkylcarbamoyl, Aminoniederalkylaminoniederalkylcarbamoyl, 2-Oxoimidazolidin-1-ylniederalkylcarbamoyl, Aminoniederalkylaminoniederalkylencarbamoyl, 2-Oxoimidazolidin-1-ylniederalkylencarbamoyl, Oxaniederalkylenaminoniederalkylcarbamoyl, 3- bis 7-gliedriges Cycloalkylcarbamoyl, 3- bis 7-gliedriges Carboxycycloalkylcarbamoyl, 3- bis 7-gliedriges Niederalkoxycarbonylcycloalkylcarbamoyl, 3- bis 7-gliedriges Cycloalkylniederalkylcarbamoyl, 3-Aza-2-oxo-cycloheptylcarbamoyl., Azaridinocarbonyl, 2-Methylazaridinocarbonyl, 2,3-Dihydoindolin-1-ylcarbamoyl, Pyrrolidinocarbonyl, Piperidinocarbonyl, Morpholinocarbonyl, Thiomorpholinocarbonyl, Piperazinocarbonyl, N'-Niederalkylpiperazinocarbonyl, unsubstituiertes oder durch Niederalkyl, Niederalkoxy, Hydroxy, Halogen und/oder Trifluormethyl substituiertes Phenylniederalkylcarbamoyl, unsubstituiertes oder durch Niederalkyl, Niederalkoxy, Hydroxy, Halogen, Nitro, Carboxy, Niederalkoxycarbonyl, Phenyl, Phenyloxy und/oder Trifluormethyl substituiertes N-Phenylcarbamoyl oder N-Niederalkyl-N-phenylcarbamoyl, Naphthylcarbamoyl, 5,6-7,8-Tetrahydronaphthylcarbamoyl, N-lndanylcarbamoyl, Furyl-2-carbamoyl, Thien-2-ylcarbamoyl, Thiazol-2-ylcarbamoyl, N-Benzthiazol-2-ylcarbamoyl, Fur-2-ylmethylcarbamoyl, Thien-2-ylmethylcarbamoyl, Thiazol-2-ylmethylcarbamoyl, N-(3-Oxo-2,3-dihydro-4H-1,4-benzoxazin-7-yl)methylcarbamoyl, Mono- oder Dihydroxyniederalkylcarbamoyl, Mono- oder Diniederalkoxyniederalkylcarbamoyl, Polyhalogenniederalkylcarbamoyl, Carboxyniederalkylcarbamoyl, Niederalkoxycarbonylniederalkylcarbamoyl, Dicarboxyniederalkylcarbamoyl, Diniederalkoxycarbonylniederalkylcarbamoyl, Cyanoniederalkylcarbamoyl, Carboxyniederalkenylcarbamoyl oder Niederalkoxycarbonylniederalkenylcarbamoyl, N-Hydroxycarbamoyl, N-Niederalkoxycarbamoyl, N-Niederalkoxy-N-niederalkyl-carbamoyl, N-Niederalkenyloxycarbamoyl oder jeweils unsubstituiertes oder durch Niederalkyl, Niederalkoxy, Hydroxy, Halogen und/oder Trifluormethyl substituiertes N-Phenyloxycarbamoyl, N-Phenylniederalkoxycarbamoyl oder N-Phenylniederalkenyloxycarbamoyl, Phosphono, Niederalkylphosphono, Diniederalkylphosphono oder Triniederalkylphosphono oder 5-Tetrazolyl darstellt,
und ihre Salze.

**5.** Verbindungen gemäß Anspruch 1 der Formel I, worin
A₁ geradkettiges oder verzweigtes C₁-C₇-Alkylen oder eine Gruppe der Formel >CH-A₄-R₄ (la) darstellt,
A₂ geradkettiges oder verzweigtes C₁-C₇-Alkylen oder eine Gruppe der Formel >CH-A₄-R₄ (Ia) oder >CH(OH)-A₅-R₄ (Ic) ist,
A₃ Oxy, Thio, Sulfinyl oder Sulfonyl bedeutet,
A₄ für geradkettiges oder verzweigtes C₁-C₇-Alkylen steht,
A₅ C₁-C₄-Alkylen oder eine direkte Bindung darstellt,
n für 0 oder 1 steht,
R₁ und R₂ unabhängig voneinander Wasserstoff, Amino, N-C₁-C₄-Alkylamino,N-C₁-C₇-Alkanoylamino, N,N-Di-C₁-C₄-Alkylamino, N-C₁-C₇-Alkanoyl-N-C₁-C₄-alkylamino, Nitro, N-C₁-C₄-Alkanoyl, Pivaloyl, Hydroxy, N-C₁-C₄-Alkanoyloxy, Pivaloyloxy, C₁-C₄-Alkoxy, C₃-C₄-Alkenyloxy, C₃-C₄-Alkinyloxy, Carboxy, C₁-C₄-Alkoxycarbonyl, Carbamoyl, Cyano, C₁-C₄-Alkyl, Halogen oder Trifluormethyl oder Halogen bedeuten,
R₃ Wasserstoff oder Hydroxy bedeutet und
R₄ Wasserstoff, Cyano, Carboxy, C₁-C₇-Alkoxycarbonyl, Carboxy-C₁-C₄-alkoxycarbonyl, C₁-C₄-Alkoxycarbonyl-C₁-C₄-alkoxycarbonyl, N-C₁-C₄-Alkanoyloxy-C₁-C₄-alkoxycarbonyl, Pivaloyloxymethoxycarbonyl, unsubstituiertes oder durch C₁-C₄-Alkyl, C₁-C₄-Alkoxy, Hydroxy, Halogen und/oder Trifluormethyl substituiertes Phenyloxycarbonyl, Benzoyloxy-C₁-C₄-alkoxycarbonyl oder Phenyl-C₁-C₄-alkoxycarbonyl, Carbamoyl, N-C₁-C₄-Alkylcarbamoyl, C₂-C₄-Alkenylcarbamoyl, N,N-Di-C₁-C₄-Alkylcarbamoyl, C₃-C₇-Cycloalkylcarbamoyl, Carboxy-C₃-C₇-cycloalkylcarbamoyl, C₁-C₄-Alkoxycarbonyl-C₃-C₇-cycloalkylcarbamoyl, 3- bis 7-gliedriges N-(C₃-C₆-Cycloalkyl)-C₁-C₄-alkylcarbamoyl, 3-Aza-2-oxo-cycloheptylcarbamoyl., Azaridinocarbonyl, 2-Methylazaridinocarbonyl, 2,3-Dihydoindolin-1-ylcarbamoyl, Pyrrolidinocarbonyl, Piperidinocarbonyl, Morpholinocarbonyl, Thiomorpholinocarbonyl, Piperazinocarbonyl, N'-C₁-C₄-Alkylpiperazinocarbonyl, unsubstituiertes oder durch C₁-C₄-Alkyl, C₁-C₄-Alkoxy, Hydroxy, Halogen und/oder Trifluormethyl substituiertes Phenyl-C₁-C₄-alkylcarbamoyl, unsubstituiertes oder durch C₁-C₄-Alkyl, C₁-C₄-Alkoxy, Hydroxy, Halogen und/oder Trifluormethyl, Amino, C₁-C₄-Alkoxycarbonylamino, Nitro, Carboxy, C₁-C₄-Alkoxycarbonyl, wie Methoxy- oder Ethoxycarbonyl, Phenyl, Phenyloxy und/oder Trifluormethyl substituiertes N-Phenylcarbamoyl oder N-C₁-C₄-Alkyl-N-phenyl-carbamoyl, Naphthylcarbamoyl, 5,6,7,8-Tetrahydronaphthylcarbamoyl, Indanylcarbamoyl, Furyl-2-carbamoyl, Thien-2-ylcarbamoyl, Thiazol-2-ylcarbamoyl, N-Benzthiazol-2-ylcarbamoyl, Fur-2-ylmethylcarbamoyl, Thien-2-ylmethylcarbamoyl, Thiazol-2-ylmethylcarbamoyl, N-(3-Oxo-2,3-dihydro-4H-1,4-benzoxazin-7-yl)methylcarbamoyl, N-(Hydroxy-C₂-C₄-alkyl)carbamoyl, C₁-C₄-Alkoxy-C₁-C₄-alkylcarbamoyl, Di-C₁-C₄-alkoxy-C₁-C₄-alkylcarbamoyl, Polyhalogen-C₂-C₄-alkylcarbamoyl, Carboxy-C₁-C₄-alkylcarbamoyl, C₁-C₄-Alkoxycarbonyl-C₁-C₄-alkylcarbamoyl, Dicarboxy-C₁-C₄-alkylcarbamoyl Di-C₁-C₄-alkoxycarbonyl-C₁-C₄-alkylcarbamoyl, Cyano-C₁-C₄-alkylcarbamoyl, Carboxy-C₂-C₄-alkenylcarbamoyl, C₁-C₄-Alkoxycarbonyl-C₂-C₄-alkenylcarbamoyl, N-Hydroxycarbamoyl, N-C₁-C₄-Alkoxycarbamoyl, N- N-C₂-C₄-alkenyloxycarbamoyl, jeweils unsubstituiertes oder durch C₁-C₄-Alkyl, C₁-C₄Alkoxy, Hydroxy, Halogen und/oder Trifluormethyl substituiertes N-Phenyloxycarbamoyl, N- Phenyl-C₁-C₄-alkoxycarbamoyl oder N-Phenyl-C₂-C₄-alkenyloxycarbamoyl, Phosphono, C₁-C₄-Alkylphosphono,Di-C₁-C₄-Alkylphosphono, Tri-C₁-C₄-Alkylphosphono oder 5-Tetrazolyl darstellt,
und ihre Salze.

**6.** Verbindungen gemäß Anspruch 3 der Formel I, worin
A₁ Methylen oder eine Gruppe der Formel >CH-A₄-R₄ (la) darstellt,
A₂ geradkettiges oder verzweigtes C₁-C₇-Alkyliden ist,
A₃ Oxy, Thio, Sulfinyl oder Sulfonyl bedeutet,
A₄ für geradkettiges oder verzweigtes C₁-C₇-Alkylen steht,
n für 0 oder 1 steht,
R₁ und R₂ unabhängig voneinander Wasserstoff Amino, N-C₁-C₇-Alkylamino, N-C₁-C₇-Alkanoylamino, Pivaloylamino, N,N-Di-C₁-C₇-Alkylamino, Hydroxy, C₁-C₇-Alkoxy, C₃-C₄-Alkenyloxy, C₃-C₄-Alkinyloxy, C₁-C₇-Alkyl, Trifluormethyl oder Halogen bedeuten,
R₃ Wasserstoff oder Hydroxy bedeutet und
R₄ Wasserstoff, Carboxy, C₁-C₇-Alkoxycarbonyl, unsubstituiertes oder durch C₁-C₄-Alkyl, C₁-C₄-Alkoxy, Hydroxy, Halogen und/oder Trifluormethyl substituiertes Phenyl-C₁-C₄-alkoxycarbonyl, Carbamoyl, Tetrazolyl, Cyano, C₁-C₄-Alkylcarbamoyl, unsubstituiertes oder durch C₁-C₄-Alkyl, C₁-C₄-Alkoxy, Hydroxy, Halogen und/oder Trifluormethyl substituiertes Phenylcarbamoyl oder N,N-Di-C₁-C₇-Alkylcarbamoyl darstellt,
und ihre Salze.

**7.** Verbindungen gemäß Anspruch 1 der Formel I, worin
einer der Rest A₁ und A₂ eine Gruppe der Formel >CH-A₄-R₄ (la) und der andere geradkettiges oder verzweigtes C₁-C₄-Alkylen darstellt,
A₃ Oxy, Thio, Sulfinyl oder Sulfonyl bedeutet,
A₄ für geradkettiges oder verzweigtes C₁-C₄-Alkylen steht,
n für 0 steht,
R₁ und R₂ unabhängig voneinander Wasserstoff, Nitro, N-C₁-C₄-Alkanoyl, Pivaloyl, Hydroxy, C₁-C₄-Alkoxy, Carboxy, C₁-C₄-Alkoxycarbonyl, Carbamoyl, Cyano, C₁-C₄-Alkyl, Halogen, Trifluormethyl oder Halogen bedeuten,
R₃ Wasserstoff bedeutet und
R₄ Wasserstoff, Carboxy, C₁-C₇-Alkoxycarbonyl, C₁-C₄-Alkoxycarbonyl-C₁-C₄-alkoxycarbonyl, N-C₁-C₄-Alkanoyloxy-C₁-C₄-alkoxycarbonyl, jeweils unsubstituiertes oder durch C₁-C₄-Alkyl, C₁-C₄-Alkoxy, Hydroxy, Halogen und/oder Trifluormethyl substituiertes Phenyloxycarbonyl, Benzoyloxy-C₁-C₄-alkoxycarbonyl oder Phenyl-C₁-C₄-alkoxycarbonyl, Carbamoyl, N-C₁-C₄-Alkylcarbamoyl, C₂-C₄-Alkenylcarbamoyl, N,N-Di-C₁-C₄-Alkylcarbamoyl, C₃-C₇-Cycloalkylcarbamoyl, C₁-C₄-Alkoxycarbonyl-C₃-C₇-cycloalkylcarbamoyl, 3- bis 7-gliedriges N-(C₃-C₆-Cycloalkyl)-C₁-C₄-alkylcarbamoyl, 3-Aza-2-oxo-cycloheptylcarbamoyl., Azaridinocarbonyl, 2-Methylazaridinocarbonyl, 2,3-Dihydoindolin-1-ylcarbamoyl, Pyrrolidinocarbonyl, Piperidinocarbonyl, unsubstituiertes oder C₁-C₄-Alkyl, C₁-C₄-Alkoxy, Hydroxy, Halogen und/oder Trifluormethyl substituiertes Phenyl-C₁-C₄-alkylcarbamoyl, unsubstituiertes oder durch C₁-C₄-Alkyl, C₁-C₄-Alkoxy, Hydroxy, Halogen und/oder Trifluormethyl, Nitro, Carboxy, C₁-C₄-Alkoxycarbonyl, wie Methoxy- oder Ethoxycarbonyl, Phenyl, Phenyloxy und/oder Trifluormethyl substituiertes N-Phenylcarbamoyl oder N-C₁-C₄-Alkyl-N-phenylcarbamoyl, Naphthylcarbamoyl, 5,6-7,8-Tetrahydronaphthylcarbamoyl, Furyl-2-carbamoyl, Thien-2-ylcarbamoyl, Thiazol-2-ylcarbamoyl, Fur-2-ylmethylcarbamoyl, Thien-2-ylmethylcarbamoyl oder Thiazol-2-ylmethylcarbamoyl, C₁-C₄-Alkoxy-C₁-C₄-alkylcarbamoyl, Di-C₁-C₄-alkoxy-C₁-C₄-alkylcarbamoyl, Carboxy-C₁-C₄-alkylcarbamoyl, C₁-C₄-Alkoxycarbonyl-C₁-C₄-alkylcarbamoyl, Dicarboxy-C₁-C₄-alkylcarbamoyl, Di-C₁-C₄-alkoxycarbonyl-C₁-C₄-alkylcarbamoyl-C₁-C₄-Alkoxycarbonyl-C₂-C₄-alkenylcarbamoyl, N-Hydroxycarbamoyl, N-C₁-C₄-Alkoxycarbamoyl, N-C₂-C₄-Alkenyloxycarbamoyl, unsubstituiertes oder durch C₁-C₄-Alkyl, C₁-C₄Alkoxy, Hydroxy, Halogen und/oder Trifluormethyl substituiertes N- Phenyl-C₁-C₄-alkoxycarbamoyl, N-Phenyl-C₂-C₄-alkenyloxycarbamoyl, Phosphono oder 5-Tetrazolyl darstellt,
und ihre Salze.

**8.** Verbindungen gemäß Anspruch 2 der Formel I, worin
A₁ Methylen oder eine Gruppe der Formel >CH-A₄-R₄ (la) darstellt,
A₂ eine Gruppe der Formel >CH-A₄-R₄ (la) oder, sofern A₁ eine Gruppe der Formel >CH-A₄-R₄ (Ia) darstellt, geradkettiges oder verzweigtes C₁-C₄-Alkylen bedeutet,
A₃ Oxy, Thio, Sulfinyl oder Sulfonyl ist,
A₄ für geradkettiges oder verzweigtes C₁-C₄-Alkylen steht,
n für 0 steht,
R₁ und R₂ unabhängig voneinander Wasserstoff, Hydroxy, C₁-C₄-Alkoxy, C₁-C₄-Alkyl, Trifluormethyl oder Halogen bedeuten,
R₃ Wasserstoff oder Hydroxy bedeutet und
R₄ Wasserstoff, Carboxy, C₁-C₄-Alkoxycarbonyl, unsubstituiertes oder durch C₁-C₄-Alkyl, C₁-C₄-Alkoxy, Hydroxy, Halogen und/oder Trifluormethyl substituiertes Phenyl-C₁-C₄-alkoxycarbonyl, Carbamoyl, C₁-C₄-Alkylcarbamoyl, unsubstituiertes oder durch C₁-C₄-Alkyl, C₁-C₄-Alkoxy, Hydroxy, Halogen und/oder Trifluormethyl substituiertes Phenylcarbamoyl N,N-Di-C₁-C₄-Alkylcarbamoyl oder 5-Tetrazolyl darstellt,
und ihre Salze.

**9.** Verbindungen gemäß Anspruch 1 der Formel I, worin
einer der Rest A₁ und A₂ eine Gruppe der Formel >CH-A₄-R₄ (la) und der andere
Methylen darstellt,
A₃ Thio bedeutet,
A₄ für Methylen steht,
n für 0 steht,
R₁ und R₂ unabhängig voneinander Wasserstoff, C₁-C₄-Alkyl, Trifluormethyl oder Halogen bedeuten,
R₃ Wasserstoff bedeutet und
R₄ Wasserstoff, Carboxy, C₁-C₄-Alkoxycarbonyl, unsubstituiertes oder durch C₁-C₄-Alkyl, C₁-C₄-Alkoxy, Hydroxy, Halogen und/oder Trifluormethyl substituiertes Benzoyloxy-C₁-C₄-alkoxycarbonyl, unsubstituiertes oder durch C₁-C₄-Alkyl, C₁-C₄-Alkoxy, Hydroxy, Halogen und/oder Trifluormethyl, Nitro, Carboxy, C₁-C₄-Alkoxycarbonyl und/oder Trifluormethyl substituiertes N-Phenylcarbamoyl, Naphthylcarbamoyl, 5,6-7,8-Tetrahydronaphthylcarbamoyl, Furyl-2-carbamoyl, Thien-2-ylcarbamoyl, Thiazol-2-ylcarbamoyl, Bicyclo[2,2,1]heptyl-, Bicyclo[2,2,2]octyl- oder Adamantylcarbamoyl, 3-Aza-2-oxo-cycloheptylcarbamoyl, N-C₁-C₄-Alkoxycarbamoyl, N-C₂-C₄-alkenyloxycarbamoyl, unsubstituiertes oder durch C₁-C₄-Alkyl, C₁-C₄Alkoxy, Hydroxy, Halogen und/oder Trifluormethyl substituiertes N- Phenyl-C₁-C₄-alkoxycarbamoyl, N-Phenyl-C₂-C₄-alkenyloxycarbamoyl oder 5-Tetrazolyl darstellt, und ihre Salze.

**10.** Verbindungen gemäß Anspruch 2 der Formel I, worin
einer der Reste A₁ und A₂ Methylen oder eine Gruppe der Formel >CH-A₄-R₄ (Ia) und der andere Methylen darstellt,
A₃ Thio bedeutet,
n für 0 steht,
R₁ und R₂ unabhängig voneinander Wasserstoff, C₁-C₄-Alkyl, Trifluormethyl oder Halogen bedeuten,
R₃ Wasserstoff darstellt und
R₄ Carboxy, C₁-C₄-Alkoxycarbonyl, 5-Tetrazolyl oder unsubstituiertes oder durch C₁-C₄-Alkyl, C₁-C₄-Alkoxy, Hydroxy, Halogen und/oder Trifluormethyl substituiertes Phenylcarbamoyl darstellt,
und ihre Salze.

**11.** Verbindungen gemäß Anspruch 1 der Formel I' 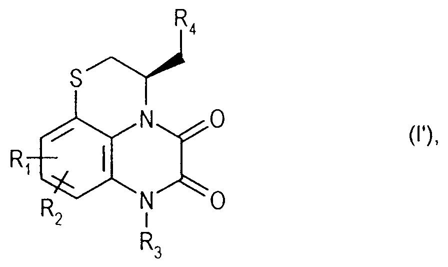 worin
R₁ und R₂ unabhängig voneinander Wasserstoff, C₁-C₄-Alkyl, Trifluormethyl oder Halogen bedeuten,
R₃ Wasserstoff bedeutet und
R₄ Wasserstoff, Carboxy, C₁-C₄-Alkoxycarbonyl, unsubstituiertes oder durch C₁-C₄-Alkyl, C₁-C₄-Alkoxy, Hydroxy, Halogen und/oder Trifluormethyl substituiertes Benzoyloxy-C₁-C₄-alkoxycarbonyl, unsubstituiertes oder durch C₁-C₄-Alkyl, C₁-C₄-Alkoxy, Hydroxy, Halogen und/oder Trifluormethyl, Nitro, Carboxy, C₁-C₄-Alkoxycarbonyl, wie Methoxy- oder Ethoxycarbonyl, und/oder Trifluormethyl substituiertes N-Phenylcarbamoyl, Naphthylcarbamoyl, 5,6-7,8-Tetrahydronaphthylcarbamoyl, Furyl-2-carbamoyl, Thien-2-ylcarbamoyl, Thiazol-2-ylcarbamoyl, Bicyclo[2,2,1]heptyl-, Bicyclo[2,2,2]octyl- oder Adamantylcarbamoyl, 3-Aza-2-oxo-cycloheptylcarbamoyl, N-C₁-C₄-Alkoxycarbamoyl, N-C₂-C₄-alkenyloxycarbamoyl, unsubstituiertes oder durch C₁-C₄-Alkyl, C₁-C₆Alkoxy, Hydroxy, Halogen und/oder Trifluormethyl substituiertes N- Phenyl-C₁-C₄-alkoxycarbamoyl, N-Phenyl-C₂-C₄-alkenyloxycarbamoyl oder 5-Tetrazolyl darstellt,
und ihre Salze.

**12..** 3,3-Dihydro-6H-1-oxa-3a,6-diaza-phenalen-4,5-dion;
4,5-Dioxo-2,3,5,6-tetrahydro-4H-1-oxa-3a,6-diaza-phenalen-3-yl-N-phenyl-acetamid;
4,5-Dioxo-2,3,5,6-tetrahydro-4H-1-oxa-3a,6-diaza-phenalen-3-ylessigsäureethylester;
4,5-Dioxo-2,3,5,6-tetrahydro-4H-1 -oxa-3a,6-diaza-phenalen-3-ylessigsäure;
8-Brom-1,4,5-trioxo-2,3,5,6-tetrahydro-1H,4H-1-thia-3a,6-diaza-phenalen-3-ylessigsäureethylester;
8-Brom-1,4,5-trioxo-2,3,5,6-tetrahydro-1H,4H-1-thia-3a,6-diaza-phenalen-3-ylessigsäure;
8-Brom-1,4,5-trioxo-2,3,5,6-tetrahydro-1H,4H-1-thia-3a,6-diaza-phenalen-3-yl-N-phenylacetamid;
8-Brom-4,5-dioxo-2,3,5,6-tetrahydro-4H-1-oxa-3a,6-diaza-phenalen-3-ylessigsäureethylester ;
8-Brom-4,5-dioxo-2,3,5,6-tetrahydro-4H-1-oxa-3a,6-diaza-phenalen-3-ylessigsäure;
8-Brom-4,5-dioxo-2,3,5,6-tetrahydro-4H-1-oxa-3a,6-diaza-phenalen-3-yl-N-phenyl-acetamid;
8-Chlor-1,4,5-triioxo-2,3,5,6-tetrahydro-1H,4H-1-thia-3a,6-diaza-phenalen-3-ylessigsäureethylester;
8-Chlor-1,4,5-triioxo-2,3,5,6-tetrahydro-1H,4H-1-thia-3a,6-diaza-phenalen-3-ylessigsäure;
8-Chlor-1,4,5-triioxo-2,3,5,6-tetrahydro-1H,4H-1-thia-3a,6-diaza-phenalen-3-yl-N-phenylacetamid;
8-Chlor-4,5-dioxo-2,3,5,6-tetrahydro-4H-1-thia-3a,6-diaza-phenalen-3-ylessigsäureethylester;
8-Chlor-4,5-dioxo-2,3,5,6-tetrahydro-4H-1-thia-3a,6-diaza-phenalen-3-ylessigsäure;
8-Chlor-4,5-dioxo-2,3,5,6-tetrahydro-4H-1-thia-3a,6-diaza-phenalen-3-yl-N-phenyl-acetamid oder 8-Chlor-6H-1-thia-3a,6-diaza-phenalen-3,4,5-trion oder jeweils ein Salz davon.

**13.** 8-Brom-4,5-dioxo-2,3,5,6-tetrahydro-4H-1-thia-3a,6-diaza-phenalen-2-ylacetamid;
8-Brom-4,5-dioxo-2,3,5,6-tetrahydro-4H-1-thia-3a,6-diaza-phenalen-2-ylacetonitril;
8-Brom-2(1H-tetrazol-5-ylmethyl)-2,3-dihydro-6H-1-thia-3a,6-diaza-phenalen-4,5-dion;
8-Brom-4,5-dioxo-2,3,5,6-tetrahydro-*4H*-1-thia-3a,6-diaza-phenalen-2-ylessigsäureethylester;
8-Brom-4,5-dioxo-2,3,5,6-tetrahydro-4H-1-thia-3a,6-diaza-phenalen-2-ylessigsäure oder
8-Brom-4,5-dioxo-2,3,5,6-tetrahydro-4H-1-thia-3a,6-diaza-phenalen-2-ylessigsäure-N-phenylamid oder jeweils ein Salz davon.

**14.** 2-(8-Brom-4,5-dioxo-2,3,5,6-tetrahydro-4H-1-thia-3a,6-diaza-phenalen-3-yl)-acetylamino-essigsäure;
1-[2-(8-Brom-4,5-dioxo-2,3,5,6-tetrahydro-4H-1-thia-3a,6-diaza-phenalen-3-yl)-acetylamino]-cyclopropan-carbonsäure-methylester;
1-[2-(8-Brom-4,5-dioxo-2,3,5,6-tetrahydro-4H-1-thia-3a,6-diaza-phenalen-3-yl)-acetylamino]-cyclopropan-carbonsäure;
2,2-Dimethyl-propionsäure-(8-brom-4,5-dioxo-2,3,5,6-tetrahydro-4H-1-thia-3a,6-diaza-phenalen-3-yl)-acetoxymethylester;
2-(3-(R)-8-Brom-4,5-dioxo-2,3,5,6-tetrahydro-4H-1-thia-3a,6-diaza-phenalen-3-yl)-N-phenyl-acetamid;
2-(3-(S)-8-Brom-4,5-dioxo-2,3,5,6-tetrahydro-4H-1-thia-3a,6-diaza-phenalen-3-yl)-N-phenyl-acetamid;
2-(8-Brom-1,1,4,5-tetraoxo-2,3,5,6-tetrahydro-1H,4H-6-thia-3a,6-diaza-phenalen-3-yl)-N-phenyl-acetamid;
2-(8-Brom-4,5-dioxo-2,3,5'6-tetrahydro-4H-1-thia-3a,6-diaza-phenalen-3-yl)-N-phenyl-acetamid;
2-(8-Brom-4,5-dioxo-2,3,5,6-tetrahydro-4H-1-thia-3a,6-diaza-phenalen-3-yl)-N-methyl-N-phenyl-acetamid;
2-(8-Brom-4,5-dioxo-2,3,5,6-tetrahydro-4H-1-thia-3a,6-diaza-phenalen-3-yl)-N-thiazol-2-yl-acetamid;
2-(8-Brom-4,5-dioxo-2,3,5,6-tetrahydro-4H-1-thia-3a,6-diaza-phenalen-3-yl)-N-cyclopropylmethyl-acetamid;
2-(8-Brom-4,5-dioxo-2,3,5,6-tetrahydro-4H-1-thia-3a,6-diaza-phenalen-3-yl)-N-cyclopropyl-acetamid;
2-(8-Brom-4,5-dioxo-2,3,5,6-tetrahydro-4H-1-thia-3a,6-diaza-phenalen-3-yl)-N,N-dibutyl-acetamid;
2-(8-Brom-4,5-dioxo-2,3,5,6-tetrahydro-4H-1-thia-3a,6-diaza-phenalen-3-yl)-N-(2-oxo-azepan-3-yl)-acetamid;
2-(8-Brom-4,5-dioxo-2,3,5,6-tetrahydro-4H-1-thia-3a,6-diaza-phenalen-3-yl)-N-(2,2-dimethoxy-ethyl)-acetamid;
2-(8-Brom-4,5-dioxo-2,3,5,6-tetrahydro-4H-1-thia-3a,6-diaza-phenalen-3-yl)-N-(5,6,7,8-tetrahydro-naphthalen-1-yl)-acetamid;
2-(8-Brom-4,5-dioxo-2,3,5,6-tetrahydro-4H-1-thia-3a,6-diaza-phenalen-3-yl)-N-furan-2-ylmethyl-acetamid;
2-(8-Brom-4,5-dioxo-2,3,5,6-tetrahydro-4H-1-thia-3a,6-diaza-phenalen-3-yl)-N-(2-methoxyethyl)-acetamid;
2-(8-Brom-4,5-dioxo-2,3,5,6-tetrahydro-4H-1-thia-3a,6-diaza-phenalen-3-yl)-N-tert.-butoxy-acetamid;
2-(8-Brom-4,5-dioxo-2,3,5,6-tetrahydro-4H-1-thia-3a,6-diaza-phenalen-3-yl)-N-prop-2-ynyl-acetamid;
55.2-(8-Brom-4,5-dioxo-2,3,5,6-tetrahydro-4H-1-thia-3a,6-diaza-phenalen-3-yl)-N-cyano-methyl-acetamid;
2-(8-Brom-4,5-dioxo-2,3,5,6-tetrahydro-4H-1-thia-3a,6-diaza-phenalen-3-yl)-N-methoxy-acetamid;
2-(8-Brom-4,5-dioxo-2,3,5,6-tetrahydro-4H-1-thia-3a,6-diaza-phenalen-3-yl)-acetamid ;
2-(8-Chlor-4,5-dioxo-2,3,5,6-tetrahydro-4H-1-oxa-3a,6-diaza-phenalen-3-yl)-N-phenyl-acetamid;
2-(8-Chlor-4,5-dioxo-2,3,5,6-tetrahydro-4H-1-oxa-3a,6-diaza-phenalen-3-yl)-N-methyl-N-phenyl-acetamid;
2-(8-Chlor-4,5-dioxo-2,3,5,6-tetrahydro-4H-1-oxa-3a'6-diaza-phenalen-3-yl)-N,N-bis-(2-hydroxy-ethyl)-acetamid;
2-(8-Chlor-4,5-dioxo-2,3,5,6-tetrahydro-4H-1-oxa-3a,6-diaza-phenalen-3-yl)-N-phenylacetamid;
2-[2-(8-chlor-4,5-dioxo-2,3,5,6-tetrahydro-4H-1-thia-3a,6-diaza-phenalen-3-yl)-N-phenyl acetamid;
2-[2-(8-Brom-4,5-dioxo-2,3,5,6-tetrahydro-4H-1-thia-3a,6-diaza-phenalen-3-yl)-acetylamino]-3,3-dimethyl-buttersäuretertiärbutylester;
2-[2-(8-Brom-4,5-dioxo-2,3,5,6-tetrahydro-4H-1-thia-3a,6-diaza-phenalen-3-yl)-acetylamino]-malonsäuredimethylester;
2-[2-(8-Brom-4,5-dioxo-2,3,5,6-tetrahydro-4H-1-thia-3a,6-diaza-phenalen-3-yl)-acetylamino]-benzoesäuremethylester;
2-[2-(8-Brom-4,5-dioxo-2,3,5,6-tetrahydro-4H-1-thia-3a,6-diaza-phenalen-3-yl)-acetylamino]-benzoesäure;
2-[2-(8-Brom-4,5-dioxo-2,3,5,6-tetrahydro-4H-1-thia-3a,6-diaza-phenalen-3-yl)-acetylamino]-3,3-dimethyl-buttersäure;
3-(R)-8-Brom-4,5-dioxo-2,3,5,6-tetrahydro-4H-1-thia-3a,6-diaza-phenalen-3-ylessigsäureethylester;
3-(R)-8-Brom-4,5-dioxo-2,3,5,6-tetrahydro-4H-1-thia-3a,6-diaza-phenalen-3-ylessigsäure;
3-(S)-8-Brom-4,5-dioxo-2,3,5,6-tetrahydro-4H-1-thia-3a,6-diaza-phenalen-3-ylessigsäureethylester;
3-(S)-8-Brom-4,5-dioxo-2,3,5,6-tetrahydro-4H-1-thia-3a,6-diaza-phenalen-3-ylessigsäure;
3-[2-(8-Brom-4,5-dioxo-2,3,5,6-tetrahydro-4H-1-thia-3a,6-diaza-phenalen-3-yl)-acetylamino]-propionsäuremethylester;
3-[2-(8-Brom-4,5-dioxo-2,3,5,6-tetrahydro-4H-1-thia-3a,6-diaza-phenalen-3-yl)-acetylamino]-propionsäure;
4,5-Dioxo-2,3,5,6-tetrahydro-4H-1-oxa-3a,6-diaza-phenalen-3-ylessigsäureethylester;
4-[2-(8-Brom-4,5-dioxo-2,3,5,6-tetrahydro-4H-1-thia-3a,6-diaza-phenalen-3-yl)-acetylamino]-benzoesäuremethylester;
7,9-Dibrom-4,5-dioxo-2,3,5,6-tetrahydro-4H-1-oxa-3a,6-diaza-phenalen-3-ylessigsäureethylester;
8-Brom-1,1,4,5-tetraoxo-2,3,5,6-tetrahydro-1H,4H-6-thia-3a,6-diaza-phenalen-3-ylessigsäureethylester;
8-Brom-1,1,4,5-tetraoxo-2,3,5,6-tetrahydro-1H,4H-6-thia-3a,6-diaza-phenalen-3-ylessigsäure;
8-Brom-3-(2-oxo-2-piperidin-1-yl-ethyl)-2,3-dihydro-6H-1-thia-3a,6-diaza-phenalen-4,5-dion;
8-Brom-3-(2H-tetrazol-5-ylmethyl)-2,3-dihydro-6H-1-thia-3a,6-diaza-phenalen-4,5-dion;
8-Brom-3-[2-(2,3-dihydro-indol-1-yl)-2-oxo-ethyl]-2,3-dihydro-6H-1-thia-3a,6-diaza-phenalen 4,5-dion;
8-Brom-3-[2-(2-methyl-aziridin-1-yl)-2-oxo-ethyl]-2,3-dihydro-6H-1-thia-3a,6-diaza-phenalen-4,5-dion;
8-Brom-4,5-dioxo-2,3,5,6-tetrahydro-4H-1-thia-3a,6-diaza-phenalen-3-ylessigsäureethylester;
8-Brom-4,5-dioxo-2,3,5,6-tetrahydro-4H-1-thia-3a,6-diaza-phenalen-3-ylessigsäure;
8-Brom-4,5-dioxo-2,3,5,6-tetrahydro-4H-1-thia-3a,6-diaza-phenalen-3-ylessigsäuremethoxycarbonyl-methylester;
8-Brom-4,5-dioxo-2,3,5,6-tetrahydro-4H-1-thia-3a,6-diaza-phenalen-3-ylacetonitril;
8-Chlor-2,3,5,6-dihydro-6H-1 -thia-3a,6-diaza-phenalen-4,5-dion;
8-Chlor-4,5-dioxo-2,3,5,6-tetrahydro-4H-1-oxa-3a,6-diaza-phenalen-3-ylessigsäureethylester;
8-Chlor-4,5-dioxo-2,3,5,6-tetrahydro-4H-1-oxa-3a,6-diaza-phenalen-3-ylessigsäure;
8-Chlor-4,5-dioxo-2,3,5,6-tetrahydro-4H-1-oxa-3a,6-diaza-phenalen-3-ylessigsäure;
8-Chlor-4,5-dioxo-2,3,5,6-tetrahydro-4H-1-oxa-3a,6-diaza-phenalen-3-ylessigsäurebutylester;
8-Chlor-4,5-dioxo-2,3,5,6-tetrahydro-4H-1-thia-3a,6-diaza-phenalen-3-ylessigsäureethylester;
8-Chlor-4,5-dioxo-2,3,5,6-tetrahydro-4H-1-thia-3a,6-diaza-phenalen-3-ylessigsäure;
8-Methyl-4,5-dioxo-2,3,5,6-tetrahydro-4H-1-thia-3a,6-diaza-phenalen-3-ylessigsäureethylester;
8-Methyl-4,5-dioxo-2,3,5,6-tetrahydro-4H-1-thia-3a,6-diaza-phenalen-3-ylessigsäure;
3-[2-(8-Brom-4,5-dioxo-2,3,5,6-tetrahydro-4H-1-thia-3a,6-diaza-phenalen-3-yl)-acetylamino]-but-2-en-säuremethylester;
Benzoesäure-(8-brom-4,5-dioxo-2,3,5,6-tetrahydro-4H-1-thia-3a,6-diaza-phenalen-3-yl)-acetoxymethylester;
N-Adamantan-1-yl-2-(8-brom-4,5-dioxo-2,3,5,6-tetrahydro-4H-1-thia-3a,6-diaza-phenalen-3-yl)-acetamid;
N-Adamantan-1-yl-2-(8-chlor-4,5-dioxo-2,3,5,6-tetrahydro-4H-1-oxa-3a,6-diaza-phenalen-3-yl)-acetamid;
1N-Adamantan-2-yl-2-(8-brom-4,5-dioxo-2,3,5,6-tetrahydro-4H-1-thia-3a,6-diaza-phenalen-3-yl)-acetamid;
N-Allyl-2-(8-brom-4,5-dioxo-2,3,5,6-tetrahydro-4H-1-thia-3a,6-diaza-phenalen-3-yl)-acetamid;
N-Allyloxy-2-(8-brom-4,5-dioxo-2,3,5,6-tetrahydro-4H-1-thia-3a,6-diaza-phenalen-3-yl)-acetamid;
N-Benzyloxy-2-(8-brom-4,5-dioxo-2,3,5,6-tetrahydro-4H-1-thia-3a,6-diaza-phenalen-3-yl)-acetamid;
N-Bicyclo[2,21]hept-2-yl-2-(8-brom-4,5-dioxo-2,3,5,6-tetrahydro-4H-1-thia-3a,6-diaza-phenalen-3-yl)-acetamid;
2,3-Dihydro-6H-1-oxa-3a,6-diaza-phenalen-4,5-dion;
2-(8-Brom-4,5-dioxo-2,3,5,6-tetrahydro-4H-1-thia-3a,6-diaza-phenalen-3-yl)-N-ethoxy-acetamid;
2-(8-Brom-4,5-dioxo-2,3,5,6-tetrahydro-4H-1-thia-3a,6-diaza-phenalen-3-yl)-N-hydroxy-acetamid;
2-(8-Brom-4,5-dioxo-2,3,5,6-tetrahydro-4H-1-thia-3a,6-diaza-phenalen-3-yl)-N-(2,2,2-trifluorethyl)-acetamid;
2-(8-Brom-4,5-dioxo-2,3,5,6-tetrahydro-4H-1-thia-3a,6-diaza-phenalen-3-yl)-N-methoxy-N-methyl-acetamid;
2-[2-(8-Brom-4,5-dioxo-2,3,5,6-tetrahydro-4H-1-thia-3a,6-diaza-phenalen-3-yl)acetylamino) -essigsäureethylester;
2-[2-(8-Brom-4,5-dioxo-2,3,5,6-tetrahydro-4H-1-thia-3a,6-diaza-phenalen-3-yl)acetylamino)-propionsäuremethytester;
2-(8-Brom-4,5-dioxo-2,3,5,6-tetrahydro-4H-1-thia-3a,6-diaza-phenalen-3-yl)-N-(2,3,4,5,6-pentafluorbenzyloxy)-acetamid;
2-(8-Brom-4,5-dioxo-2,3,5,6-tetrahydro-4H-1-thia-3a,6-diaza-phenalen-3-yl)-N-phenoxy-acetamid;
2-(8-Brom-4,5-dioxo-2,3,5,6-tetrahydro-4H-1-thia-3a,6-diaza-phenalen-3-yl)-N-(3-oxo-3,4-dihydro-2H-benzo[1,4]oxazin-7-ylmethyl)-acetamid;
2-[2-(8-Brom-4,5-dioxo-2,3,5,6-tetrahydro-4H-1-thia-3a,6-diaza-phenalen-3-yl)acetylomino)-5-(tertiärbutyloxycarbonylaminomethyl)benzoesäuremethylester;
2-[2-(8-Brom-4,5-dioxo-2,3,5,6-tetrahydro-4H-1-thia-3a,6-diaza-phenalen-3-yl)acetylomino)-5-(tertiärbutyloxycorbonylominomethyl) benzoesäure;
4-[2-(8-Brom-4,5-dioxo-2,3,5,6-tetrahydro-4H-1-thia-3a,6-diaza-phenalen-3-yl)acetylomino)-benzoesäure;
2-(8-Brom-4,5-dioxo-2,3,5,6-tetrahydro-4H-1-thia-3a,6-diaza-phenalen-3-yl)-N-(3,5-bistrifluormethylphenyl)-acetamid;
2-(8-Brom-4,5-dioxo-2,3,5,6-tetrahydro-4H-1-thia-3a,6-diaza-phenalen-3-yl)-N-(2-chlor-5-trifluormethylphenyl)-acetamid;
2-(8-Brom-4,5-dioxo-2,3,5,6-tetrahydro-4H-1-thia-3a,6-diaza-phenalen-3-yl)-N-(2-methoxyphenyl)-acetamid;
2-(8-Brom-4,5-dioxo-2,3,5,6-tetrahydro-4H-1-thia-3a,6-diaza-phenalen-3-yl)-N-(2-trifluormethoxyphenyl)-acetamid;
2-(8-Brom-4,5-dioxo-2,3,5,6-tetrahydro-4H-1-thia-3a,6-diaza-phenalen-3-yl)-N-(4-methoxyphenyl)-acetamid;
2-(8-Brom-4,5-dioxo-2,3,5,6-tetrahydro-4H-1-thia-3a,6-diaza-phenalen-3-yl)-N-(2,5-dimethoxyphenyl)-acetamid;
2-(8-Brom-4,5-dioxo-2,3,5,6-tetrahydro-4H-1-thia-3a,6-diaza-phenalen-3-yl)-N-(2-fluor-5-trifluormethyl-phenyl)-acetamid;
2-(8-Brom-4,5-dioxo-2,3,5,6-tetrahydro-4H-1-thia-3a,6-diaza-phenalen-3-yl)-N-(4-phenoxyphenyl)-acetamid;
2-(8-Brom-4,5-dioxo-2,3,5,6-tetrahydro-4H-1-thia-3a,6-diaza-phenalen-3-yl)-N-(4-(4-chlorphenoxy)phenyl]-acetamid;
2-(8-Brom-4,5-dioxo-2,3,5,6-tetrahydro-4H-1-thia-3a,6-diaza-phenalen-3-yl)-N-(3,4,5-trimethoxyphenyl)-acetamid;
2-(8-Brom-4,5-dioxo-2,3,5,6-tetrahydro-4H-1-thia-3a,6-diaza-phenalen-3-yl)-N-(4-fluor-2-trifluormethyl-phenyl)-acetamid;
2-(8-Brom-4,5-dioxo-2,3,5,6-tetrahydro-4H-1-thia-3a,6-diaza-phenalen-3-yl)-N-(3-phenoxyphenyl)-acetamid;
2-(8-Brom-4,5-dioxo-2,3,5,6-tetrahydro-4H-1-thia-3a,6-diaza-phenalen-3-yl)-N-(1-cyano-1-methylethyl)-acetamid;
2-(8-Brom-4,5-dioxo-2,3,5,6-tetrahydro-4H-1-thia-3a,6-diaza-phenalen-3-yl)-N-(2-methoxy-5-methyl-phenyl)-acetamid;
2-(8-Brom-4,5-dioxo-2,3,5,6-tetrahydro-4H-1-thia-3a,6-diaza-phenalen-3-yl)-N-(3-methoxy-phenyl)-acetamid;
2-(8-Brom-4,5-dioxo-2,3,5,6-tetrahydro-4H-1-thia-3a,6-diaza-phenalen-3-yl)-N-(3,5-dimethoxy-phenyl)-acetamid;
8-Methyl-4,5-dioxo-2,3,5,6-tetrahydro-4H-1-thia-3a,6-diaza-phenalen-3-ylessigsäure;
2-(8-Methyl-4,5-dioxo-2,3,5,6-tetrahydro-4H-1-thia-3a,6-diaza-phenalen-3-yl)-N-phenyl-acetamid oder
2-(8-Methyl-4,5-dioxo-2,3,5,6-tetrahydro-4H-1-thia-3a,6-diaza-phenalen-3-yl)-N-(tertiärbutyloxy)-acetamid oder jeweils ein Salz davon.

**15.** Eine Verbindung gemäß einem der Ansprüche 1 bis 14 zur Behandlung des menschlichen oder tierischen Körpers.

**16.** Pharmazeutische Präparate, enthaltend eine Verbindung gemäß einem der Ansprüche 1, 4, 5, 7, 9, 11 und 14 oder ein pharmazeutisch verwendbares Salz davon neben üblichen pharmazeutischen Hilfs- und Trägerstoffen.

**17.** Pharmazeutische Präparate, enthaltend eine Verbindung gemäß einem der Ansprüche 2, 6, 8, 10 und 13, oder ein pharmazeutisch verwendbares Salz davon neben üblichen pharmazeutischen Hilfs- und Trägerstoffen.

**18.** Pharmazeutische Präparate, enthaltend eine Verbindung gemäß einem der Ansprüche 3 und 12 oder ein pharmazeutisch verwendbares Salz davon neben üblichen pharmazeutischen Hilfs- und Trägerstoffen.

**19.** Verfahren zur Herstellung neuer oxa- und thiaaliphatisch überbrückter Chinoxalin-2,3-dione der allgemeinen Formel I 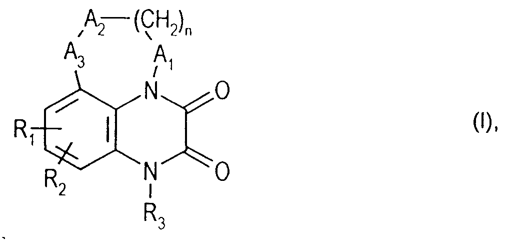 worin
A₁ Niederalkyliden oder eine Gruppe der Formel >CH-A₄-R₄ (la) darstellt,
A₂ Niederalkyliden oder eine Gruppe der Formeln >CH-A₄-R₄ (la), >C=O (Ib) oder >CH(OH)- A₅-R₄ (Ic) ist,
A₃ Oxy, gegebenenfalls oxidiertes Thio oder eine Gruppe >C(=O) (Ib) bedeutet,
A₄ für Niederalkylen steht,
A₅ Niederalkylen oder eine direkte Bindung darstellt,
n für 0 oder 1 steht,
R₁ und R₂ unabhängig voneinander Wasserstoff, gegebenenfalls durch Niederalkyl und/oder Niederalkanoyl substituiertes Amino, Nitro, Niederalkanoyl, gegebenenfalls verethertes oder verestertes Hydroxy, gegebenenfalls verestertes oder amidiertes Carboxy, Cyano, gegebenenfalls halogeniertes Niederalkyl oder Halogen bedeuten,
R₃ Wasserstoff oder Hydroxy bedeutet und
R₄ Wasserstoff, Cyano, gegebenenfalls verestertes oder amidiertes Carboxy, gegebenenfalls verestertes Phosphono oder 5-Tetrazolyl darstellt,
und ihrer Salze, dadurch gekennzeichnet, daß man
a) eine Verbindung der Formel II 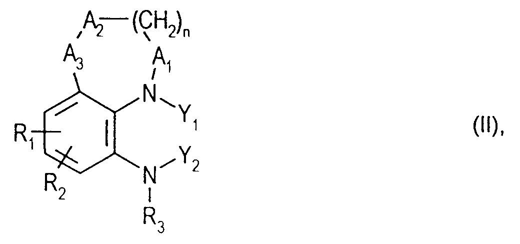 worin
einer der Reste Y₁ und Y₂ für eine Gruppe der Formel -C (=O) -Y₃ (IIa) steht, in der Y₃ eine funktionell abgewandelte Carboxygruppe darstellt, und der andere Wasserstoff bedeutet, oder jeweils ein Salz davon intramolekular cyclisiert oder
b) eine Verbindung der Formel III 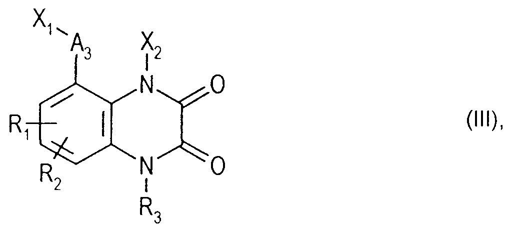 worin X₁ eine Gruppe der Formel -A₂-(CH₂)ₙ-CH(Y₃)-A₄-R₄ (IIIa)
oder-A₂-(CH₂)ₙ-CH=A'₄-R₄ (IIIb) darstellt, wobei Y₃ eine nukleofuge Abgangsgruppe und A'₄ eine der Gruppe A₄ entsprechende Niederalkanylylidengruppe bedeutet, X₂ für Wasserstoff steht und R₁, R₂, R₃, A₁, A₂, A₃ und A₄ die angebenen Bedeutungen haben,
intramolekular cyclisiert oder
c) in einer Verbindung der Formel IV 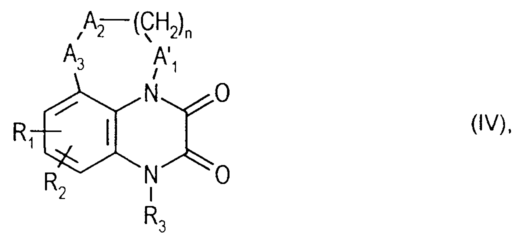 worin A'₁ eine Gruppe der Formel >C=A'₄-R₄ (IIIa) darstellt, in der A'₄ für Niederalkanylyliden bedeutet, die extracyclische Doppelbindung zu eine Einfachbindung reduziert und jeweils gewünschtenfalls eine erhaltene Verbindung in eine andere Verbindung der Formel I überführt, ein verfahrensgemäß erhältliches Isomerengemisch in die Komponenten auftrennt und das jeweils bevorzugte Isomere abtrennt und/oder eine verfahrensgemäß erhältliche freie Verbindung in ein Salz oder eine verfahrensgemäß erhältliches Salz in die entsprechende freie Verbindung überführt.

**20.** Verwendung einer Verbindung gemäß einem der Ansprüche 1 bis 14 zur Herstellung eines für die Behandlung von pathologischen Zuständen, die auf Glycin-antagonistische Blockierung von NMDA-sensitiven Rezeptoren ansprechen, bestimmten Arzneimittels.
